# EUROPEAN PATENT SPECIFICATION

(11) **EP 2 529 030 B1**
(45) Date of publication and mention of the grant of the patent: **13.03.2019**
(21) Application number: 11702567.6
(22) Date of filing: 31.01.2011
(51) Int. Cl.: C12Q 1/6841, C12Q 1/6881

(54) **METHODS OF IN SITU DETECTION OF NUCLEIC ACIDS**
VERFAHREN ZUM IN SITU NACHWEIS VON NUKLEINSÄUREN
METHODE DE DETECTION D'ACIDES NUCLEIQUES IN SITU

(30) Priority: 29.01.2010 US 336944 P
(43) Date of publication of application: 05.12.2012
(73) Proprietor: Advanced Cell Diagnostics, Inc., Newark, CA 94560 (US)
(72) Inventor: LUO, Yuling, San Ramon, CA 94582 (US); FLANAGAN, John, James, Walnut Creek, CA 94598 (US); CHEN, Shiping, Fremont, CA 94539 (US); WANG, Huei-yu, Fay, San Francisco, CA 94131 (US)
(74) Representative: Weber, Martin
(86) International application number: PCT/US2011/023126
(87) International publication number: WO 2011/094669

(56) References cited:
- WO-A2-01/94632
- WO-A2-2007/001986
- WO-A2-2007/002006
- US-A1- 2008 008 994
- US-A1- 2010 081 131
- NOLTE F S: "BRANCHED DNA SIGNAL AMPLIFICATION FOR DIRECT QUANTITATION OF NUCLEIC ACID SEQUENCES IN CLINICAL SPECIMENS", ADVANCES IN CLINICAL CHEMISTRY, ACADEMIC PRESS, LONDON, GB, vol. 33, 1 January 1998 (1998-01-01), pages 201-235, XP001041847, ISSN: 0065-2423
- SLEIJFER ET AL: "Circulating tumour cell detection on its way to routine diagnostic implementation?", EUROPEAN JOURNAL OF CANCER, PERGAMON PRESS, OXFORD, GB, vol. 43, no. 18, 30 October 2007 (2007-10-30), pages 2645-2650, XP022361345, ISSN: 0959-8049
- MOCELLIN SIMONE ET AL: "Molecular detection of circulating tumor cells is an independent prognostic factor in patients with high-risk cutaneous melanoma", INTERNATIONAL JOURNAL OF CANCER, vol. 111, no. 5, 20 September 2004 (2004-09-20), pages 741-745, XP002628788, ISSN: 0020-7136 cited in the application
- WANG FAY ET AL: "Multiplex analysis of gene expression in single cells for circulating tumor cell detection", PROCEEDINGS OF THE AMERICAN ASSOCIATION FOR CANCER RESEARCH ANNUAL MEETING, vol. 49, April 2008 (2008-04), pages 1224-1225, XP002628789, & 99TH ANNUAL MEETING OF THE AMERICAN-ASSOCIATION-FOR-CANCER-RESEARCH; SAN DIEGO, CA, USA; APRIL 12 -16, 2008 ISSN: 0197-016X
- Anton H.N. Hopman ET AL: "HPV in situ hybridization: Impact of different protocols on the detection of integrated HPV", International Journal of Cancer, vol. 115, no. 3, 1 February 2005 (2005-02-01), pages 419-428, XP055145625, ISSN: 0020-7136, DOI: 10.1002/ijc.20862

## Description

### STATEMENT AS TO RIGHTS TO INVENTIONS MADE UNDER FEDERALLY SPONSORED RESEARCH AND DEVELOPMENT

This invention was made with government support under Grant No. R43CA122444-01 from the National Cancer Institute Small Business Innovation Research Program and Grant No. W81XWH-06-1-0682 from the United States Army Medical Research Acquisition Activity Breast Cancer Research Program. The government may have certain rights to this invention.

### FIELD OF THE INVENTION

The invention relates generally to nucleic acid chemistry and biochemical assays. More particularly, the invention relates to methods for in situ detection of nucleic acid analytes in single cells. The invention also relates to detection and identification of single cells, particularly rare cells.

### BACKGROUND OF THE INVENTION

Ample evidence has demonstrated that cancer cells can dissociate from the primary tumor and circulate in the lymph node, bone marrow, peripheral blood or other body fluids. These circulating tumor cells (CTC) have been shown to reflect the biological characteristics of the primary tumors, including the potential for metastasis development and tumor recurrence. Therefore, the detection of CTC may indicate disease recurrence, tumor cell spreading, and a high potential for distant metastasis. All of these are significant informative clinical factors in identifying high-risk cancer patients' disease status (e.g. Vogel et al., 2002; Gilbey et al., 2004; Molnar et al., 2003; Vlems et al., 2003; Ma et al., 2003).

Validation of the clinical utility of CTC detection as a prognostic indicator has not been progressing as fast as expected, in large part due to lack of suitable detection technologies. One key difficulty in detecting CTC in peripheral blood or other body fluids is that CTC are present in the circulation in extremely low concentrations, estimated to be in the range of one tumor cell among 106-107 normal white blood cells. As a result, any detection technology for this application has to exhibit exceptional sensitivity and specificity in order to limit both false negative and false positive rate to an acceptable level.

One existing approach incorporates immunomagnetic separation technology in detection of intact CTC (US 6,365,362; US 6,645,731). Using this technology, a blood sample from a cancer patient is incubated with magnetic beads coated with antibodies directed against an epithelial surface antigen as for example EpCAM (Cristofanilli et al., 2004). The magnetically labeled cells are then isolated using a magnetic separator. The immunomagnetically-enriched fraction is further processed for downstream analysis for CTC identification. Using this technology, it was shown in a prospective study that the number of CTC after treatment is an independent predictor of progression-free survival and overall survival in patients with metastatic breast cancer (Cristofanilli et al., 2004). Although this technology has reported high sensitivity, its applicability is limited by the availability of detection antibodies that are highly sensitive and specific to particular types of CTC. The antibodies can exhibit non-specific binding to other cellular components which can lead to low signal to noise ratio and impair later detection. The antibodies binding to CTC may also bind to antigen present in other types of cells at low level, resulting in a high level of false positives.

Another approach for determining the presence of CTC has been to test for the tumor cell specific expression of messenger RNA in blood. Real time reverse transcription-polymerase chain reaction (QPCR) has been used to correlate the detection of CTC with patient prognosis. Real-time RT-PCR has been used for detecting CEA mRNA in peripheral blood of colorectal cancer patients (Ito et al., 2002). Disease free survival of patients with positive CEA mRNA in post-operative blood was significantly shorter than in cases that were negative for CEA mRNA. These results suggest that tumor cells were shed into the bloodstream and resulted in poor patient outcomes in patients with colorectal cancer. Another report demonstrated the clinical utility of molecular detection of CTC in high-risk AJCC stage IIBC and IIIAB melanoma patients using multiple mRNA markers by QPCR (Mocellin et al., 2004). The advantage of detecting tumor specific mRNA expression is that any tumor-specific gene can be used to serve as a diagnostic/prognostic marker. However, the QPCR approach requires the laborious procedure of mRNA isolation from the blood sample and reverse transcription before the PCR reaction. False positives are often observed using this technique due to sample contamination by chromosomal DNA or low-level expression of the chosen marker gene in normal blood cells (Fava et al. 2001). In addition, the limit of detection sensitivity of this technique is at most about one tumor cell per 1 ml of blood, and the technology cannot provide an accurate count of CTC numbers.

WO 2007/001986 discloses methods of detecting multiple nucleic acid targets in individual cells and methods of identifying rare cells from large heterogeneous cell populations.

US 2008/0008994 discloses peptide nucleic acid (PNA) probes and methods for the analysis of certain *Staphylococcus* species in a sample, in particular methods of differentiation between *Staphylococcus* species other than *S. aureus.*

Rapid and sensitive techniques for detection of CTCs, and more generally for detection of nucleic acids in cells, are thus desirable. The present invention meets these and other needs, *inter alia* providing methods for detecting nucleic acids in and for identifying individual cells. A complete understanding of the invention will be obtained upon review of the following.

### SUMMARY OF THE INVENTION

The present invention provides a method of detecting an individual cell of a specified type in a sample comprising a mixture of cell types, which mixture comprises at least one cell of the specified type, wherein the cell comprises a first nucleic acid target and a second nucleic acid target, wherein the first and second nucleic acid targets always co-exist in the cell, the method comprising:
(a) providing a first label probe comprising a first label, and providing a second label probe comprising a second label, wherein a first signal from the first label is indistinguishable from a second signal from the second label;
(b) capturing, in the cell, the first label probe to the first nucleic acid target, when present in the cell, and the second label probe to the second nucleic acid target, when present in the cell, wherein the first and second nucleic acid targets are separate molecules, wherein the capturing comprises:
   providing for the first nucleic acid target at least a first capture probe and providing for the second nucleic acid target at least a second capture probe;
   capturing the first label probe to the first nucleic acid target,
   comprising hybridizing in the cell the first capture probe to the first target nucleic acid, and hybridizing the first label probe to the first capture probe, thereby capturing the first label probe to the first nucleic acid target; and
   capturing the second label probe to the second nucleic acid target, comprising hybridizing in the cell the second capture probe to the second target nucleic acid, and hybridizing the second label probe to the second capture probe, thereby capturing the second label probe to the second nucleic acid target;
(c) detecting the signal from the labels;
(d) correlating the signal detected from the cell with the presence or amount of the first and second nucleic acid targets in the cell; and
(e) identifying the cell as being of the specified type based on detection of the presence or amount of the first and second nucleic acid targets within the cell, wherein the specified type of cell is distinguishable from the other cell type(s) in the mixture on the basis of either the presence or amount of the first nucleic acid target and the presence or amount of the second nucleic acid target in the cell.

According to some embodiments of the invention, providing at least a first capture probe comprises providing two or more different first capture probes as a set, wherein providing at least a second capture probe comprises providing two or more different second capture probes as a set; wherein capturing the first label probe to the first nucleic acid target comprises hybridizing in the cell the two or more different first capture probes to the first target nucleic acid, and hybridizing the first label probe to the two or more different first capture probes, thereby capturing the first label probe to the first nucleic acid target; wherein capturing the second label probe to the second nucleic acid target comprises hybridizing in the cell the two or more different second capture probes to the second target nucleic acid, and hybridizing the second label probe to the two or more different second capture probes, thereby capturing the second label probe to the second nucleic acid target.

According to some embodiments of the invention, providing at least a first capture probe comprises providing two or more different first capture probes as a set; wherein providing at least a second capture probe comprises providing two or more different second capture probes as a set; wherein the method further comprises providing an amplifier, wherein amplifier is a molecule capable of hybridizing to multiple label probes; wherein capturing the first label probe to the first nucleic acid target comprises hybridizing in the cell the first target nucleic acid to the two or more different first capture probes, hybridizing the two or more different first capture probes to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the amplifier, and hybridizing the amplifier to the second label probe, thereby capturing the second label probe to the second nucleic acid target.

According to some embodiments of the invention, providing at least a first capture probe comprises providing two or more different first capture probes as a set; wherein providing at least a second capture probe comprises providing two or more different second capture probes as a set; wherein the method further comprises providing an amplifier and a preamplifier; wherein capturing the first label probe to the first nucleic acid target comprises hybridizing in the cell the first target nucleic acid to the two or more different first capture probes, hybridizing the two or more different first capture probes to the preamplifier, hybridizing the preamplifier to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the preamplifier, hybridizing the preamplifier to the amplifier, and hybridizing the amplifier to the second label probe, thereby capturing the second label probe to the second nucleic acid target.

According to some embodiments of the invention, hybridizing the two or more different capture probes to the label probe, amplifier, or preamplifier is performed at a hybridization temperature that is greater than a melting temperature Tm of a complex between each individual capture probe and the label probe, amplifier, or preamplifier.

According to some embodiments of the invention, the two or more different capture probes hybridize to unique and adjacent sections on the nucleic acid target.

According to some embodiments of the invention, hybridizing the two or more different capture probes to their corresponding nucleic acid target is performed at a hybridization temperature that is lower than a melting temperature Tm of a complex between each individual capture probe and the nucleic acid target, or
wherein hybridizing the two or more different capture probes to their corresponding nucleic acid target is performed at a hybridization temperature that is greater than a melting temperature Tm of a complex between each individual capture probe and the nucleic acid target.

According to some embodiments of the invention, the method further comprising providing a plurality of additional nucleic acid targets; wherein the method further comprises: providing a plurality of label probes each comprising a label, wherein the signal from each label is indistinguishable from the first and second signals or from each other; providing at least one capture probe for each of the additional nucleic acid targets, hybridizing in the cell each capture probe to its corresponding nucleic acid target, when present in the cell, and hybridizing each label probe to its corresponding capture probe, and detecting the signal from the labels.

According to some embodiments of the invention, the first nucleic acid target and the second nucleic acid target are independently selected from the group consisting of: a DNA, an RNA, a chromosomal DNA, an mRNA, a nucleic acid endogenous to the cell, a nucleic acid introduced to or expressed in the cell by infection of the cell with a pathogen and a cytoplasmic RNA.

According to some embodiments of the invention,
(i) the first nucleic acid target is a first mRNA and wherein the second nucleic acid target is a second mRNA; or
(ii) the first nucleic acid target comprises a first chromosomal DNA polynucleotide sequence and wherein the second nucleic acid target comprises a second chromosomal DNA polynucleotide sequence; or
(iii) the first nucleic acid target and/or the second nucleic acid target comprises a double-stranded DNA molecule, the method comprising denaturing the double-stranded DNA prior to hybridization of the first and second capture probes to the first and second nucleic acid targets.

According to some embodiments of the invention,
(i) about 1000 copies or less of the first nucleic acid target and/or about 1000 copies or less of the second nucleic acid target are present in the cell; or
(ii) about 100 copies or less of the first nucleic acid target and/or about 100 copies or less of the second nucleic acid target are present in the cell; or
(iii) about 10 copies or less of the first nucleic acid target and/or about 10 copies or less of the second nucleic acid target are present in the cell; or
(iv) about 5 copies or less of the first nucleic acid target and/or about 5 copies or less of the second nucleic acid target are present in the cell; or
(v) a single copy of the first nucleic acid target and/or a single copy of the second nucleic acid target is present in the cell.

According to some embodiments of the invention, the first nucleic acid target, the second nucleic acid target, and/or the additional nucleic acid target is selected from the group consisting of: CK8, CK14, CK17, CK18, CK19, CK20, EpCAM, MucI, EGFR, Twist, N-Cadherin and Fibronectin.

Further, the present invention provides a method of detecting the presence or absence of a plurality of nucleic acid targets in an individual cell in a sample comprising the cell, which cell comprises or is suspected of comprising said plurality of nucleic acid targets, wherein said nucleic acid targets always co-exist in the cell, the method comprising:
(a) providing, for each of the plurality of nucleic acid targets, a label probe comprising a label, wherein a signal from the label of a first nucleic acid target in the plurality of nucleic acid targets is indistinguishable from a signal from the label of a second nucleic acid target in the plurality of nucleic acid targets, wherein the first and the second nucleic acid targets are separate molecules;
(b) providing, for each of the plurality of nucleic acid targets, at least a capture probe;
(c) hybridizing in the cell, for each of the plurality of nucleic acid targets, the capture probe to its corresponding nucleic acid target, when present in the cell;
(d) capturing, for each of the plurality of nucleic acid targets, the label probe to the capture probe, thereby capturing the label probe to its corresponding nucleic acid target; and
(e) detecting the signal from the label of the label probes captured to the plurality of nucleic acid targets,
wherein the presence of signal indicates the presence of a member of the plurality of nucleic acid targets and the absence of signal indicates the absence of the nucleic acid targets in the cell.

According to some embodiments of the invention, the plurality of nucleic acid targets comprise housekeeping genes of different tissues in a particular species, wherein the species is preferably a human.

According to some embodiments of the invention,
(i) providing at least a capture probe comprises providing two or more capture probes; wherein each of the two or more capture probes comprises a T section which is complementary to a region of its corresponding nucleic acid target and a L section which is complementary to a region of its corresponding label probe; further, the T sections of two or more capture probes are complementary to non-overlapping regions of the nucleic acid target and the L sections of the two or more capture probes are complementary to non-overlapping regions of the label probe, and wherein the method comprises hybridizing a label probe to the two or more capture probes;
(ii) providing at least a capture probe comprises providing two or more capture probes; wherein the method further comprises providing an amplifier; and wherein the method comprises hybridizing a label probe to an amplifier and hybridizing the amplifier to said two or more capture probes; or
(iii) providing at least a capture probe comprises providing two or more capture probes; wherein the method further comprises providing an amplifier and a preamplifier; and wherein the method comprises hybridizing the preamplifier to the two or more capture probes, hybridizing the amplifier to the preamplifier and hybridizing a label probe to the amplifier.

According to some embodiments of the invention, the method comprises the step of hybridizing each set of two or more capture probes to the corresponding target nucleic acid at a hybridization temperature (a) greater than the melting temperature of each T section of two or more capture probes in the set, or (b) greater than the melting temperature of each L section of two or more capture probes in the set.

According to some embodiments of the invention, the method comprises the step of hybridizing each set of two or more capture probes to the corresponding target nucleic acid at a hybridization temperature (a) greater than the melting temperature of each T section of two or more capture probes in the set and lower than the melting temperature of each L section of two or more capture probes in the set, or (b) greater than the melting temperature of each L section of two or more capture probes in the set and lower than the melting temperature of each T section of two or more capture probes in the set.

According to some embodiments of the invention, the plurality of nucleic acid targets are independently selected from the group consisting of: a DNA, an RNA, a chromosomal DNA, an mRNA, a nucleic acid endogenous to the cell, a nucleic acid introduced to or expressed in the cell by infection of the cell with a pathogen and cytoplasmic RNAs.

According to some embodiments of the invention, the two or more capture probes in each set all have the T sections 5' of the L sections, or wherein the two or more capture probes in each set all have the T sections 3' of the L sections.

According to some embodiments of the invention, each hybridization or capture step is accomplished for all of the nucleic acid targets at the same time.

According to some embodiments of the invention, the cell is a circulating tumor cell.

According to some embodiments of the invention, the cell is in suspension in the sample comprising the cell, and/or wherein the cell is in suspension during the hybridizing, capturing, and/or detecting steps.

According to some embodiments of the invention,the sample comprising the cell is derived from a bodily fluid, blood, or a tissue section.

According to some embodiments of the invention, providing a sample comprises fixing and permeabilizing the cell.

According to some embodiments of the invention, the method comprises washing the cell to remove materials not captured to one of the nucleic acid targets.

According to some embodiments of the invention, detecting the signal comprises performing flow cytometry.

In this application, methods of detecting the presence or absence of a class of nucleic acid targets in single cells through direct or indirect capture of labels to the nucleic acids are disclosed, where such labels to the class of nucleic acid targets are indistinguishable from each other. Also described are methods of detecting individual cells, particularly a cell of a specific type from large heterogeneous cell populations, through detection of one or more of nucleic acid targets, where the labels to the one or more of nucleic acid targets are indistinguishable from each other. Related kits are also described.

Disclosed herein are methods of detecting an individual cell of a specified type, the method comprises the steps of: providing a sample comprising a mixture of cell types, which mixture comprises at least one cell of the specified type; providing a first label probe comprising a first label, and providing a second label probe comprising a second label, wherein a first signal from the first label is indistinguishable from a second signal from the second label; capturing, in the cell, the first label probe to a first nucleic acid target, when present in the cell, and the second label probe to a second nucleic acid target, when present in the cell; detecting the signal from the labels; correlating the signal detected from the cell with the presence, absence, or amount of the first or second nucleic acid targets in the cell; and identifying the cell as being of the specified type based on detection of the presence, absence, or amount of either the first or second nucleic acid targets within the cell, wherein the specified type of cell is distinguishable from the other cell type(s) in the mixture on the basis of either the presence, absence, or amount of the first nucleic acid target or the presence, absence, or amount of the second nucleic acid target in the cell.

In one embodiment, the method provides at least a first capture probe and a second capture probe, wherein each of the capture probes comprises a T section which is complementary to a region of the target nucleic acid and a L section which is complementary to the label probe; wherein capturing the first label probe to the first nucleic acid target comprises hybridizing in the cell the first capture probe to the first target nucleic acid, and hybridizing the first label probe to the first capture probe, thereby capturing the first label probe to the first nucleic acid target; wherein capturing the second label probe to the second nucleic acid target comprises hybridizing in the cell the second capture probe to the second target nucleic acid, and hybridizing the second label probe to the second capture probe, thereby capturing the second label probe to the second nucleic acid target.

In a preferred embodiment, the method provides at least a first capture probe comprises providing two or more different first capture probes as a set, wherein providing at least a second capture probe comprises providing two or more different second capture probes as a set; wherein capturing the first label probe to the first nucleic acid target comprises hybridizing in the cell the two or more different first capture probes to the first target nucleic acid, and hybridizing the first label probe to the two or more different first capture probes, thereby capturing the first label probe to the first nucleic acid target; wherein capturing the second label probe to the second nucleic acid target comprises hybridizing in the cell the two or more different second capture probes to the second target nucleic acid, and hybridizing the second label probe to the two or more different second capture probes, thereby capturing the second label probe to the second nucleic acid target.

In another aspect, the label probes are captured to the capture probes indirectly, for example, through binding of preamplifiers and/or amplifiers. In one class of embodiments in which amplifiers are employed, the method further provides an amplifier; wherein capturing the first label probe to the first nucleic acid target comprises hybridizing in the cell the first target nucleic acid to the two or more different first capture probes, hybridizing the two or more different first capture probes to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the amplifier, and hybridizing the amplifier to the second label probe, thereby capturing the second label probe to the second nucleic acid target.

In one class of embodiments in which preamplifiers are employed, the method further provides an amplifier and a preamplifer wherein capturing the first label probe to the first nucleic acid target comprises hybridizing in the cell the first target nucleic acid to the two or more different first capture probes, hybridizing the two or more different first capture probes to the preamplifier, hybridizing the preamplifier to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the preamplifier, hybridizing the preamplifier to the amplifier, and hybridizing the amplifier to the second label probe, thereby capturing the second label probe to the second nucleic acid target.

In one aspect, the hybridization of the two or more different capture probes to the label probe, amplifier, or preamplifier may be performed at a hybridization temperature that is greater than a melting temperature Tm of a complex between each individual capture probe and the label probe, amplifier, or preamplifier.

In addition, the two or more different capture probes may be hybridized to unique and adjacent sections on the nucleic acid target.

In another aspect, the hybridization of the two or more different capture probes to their corresponding nucleic acid target may be performed at a hybridization temperature that is lower than a melting temperature Tm of a complex between each individual capture probe and the nucleic acid target.

In another aspect, the hybridization of the two or more capture probes to their corresponding nucleic acid target may be performed at a hybridization temperature that is greater than a melting temperature Tm of a complex between each individual capture probe and the nucleic acid target.

In one aspect, the two or more capture probes in each set all have the T section 5' of the L sections or wherein the two or more capture probes in each set all have the T section 3' of the L sections.

In another embodiment, the method may further provide a plurality of additional nucleic acid targets; wherein the method comprising: providing a plurality of label probes each comprising a label, wherein the signal from each label is indistinguishable from the first and second signals or from each other; providing at least one capture probe for each of the additional nucleic acid targets, hybridizing in the cell each capture probe to its corresponding nucleic acid target, when present in the cell, and hybridizing each label probe to its corresponding capture probe, and detecting the signal from the labels.

In one aspect, each hybridization or capture step of the method may be accomplished for all of the nucleic acid targets at the same time.

In one aspect of the method, the first nucleic acid target and the second nucleic acid target are independently selected from the group consisting of: a DNA, an RNA, a chromosomal DNA, an mRNA, a nucleic acid endogenous to the cell, and a nucleic acid introduced to or expressed in the cell by infection of the cell with a pathogen. In one embodiment, the first nucleic acid target may be a first mRNA and the second nucleic acid target may be a second mRNA. In another embodiment, the first nucleic acid target comprises a first chromosomal DNA polynucleotide sequence and the second nucleic acid target comprises a second chromosomal DNA polynucleotide sequence. The first nucleic acid target may comprise a first region of an mRNA and wherein the second nucleic acid target may comprise a second region of the same mRNA. In another embodiment, the first nucleic acid target and/or the second nucleic acid target is a cytoplasmic RNA.

In one aspect of the method, the cell to be detected is a circulating tumor cell.

In another aspect of the method, the sample comprising the cell is derived from a bodily fluid, blood, or a tissue section. In one embodiment, the method of identifying the cell as a desired target cell may be based on detection of the signals from within the cell.

In one aspect of the method, providing a sample comprises fixing and permeabilizing the cell. In another aspect of the method, the method comprises washing the cell to remove materials not captured to one of the nucleic acid targets.

In one aspect of the method, the first nucleic acid target and/or the second nucleic acid target comprises a double-stranded DNA molecule, the method comprising denaturing the double-stranded DNA prior to hybridization of the first and second capture probes to the first and second nucleic acid targets. In another aspect of the method, the cell is in suspension in the sample comprising the cell, and/or wherein the cell is in suspension during the hybridizing, capturing, and/or detecting steps. In yet another aspect of the method, detecting the first and second signals comprises performing flow cytometry.

The methods permit detection of even low or single copy number targets. Thus, in one class of embodiments, about 1000 copies or less of the first nucleic acid target and/or about 1000 copies or less of the second nucleic acid target are present in the cell (e.g., about 100 copies or less, about 50 copies or less, about 10 copies or less, about 5 copies or less, or even a single copy).

In one embodiment of the method, the first nucleic acid target, the second nucleic acid target, and/or the additional nucleic acid target is selected from the group consisting of: CK8, CK14, C17, CK18, CK19, CK20, EpCAM, MucI, EGFR, Twist, N-Cadherin and Fibronectin.

Further disclosed herein is a kit for detecting an individual cell of a specified type from a mixture of cell types. The kit comprises: at least a first capture probe capable of hybridizing to the first nucleic acid target; at least a second capture probe capable of hybridizing to the second nucleic acid target; a first label probe comprising a first label and a second label probe comprising a second label, wherein the first label probe is capable of hybridizing to the first capture probe and the second label probe is capable of hybridizing to the second capture probe; wherein a first signal from the first label is indistinguishable from a second signal from the second label; wherein the specified type of cell is distinguishable from the other cell types in the mixture by presence, absence, or amount of the first nucleic acid and/or the second nucleic acid target; and packaged in one or more containers.

Essentially all of the features noted for the above method of detection embodiments apply to these kits as well, as relevant; for example, with respect to number of nucleic acid targets, inclusion of capture probes, configuration and number of the label and/or capture probes, indistinguishable labels, inclusion of preamplifiers and/or amplifiers, inclusion of amplification reagents, type of nucleic acid target, location of various targets on a single molecule or on different molecules, type of labels, various hybridization temperature schemes, capture probes that all have the T section 5' of the L sections or all have the T section 3' of the L sections, sample processing methods, copy number of the target, and/or the like.

Disclosed herein are methods of detecting the presence or absence of a class of nucleic acid targets in an individual cell, wherein the class of nucleic acid targets consists of a plurality of nucleic acid targets. The method comprises: providing a sample comprising the cell, which cell comprises or is suspected of comprising said class of nucleic acid targets; providing, for each of the plurality of nucleic acid targets, a label probe comprising a label, wherein a signal from the label of one nucleic acid target in the plurality of nucleic acid targets is distinguishable from a signal from the label of another nucleic acid target in the plurality of nucleic acid targets; providing, for each of the plurality of nucleic acid targets, at least a capture probe; hybridizing in the cell, for each of the plurality of nucleic acid targets, the capture probe to its corresponding nucleic acid target, when present in the cell; capturing, for each of the plurality of nucleic acid targets, the label probe to the capture probe, thereby capturing the label probe to its corresponding nucleic acid target; and detecting the signal from the label of the label probes captured to the plurality of nucleic acid targets.

The class of nucleic acid targets may consist of high risk human Papillomavirus (HPV). Specifically, the high risk HPV may comprise HPV subtypes of: HPV16, 18, 26, 31, 33, 35, 39, 45, 51, 52, 53, 56, 58, 59, 66, 68, 73, and 82.

In another embodiment, the plurality of nucleic acid targets comprise house-keeping genes of different tissues in a particular species. In a specific embodiment, the species is a human.

Essentially all of the features noted for the above embodiments relating to method of detecting individual cell of a specific type apply to these embodiments of method of detecting a class of nucleic acid targets as well, as relevant; for example, with respect to inclusion of capture probes, configuration and number of the label and/or capture probes, indistinguishable labels, inclusion of preamplifiers and/or amplifiers, inclusion of amplification reagents, type of nucleic acid target, location of various targets on a single molecule or on different molecules, type of labels, various hybridization temperature schemes, capture probes that all have the T section 5' of the L sections or all have the T section 3' of the L sections, sample processing methods, and/or the like.

### BRIEF DESCRIPTION OF THE DRAWINGS

The file of this patent contains at least one drawing executed in color. Copies of this patent with color drawing(s) will be provided by the Patent and Trademark Office upon request and payment of the necessary fee.
**Figure 1** schematically illustrates QMAGEX technology workflow for an exemplary embodiment.
**Figure 2** schematically illustrates a direct labeling approach in which label probes are hybridized to the target nucleic acid.
**Figure 3** schematically illustrates an indirect labeling approach in which label probes are hybridized to capture probes hybridized to the target nucleic acid.
**Figure 4** schematically illustrates an indirect labeling capture probe design approach that utilizes a pair of independent capture probes to enhance the specificity of the label probe capture to the target nucleic acid.
**Figure 5** schematically illustrates an indirect labeling capture probe design approach that utilizes three or more independent capture probes to enhance the specificity of the label probe capture to the target nucleic acid.
**Figure 6** schematically illustrates probe design approaches to detect multiple target molecules in parallel using either direct labeling (**Panel A**) or indirect labeling with two independent capture probes (**Panel B**).

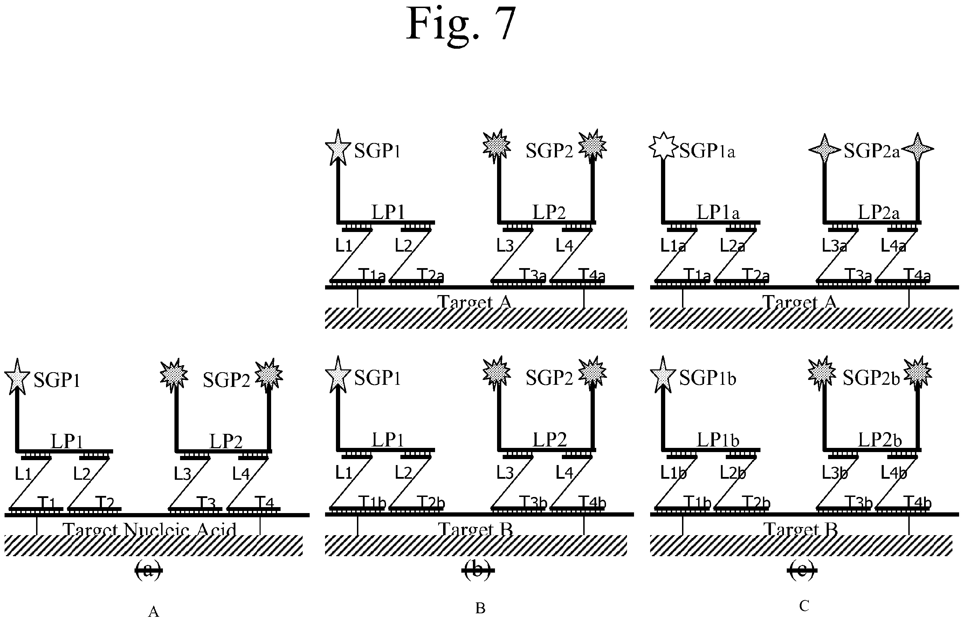

**Figure 7** schematically illustrates probe design approaches to reducing false positive rates in rare cell identification by attaching multiple types of signal-generating particles (labels) to the same target molecule. **Panel A** shows multiple types of signal-generating particles (labels) on one target. **Panel B** shows multiple types of signal-generating particles (labels) on more than one target, where the relative signal strengths of the particle set are maintained across all targets. **Panel C** shows a set of signal-generating particles (labels) on a target molecule, where different targets have distinctively different sets.
**Figure 8** schematically illustrate a multiplex assay for two nucleic acids in cells in suspension.
**Figure 9** **Panels A-D** schematically illustrate different structures of exemplary amplifiers.
**Figure 10** schematically illustrates utilizing rolling circle amplification to amplify signal. As shown in **Panel A,** a circular nucleotide molecule is attached to capture probe(s). As shown in **Panel B,** a long chain molecule with many repeated sequences appears as a result of rolling circle amplification. As shown in **Panel C,** many signal probes can be hybridized to the repeated sequences to achieve signal amplification.
**Figure 11** schematically illustrates one embodiment of the assay instrument configuration.
**Figure 12** **Panels A-E** illustrate detection of 18S RNA in HeLa cells using the 16XAMP2 system (**Panel A**) versus controls using the 1XAMP3 system (**Panel B**), capture probes complementary to the antisense strand (**Panel C**), and half of the capture probe set (Panels D and E).
**Figure 13** **Panels A-D** illustrate multiplex detection of 18S RNA and Her-2 mRNA in HeLa cells (**Panels A and C**) and SKBR3 cells (**Panels B and D**). Panels C-D represent a control experiment, in which capture probes targeting the anti-sense strand of the Her-2 intron sequence were used.
**Figure 14** presents a graph comparing Alexa488 and Fast Red detection.
**Figure 15** **Panels A-D** illustrate detection of changes in expression of IL-6 and IL-8 in single cells. Resting HeLa cells are shown in Panels A-B and PMA-treated cells in **Panels C-D.** Expression of IL-6 is shown in **Panels A and C** and expression of IL-8 is shown in **Panels B and D.**
**Figure 16** illustrates detection of cancer cells in mixed cell populations. **Panel A** illustrates detection of SKBR3 cells mixed with Jurkat cells. **Panel B** illustrates detection of BT474 breast cancer cells mixed with blood cells.
**Figure 17** illustrates detection in suspended HeLa cells. **Panel A** shows cells not hybridized with capture probes or signal amplifiers. **Panel B** shows cells hybridized with 18S capture probes and a 1XAMP3 system. **Panel C** shows cells hybridized with 18S capture probes and a 16XAMP2 system. **Panel D** shows a corresponding flow cytometric histogram.
**Figure 18** presents a flow cytometric histogram illustrating detection of low copy mRNAs.
**Figure 19** Panels A-I schematically illustrate different capture probe configurations. The solid horizontal line represents the target nucleic acid, and the dashed horizontal line represents a label probe, amplifier, or preamplifier.
**Figure 20** illustrates specific detection of a splice variant. Binding of two capture probes to the splice variant results in its detection (Panel A). Another variant, to which only one of the two capture probes binds, is not detected (Panel B).
**Figure 21** illustrates specific detection of a splice variant through capture of two different labels to different regions of the variant.
**Figure 22** **Panels A-D** illustrate MAGEX detection of mRNAs in breast cancer FFPE tissue section: 18S in **Panel A,** β-actin in **Panel B,** Ck19 in **Panel C,** and control 18S intron in **Panel D.** Sections shown in **Panels A-D** are also stained with DAPI.

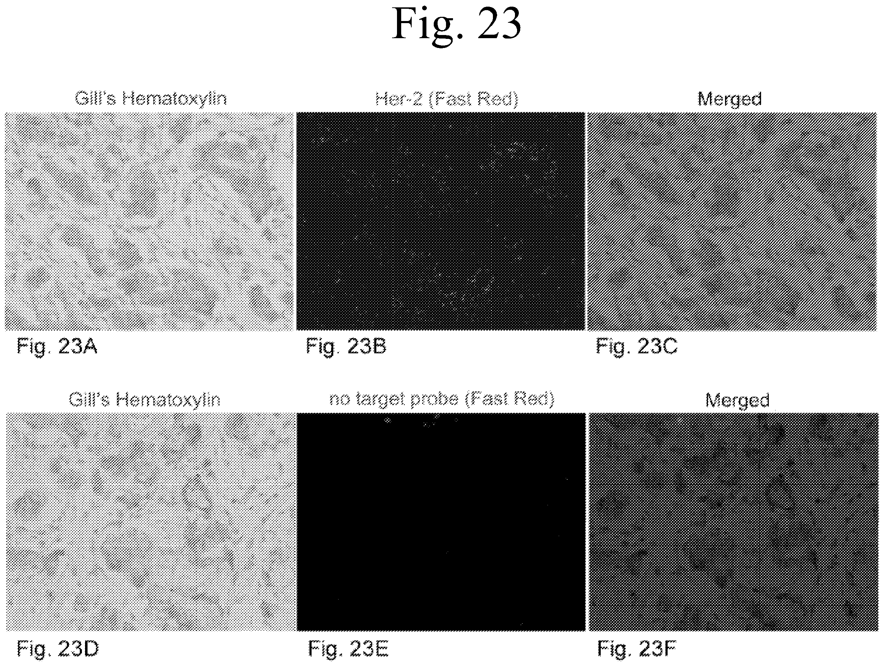

**Figure 23** **Panels A-F** illustrate detection of a low copy mRNA in breast cancer FFPE tissue sections. Detection of Her-2 is shown in **Panels A-C;** Panel A shows Gill's Hematoxylin staining of cell nuclei, **Panel B** shows detection of Her-2 mRNA using a MAGEX assay with a probe set for Her-2 and Fast Red substrate, and **Panel C** shows a merged picture for Her-2 and Gill's Hematoxylin. A control in which no target probe was employed is shown in **Panels D-F;** Panel D shows Gill's Hematoxylin staining of cell nuclei, **Panel E** shows detection using Fast Red (but no target probe), and **Panel F** shows a merged picture for Her-2 and Gill's Hematoxylin.
**Figure 24** **Panels A-I** illustrate detection of an mRNA in tissue microarray. Panels A-C show Gill's Hematoxylin staining of cell nuclei in the tissue sections. **Panels D-F** show the tissue sections labeled with a MAGEX assay using probes against CK19 (**Panel D**), Her-2 (**Panel F**), or a control with no probe (**Panel E**). **Panels G-I** show merged pictures for CK19 and Gill's Hematoxylin (**Panel G**), Her-2 and Gill's Hematoxylin (**Panel I**), and no probe control and Gill's Hematoxylin (**Panel H**).
**Figure 25** **Panels A-D** schematically illustrate identification of CTCs in blood samples from four different breast cancer patients. Staining is Fast Red (for CK19) and DAPI.
**Figure 26** **Panels A-B** schematically illustrate additional different label probe configurations in situ. **Panel A** shows a large label probe comprises multiple label particles or molecules captured by a capture probe. **Panel B** shows a single large label probe captured by each of two or more capture probes.
**Figure 27** **Panels A-B** schematically illustrate additional different capture probe configurations in situ. **Panel A** shows one capture probe captures multiple label probes, or amplifiers or preamplifiers. **Panel B** shows two or more capture probes jointly capture multiple labels, or amplifiers or preamplifiers.
**Figure 28** shows the experimental validation data of nine RNA markers (CK8, CK14, CK17, CK18, CK19, CK20, EpCAM, Muc1 and EGFR) selected to identify epithelial-type CTCs.
**Figure 29** shows the experimental validation data of three RNA markers (Twist, N-Cadherin and Fibronectin) selected to identify EMT CTCs.
**Figure 30** shows the comparative signals from spiked in tumor cells using CK19, pan-CK and pan-CTC marker groups.

Schematic figures are not necessarily to scale.

### DEFINITIONS

Unless defined otherwise, all technical and scientific terms used herein have the same meaning as commonly understood by one of ordinary skill in the art to which the invention pertains. The following definitions supplement those in the art and are directed to the current application and are not to be imputed to any related or unrelated case, e.g., to any commonly owned patent or application. Although any methods and materials similar or equivalent to those described herein can be used in the practice for testing of the present invention, the preferred materials and methods are described herein. Accordingly, the terminology used herein is for the purpose of describing particular embodiments only, and is not intended to be limiting.

As used in this specification and the appended claims, the singular forms "a," "an" and "the" include plural referents unless the context clearly dictates otherwise. Thus, for example, reference to "a molecule" includes a plurality of such molecules, and the like.

The term "about" as used herein indicates the value of a given quantity varies by +/-10% of the value, or optionally +/- 5% of the value, or in some embodiments, by +/- 1% of the value so described.

The term "polynucleotide" (and the equivalent term "nucleic acid") encompasses any physical string of monomer units that can be corresponded to a string of nucleotides, including a polymer of nucleotides (e.g., a typical DNA or RNA polymer), peptide nucleic acids (PNAs), modified oligonucleotides (e.g., oligonucleotides comprising nucleotides that are not typical to biological RNA or DNA, such as 2'-O-methylated oligonucleotides), and the like. The nucleotides of the polynucleotide can be deoxyribonucleotides, ribonucleotides or nucleotide analogs, can be natural or non-natural, and can be unsubstituted, unmodified, substituted or modified. The nucleotides can be linked by phosphodiester bonds, or by phosphorothioate linkages, methylphosphonate linkages, boranophosphate linkages, or the like. The polynucleotide can additionally comprise non-nucleotide elements such as labels, quenchers, blocking groups, or the like. The polynucleotide can be, e.g., single-stranded or double-stranded.

A "nucleic acid target" or "target nucleic acid" refers to a nucleic acid, or optionally a region thereof, that is to be detected.

A "polynucleotide sequence" or "nucleotide sequence" is a polymer of nucleotides (an oligonucleotide, a DNA, a nucleic acid, etc.) or a character string representing a nucleotide polymer, depending on context. From any specified polynucleotide sequence, either the given nucleic acid or the complementary polynucleotide sequence (e.g., the complementary nucleic acid) can be determined.

The term "gene" is used broadly to refer to any nucleic acid associated with a biological function. Genes typically include coding sequences and/or the regulatory sequences required for expression of such coding sequences. The term gene can apply to a specific genomic sequence, as well as to a cDNA or an mRNA encoded by that genomic sequence. Genes also include non-expressed nucleic acid segments that, for example, form recognition sequences for other proteins. Non-expressed regulatory sequences include promoters and enhancers, to which regulatory proteins such as transcription factors bind, resulting in transcription of adjacent or nearby sequences.

Two polynucleotides "hybridize" when they associate to form a stable duplex, e.g., under relevant assay conditions. Nucleic acids hybridize due to a variety of well characterized physico-chemical forces, such as hydrogen bonding, solvent exclusion, base stacking and the like. An extensive guide to the hybridization of nucleic acids is found in Tijssen (1993) Laboratory Techniques in Biochemistry and Molecular Biology-Hybridization with Nucleic Acid Probes, part I chapter 2, "Overview of principles of hybridization and the strategy of nucleic acid probe assays" (Elsevier, New York), as well as in Ausubel, infra.

A first polynucleotide "capable of hybridizing" to a second polynucleotide contains a first polynucleotide sequence that is complementary to a second polynucleotide sequence in the second polynucleotide. The first and second polynucleotides are able to form a stable duplex, e.g., under relevant assay conditions.

The "Tm" (melting temperature) of a nucleic acid duplex under specified conditions (e.g., relevant assay conditions) is the temperature at which half of the base pairs in a population of the duplex are disassociated and half are associated. The Tm for a particular duplex can be calculated and/or measured, e.g., by obtaining a thermal denaturation curve for the duplex (where the Tm is the temperature corresponding to the midpoint in the observed transition from double-stranded to single-stranded form).

The term "complementary" refers to a polynucleotide that forms a stable duplex with its "complement," e.g., under relevant assay conditions. Typically, two polynucleotide sequences that are complementary to each other have mismatches at less than about 20% of the bases, at less than about 10% of the bases, preferably at less than about 5% of the bases, and more preferably have no mismatches.

A "label" is a moiety that facilitates detection of a molecule. Common labels in the context of the present invention include fluorescent, luminescent, light-scattering, radioactive and/or colorimetric labels. Suitable labels include enzymes and fluorescent moieties, as well as radionuclides, substrates, cofactors, inhibitors, chemiluminescent moieties, magnetic particles, quantum dots and the like. Patents teaching the use of such labels include U.S. Pat. Nos. 3,817,837; 3,850,752; 3,939,350; 3,996,345; 4,277,437; 4,275,149; and 4,366,241. Many labels are commercially available and can be used in the context of the invention.

The term "label probe" refers to an entity that binds to a target molecule, directly or indirectly, and enables the target to be detected, e.g., by a readout instrument. A label probe (or "LP") is typically a single-stranded polynucleotide that comprises one or more label which directly or indirectly provides a detectable signal. The label can be covalently attached to the polynucleotide, or the polynucleotide can be configured to bind to the label (e.g., a biotinylated polynucleotide can bind a streptavidin-associated label). The label probe can, for example, hybridize directly to a target nucleic acid, or it can hybridize to a nucleic acid that is in turn hybridized to the target nucleic acid or to one or more other nucleic acids that are hybridized to the nucleic acid. Thus, the label probe can comprise a polynucleotide sequence that is complementary to a polynucleotide sequence of the target nucleic acid, or it can comprise at least one polynucleotide sequence that is complementary to a polynucleotide sequence in a capture probe, amplifier, or the like.

A "capture probe" is a polynucleotide that is capable of hybridizing to a target nucleic acid and capturing one or more label probe(s) to that target nucleic acid. The capture probe can hybridize directly to the label probe(s), or it can hybridize to one or more nucleic acids that in turn hybridize to the label probe; for example, the capture probe can hybridize to an amplifier or a preamplifier. The capture probe thus includes a first polynucleotide sequence that is complementary to a polynucleotide sequence of the target nucleic acid and a second polynucleotide sequence that is complementary to a polynucleotide sequence of the label probe, amplifier, preamplifier, or the like. The second polynucleotide sequence may also comprise multiple sections of identical or different sequences. The capture probe is preferably single-stranded.

An "amplifier" is a molecule, typically a polynucleotide, that is capable of hybridizing to multiple label probes. Typically, the amplifier hybridizes to multiple identical label probes. The amplifier also hybridizes to at least one capture probe or nucleic acid bound to a capture probe. For example, the amplifier can hybridize to at least one capture probe and to a plurality of label probes, or to a preamplifier and a plurality of label probes. The amplifier can be, e.g., a linear, forked, comb-like, or branched nucleic acid. As noted for all polynucleotides, the amplifier can include modified nucleotides and/or nonstandard internucleotide linkages as well as standard deoxyribonucleotides, ribonucleotides, and/or phosphodiester bonds. Suitable amplifiers are described, for example, in USPN 5,635,352, USPN 5,124,246, USPN 5,710,264, and USPN 5,849,481.

A "preamplifier" is a molecule, typically a polynucleotide, that serves as an intermediate between one or more capture probes and amplifiers. Typically, the preamplifier hybridizes simultaneously to one or more capture probes and to a plurality of amplifiers. Exemplary preamplifiers are described, for example, in USPN 5,635,352 and USPN 5,681,697.

A "pathogen" is a biological agent, typically a microorganism, that causes disease or illness to its host.

A "microorganism" is an organism of microscopic or submicroscopic size. Examples include, but are not limited to, bacteria, fungi, yeast, protozoans, microscopic algae (e.g., unicellular algae), viruses (which are typically included in this category although they are incapable of growth and reproduction outside of host cells), subviral agents, viroids, and mycoplasma.

A variety of additional terms are defined or otherwise characterized herein.

### DETAILED DESCRIPTION

Detection of nucleic acid analytes in biological samples can be broadly categorized into two types of methods: "whole-sample" and "in situ" detection. In the whole-sample detection method, the cells in the sample are lysed, which releases the molecules contained in the cells, including the nucleic acid analytes, into sample solution. Then the quantities of the nucleic acid analytes of the entire biological sample are measured in the solution. In the in situ detection method, the nucleic acid analytes are fixed within the host cells and their quantities are measured at an individual cell level. The methods of the instant invention concern in situ detection.

In situ detection of nucleic acid analytes is highly desirable for two major reasons. First, biological samples are usually heterogeneous, e.g., containing different types of cells where only a sub-population of the cells is disease relevant. Early in the onset of disease, the fraction of cells in the sample that are affected by the disease can be very small. Since many nucleic acid analytes that serve as disease markers exist not only in disease cells but also in normal cells, albeit at different levels, in such instances a whole-sample detection approach can distort measurement results. This problem is particularly acute if the disease cell population represents a tiny fraction of the cells in the sample. The second reason is that in situ detection maintains cell morphology and/or tissue structure intact. The fusion of information provided by molecular disease markers and cell morphology and/or tissue structure may yield additional scientific or clinical diagnostic value.

Fluorescent In Situ Hybridization (FISH) is a well established method of localizing and detecting DNA sequences in morphologically preserved tissue sections or cell preparations (Pinkel et al., 1986). The FISH assay typically employs specially constructed DNA probes, which are directly labeled with fluorescent dyes and collectively cover about 100,000 nucleotides per target. The methods described herein can also be adapted to detect and localize DNA sequences in situ, although they can employ signal amplification to add hundreds of fluorescent labels per probe pair that hybridizes to approximately 50 bases of target sequence. As a result, the base pair detection resolution is in the order of one thousand nucleotides or less, i.e. over one hundred times better than that of traditional FISH. In addition, unique features in the probe set design can significantly improve hybridization specificity, which facilitates easy multiplexing and improves signal-to-noise ratios. Use of synthetic oligos also brings the benefit of product scalability and quality consistency.

Similar in situ hybridization techniques, which are generally referred to as "ISH" technology, have been used to detect mRNA within individual cells (Hicks et al., 2004). There are four main types of probes that are typically used in performing ISH: oligonucleotide probes (usually 20-40 bases in length), single-stranded DNA probes (200-500 bases in length), double stranded DNA probes, or RNA probes (200-5000 bases in length). RNA probes are currently the most widely used probes for in situ hybridization as they have the advantage that RNA-RNA hybrids are very thermostable and are resistant to digestion by RNases. However, RNA probe is a direct labeling method that suffers a number of difficulties. First, separate labeled probes have to be prepared for detecting each mRNA of interest. Second, it is technically difficult to detect the expression of multiple mRNAs of interest in situ at the same time. As a result, only sequential detection of multiple mRNAs using different labeling methods has recently been reported (Schrock et al, 1996; Kosman et al, 2004). Furthermore, with direct labeling methods, there is no good way to control for potential cross-hybridization with non-specific sequences in cells. In short, the detection sensitivity of traditional ISH is limited to 10 - 20 mRNA copies per cell. In fact, there is currently no commercial ISH products available that can reliably detect mRNA below 50 copies per cell. This is a major handicap for the use of traditional ISH in diagnostics because more than 95% of human genes express at a level below 50 copies per cell (Zhang et al. 1997) and many of the detectable human genes that are high expressors are constitutively expressed house-keeping genes of less diagnostic interest.

A new type of in situ hybridization method employing Branched DNA (bDNA) has recently been developed for detecting mRNA in single cells (Player et al, 2001). This method uses a series of oligonucleotide probes that have one portion hybridizing to the specific mRNA of interest and another portion hybridizing to the bDNA for signal amplification and detection. bDNA ISH has the advantages that unlabeled oligonucleotide probes are used for detecting every mRNA of interest and that the signal amplification and detection reagents are generic components in the assay. However, the amplifier used is typical bDNA assays has large, complex molecular structure. The experience of authors of this invention in in-situ assays indicates that such large molecules prone to congregation in cell, which increases background noise and produces false positive signals. This problem is particularly serious in tissue section samples. In addition, nonspecific hybridization of the oligonucleotide probes in bDNA ISH can become a serious problem when multiple of those probes have to be used for the detection of a low abundance mRNA. Some of the probes may hybridize to unintended sequences, leading to signal amplification of the background, thus reducing detection sensitivity. Similarly, although use of bDNA ISH to detect or quantitate multiple mRNAs is desirable, such nonspecific hybridization of the oligonucleotide probes is a potential problem.

Among other benefits, methods of the present invention overcome the above noted difficulties and provide unique mechanisms for background noise reduction and for improving detection sensitivity and specificity. As a result, they are capable of reliable detection of nucleic acid targets within individual cells at a sensitivity well below 50 copies per cell in a wide range of biological sample types, including, e.g., FFPE tissue sections. In addition, the methods of the present invention are particularly useful for identifying rare cells in a sample with mixed cell populations. Important exemplary applications include, but are not limited to, the detection of circulating tumor cells (CTC) in blood or other bodily fluids, detection of tumor cells in solid tissue sections, detection of cancer stem cells in solid tumor sections or in bodily fluids such as blood, and detection of fetal cells in maternal blood.

Among other aspects, the present disclosure provides multiplex assays that can be used for simultaneous detection, and optionally quantitation, of two or more nucleic acid targets in a single cell. The methods may be used for detecting the level of one or more target nucleic acids, e.g., absolute or relative to that of a reference nucleic acid in an individual cell.

The present invention provides a method of detecting an individual cell of a specified type in a sample comprising a mixture of cell types, which mixture comprises at least one cell of the specified type, wherein the cell comprises a first nucleic acid target and a second nucleic acid target, wherein the first and second nucleic acid targets always co-exist in the cell, the method comprising:
(a) providing a first label probe comprising a first label, and providing a second label probe comprising a second label, wherein a first signal from the first label is indistinguishable from a second signal from the second label;
(b) capturing, in the cell, the first label probe to the first nucleic acid target, when present in the cell, and the second label probe to the second nucleic acid target, when present in the cell, wherein the first and second nucleic acid targets are separate molecules, wherein the capturing comprises:
   providing for the first nucleic acid target at least a first capture probe and providing for the second nucleic acid target at least a second capture probe;
   capturing the first label probe to the first nucleic acid target,
   comprising hybridizing in the cell the first capture probe to the first target nucleic acid, and hybridizing the first label probe to the first capture probe, thereby capturing the first label probe to the first nucleic acid target; and
   capturing the second label probe to the second nucleic acid target, comprising hybridizing in the cell the second capture probe to the second target nucleic acid, and hybridizing the second label probe to the second capture probe, thereby capturing the second label probe to the second nucleic acid target;
(c) detecting the signal from the labels;
(d) correlating the signal detected from the cell with the presence or amount of the first and second nucleic acid targets in the cell; and
(e) identifying the cell as being of the specified type based on detection of the presence or amount of the first and second nucleic acid targets within the cell, wherein the specified type of cell is distinguishable from the other cell type(s) in the mixture on the basis of either the presence or amount of the first nucleic acid target and the presence or amount of the second nucleic acid target in the cell.

According to some embodiments of the invention, providing at least a first capture probe comprises providing two or more different first capture probes as a set, wherein providing at least a second capture probe comprises providing two or more different second capture probes as a set; wherein capturing the first label probe to the first nucleic acid target comprises hybridizing in the cell the two or more different first capture probes to the first target nucleic acid, and hybridizing the first label probe to the two or more different first capture probes, thereby capturing the first label probe to the first nucleic acid target; wherein capturing the second label probe to the second nucleic acid target comprises hybridizing in the cell the two or more different second capture probes to the second target nucleic acid, and hybridizing the second label probe to the two or more different second capture probes, thereby capturing the second label probe to the second nucleic acid target.

According to some embodiments of the invention, providing at least a first capture probe comprises providing two or more different first capture probes as a set; wherein providing at least a second capture probe comprises providing two or more different second capture probes as a set; wherein the method further comprises providing an amplifier, wherein amplifier is a molecule capable of hybridizing to multiple label probes; wherein capturing the first label probe to the first nucleic acid target comprises hybridizing in the cell the first target nucleic acid to the two or more different first capture probes, hybridizing the two or more different first capture probes to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the amplifier, and hybridizing the amplifier to the second label probe, thereby capturing the second label probe to the second nucleic acid target.

According to some embodiments of the invention, providing at least a first capture probe comprises providing two or more different first capture probes as a set; wherein providing at least a second capture probe comprises providing two or more different second capture probes as a set; wherein the method further comprises providing an amplifier and a preamplifier; wherein capturing the first label probe to the first nucleic acid target comprises hybridizing in the cell the first target nucleic acid to the two or more different first capture probes, hybridizing the two or more different first capture probes to the preamplifier, hybridizing the preamplifier to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the preamplifier, hybridizing the preamplifier to the amplifier, and hybridizing the amplifier to the second label probe, thereby capturing the second label probe to the second nucleic acid target.

According to some embodiments of the invention, hybridizing the two or more different capture probes to the label probe, amplifier, or preamplifier is performed at a hybridization temperature that is greater than a melting temperature Tm of a complex between each individual capture probe and the label probe, amplifier, or preamplifier.

According to some embodiments of the invention, the two or more different capture probes hybridize to unique and adjacent sections on the nucleic acid target.

According to some embodiments of the invention, hybridizing the two or more different capture probes to their corresponding nucleic acid target is performed at a hybridization temperature that is lower than a melting temperature Tm of a complex between each individual capture probe and the nucleic acid target, or
wherein hybridizing the two or more different capture probes to their corresponding nucleic acid target is performed at a hybridization temperature that is greater than a melting temperature Tm of a complex between each individual capture probe and the nucleic acid target.

According to some embodiments of the invention, the method further comprising providing a plurality of additional nucleic acid targets; wherein the method further comprises: providing a plurality of label probes each comprising a label, wherein the signal from each label is indistinguishable from the first and second signals or from each other; providing at least one capture probe for each of the additional nucleic acid targets, hybridizing in the cell each capture probe to its corresponding nucleic acid target, when present in the cell, and hybridizing each label probe to its corresponding capture probe, and detecting the signal from the labels.

According to some embodiments of the invention, the first nucleic acid target and the second nucleic acid target are independently selected from the group consisting of: a DNA, an RNA, a chromosomal DNA, an mRNA, a nucleic acid endogenous to the cell, a nucleic acid introduced to or expressed in the cell by infection of the cell with a pathogen and a cytoplasmic RNA.

According to some embodiments of the invention,
(i) the first nucleic acid target is a first mRNA and wherein the second nucleic acid target is a second mRNA; or
(ii) the first nucleic acid target comprises a first chromosomal DNA polynucleotide sequence and wherein the second nucleic acid target comprises a second chromosomal DNA polynucleotide sequence; or
(iii) the first nucleic acid target and/or the second nucleic acid target comprises a double-stranded DNA molecule, the method comprising denaturing the double-stranded DNA prior to hybridization of the first and second capture probes to the first and second nucleic acid targets.

According to some embodiments of the invention,
(i) about 1000 copies or less of the first nucleic acid target and/or about 1000 copies or less of the second nucleic acid target are present in the cell; or
(ii) about 100 copies or less of the first nucleic acid target and/or about 100 copies or less of the second nucleic acid target are present in the cell; or
(iii) about 10 copies or less of the first nucleic acid target and/or about 10 copies or less of the second nucleic acid target are present in the cell; or
(iv) about 5 copies or less of the first nucleic acid target and/or about 5 copies or less of the second nucleic acid target are present in the cell; or
(v) a single copy of the first nucleic acid target and/or a single copy of the second nucleic acid target is present in the cell.

According to some embodiments of the invention, the first nucleic acid target, the second nucleic acid target, and/or the additional nucleic acid target is selected from the group consisting of: CK8, CK14, CK17, CK18, CK19, CK20, EpCAM, MucI, EGFR, Twist, N-Cadherin and Fibronectin.

Further, the present invention provides a method of detecting the presence or absence of a plurality of nucleic acid targets in an individual cell in a sample comprising the cell, which cell comprises or is suspected of comprising said plurality of nucleic acid targets, wherein said nucleic acid targets always co-exist in the cell, the method comprising:
(a) providing, for each of the plurality of nucleic acid targets, a label probe comprising a label, wherein a signal from the label of a first nucleic acid target in the plurality of nucleic acid targets is indistinguishable from a signal from the label of a second nucleic acid target in the plurality of nucleic acid targets, wherein the first and the second nucleic acid targets are separate molecules;
(b) providing, for each of the plurality of nucleic acid targets, at least a capture probe;
(c) hybridizing in the cell, for each of the plurality of nucleic acid targets, the capture probe to its corresponding nucleic acid target, when present in the cell;
(d) capturing, for each of the plurality of nucleic acid targets, the label probe to the capture probe, thereby capturing the label probe to its corresponding nucleic acid target; and
(e) detecting the signal from the label of the label probes captured to the plurality of nucleic acid targets,
wherein the presence of signal indicates the presence of a member of the plurality of nucleic acid targets and the absence of signal indicates the absence of the nucleic acid targets in the cell.

According to some embodiments of the invention, the plurality of nucleic acid targets comprise housekeeping genes of different tissues in a particular species, wherein the species is preferably a human.

According to some embodiments of the invention,
(i) providing at least a capture probe comprises providing two or more capture probes; wherein each of the two or more capture probes comprises a T section which is complementary to a region of its corresponding nucleic acid target and a L section which is complementary to a region of its corresponding label probe; further, the T sections of two or more capture probes are complementary to non-overlapping regions of the nucleic acid target and the L sections of the two or more capture probes are complementary to non-overlapping regions of the label probe, and wherein the method comprises hybridizing a label probe to the two or more capture probes;
(ii) providing at least a capture probe comprises providing two or more capture probes; wherein the method further comprises providing an amplifier; and wherein the method comprises hybridizing a label probe to an amplifier and hybridizing the amplifier to said two or more capture probes; or
(iii) providing at least a capture probe comprises providing two or more capture probes; wherein the method further comprises providing an amplifier and a preamplifier; and wherein the method comprises hybridizing the preamplifier to the two or more capture probes, hybridizing the amplifier to the preamplifier and hybridizing a label probe to the amplifier.

According to some embodiments of the invention, the method comprises the step of hybridizing each set of two or more capture probes to the corresponding target nucleic acid at a hybridization temperature (a) greater than the melting temperature of each T section of two or more capture probes in the set, or (b) greater than the melting temperature of each L section of two or more capture probes in the set.

According to some embodiments of the invention, the method comprises the step of hybridizing each set of two or more capture probes to the corresponding target nucleic acid at a hybridization temperature (a) greater than the melting temperature of each T section of two or more capture probes in the set and lower than the melting temperature of each L section of two or more capture probes in the set, or (b) greater than the melting temperature of each L section of two or more capture probes in the set and lower than the melting temperature of each T section of two or more capture probes in the set.

According to some embodiments of the invention, the plurality of nucleic acid targets are independently selected from the group consisting of: a DNA, an RNA, a chromosomal DNA, an mRNA, a nucleic acid endogenous to the cell, a nucleic acid introduced to or expressed in the cell by infection of the cell with a pathogen and cytoplasmic RNAs.

According to some embodiments of the invention, the two or more capture probes in each set all have the T sections 5' of the L sections, or wherein the two or more capture probes in each set all have the T sections 3' of the L sections.

According to some embodiments of the invention, each hybridization or capture step is accomplished for all of the nucleic acid targets at the same time.

According to some embodiments of the invention, the cell is a circulating tumor cell.

According to some embodiments of the invention, the cell is in suspension in the sample comprising the cell, and/or wherein the cell is in suspension during the hybridizing, capturing, and/or detecting steps.

According to some embodiments of the invention,the sample comprising the cell is derived from a bodily fluid, blood, or a tissue section.

According to some embodiments of the invention, providing a sample comprises fixing and permeabilizing the cell.

According to some embodiments of the invention, the method comprises washing the cell to remove materials not captured to one of the nucleic acid targets.

According to some embodiments of the invention, detecting the signal comprises performing flow cytometry.

In general, in the assays, a label probe is captured to each target nucleic acid. The label probe can be captured to the target through direct binding of the label probe to the target. Preferably, however, the label probe is captured indirectly through binding to capture probes, amplifiers, and/or preamplifiers that bind to the target. Use of the optional amplifiers and preamplifiers facilitates capture of multiple copies of the label probe to the target, thus amplifying signal from the target without requiring enzymatic amplification of the target itself. Preferably, label probes, capture probes, amplifiers and preamplifiers used in the invented method all have simple structure and relatively small molecule size. Therefore, unbound molecules can be easily washed away, thus reducing background and the chance of false positive signal. In one preferred embodiment, label probes, capture probes, amplifiers and preamplifiers are all single-stranded. Binding of the capture probes is optionally cooperative, reducing background caused by undesired cross hybridization of capture probes to non-target nucleic acids (a greater problem in multiplex assays than singleplex assays since more probes must be used in multiplex assays, increasing the likelihood of cross hybridization).

One aspect of the invention relates to detection of single cells, including detection of rare cells from a heterogeneous mixture of cells, e.g., in suspension, bound to solid surface or in solid tissue samples. Individual cells are detected through detection of nucleic acids whose presence, absence, copy number, or the like are characteristic of the cell.

Compositions, kits, and systems related to the methods are also disclosed.

### METHODS OF DETECTING NUCLEIC ACIDS AND CELLS

### Detection of nucleic acids in cell

As noted, disclosed herein are nucleic acid assays in single cells. Thus, a first general class of aspects of the disclosure includes methods of detecting one or more nucleic acid targets in an individual cell. In the methods, a sample comprising the cell is provided. The cell comprises, or is suspected of comprising, a nucleic acid target. A label probe and a capture probe are also provided. The label probe may comprise a single label particle or molecule that provide detectable signal. The label probe may have a larger molecular structure enabling that attachment of a plurality of label particle or molecules that provide stronger signal than a single label particle or molecule.

The capture probe is hybridized, in the cell, to the nucleic acid target (when the nucleic acid target is present in the cell). The label probe is captured to the capture probe, thereby capturing the label probe to the nucleic acid target. The signal from the label is then detected. Since the label is associated with its respective nucleic acid target through the capture probes, presence of the label in the cell indicates the presence of the corresponding nucleic acid target in the cell. The methods are optionally quantitative. Thus, an intensity of the signal can be measured and correlated with a quantity of the nucleic acid target in the cell. As another example, a signal spot can be counted for each copy of the nucleic acid target to quantify them.

In one aspect, the label probes bind directly to the capture probes. For example, in one class of embodiments, one capture probe and one label probe are provided. The label probe is hybridized to the capture probe and the capture probe is hybridized to the target. In another class of embodiments, one capture probe and a plurality of label probe are provided. Multiple label probes are hybridized to the capture probe and the capture probe is hybridized to the target. In a different class of embodiments, two or more capture probes and a label probe are provided. The two or more capture probes are hybridized to the nucleic acid target. A single label probe is hybridized to each of the two or more capture probes. In yet another class of embodiments, two or more capture probes and a plurality of label probes are provided. The two or more capture probes are hybridized to the nucleic acid target. Each of the multiple label probes is hybridized to each of the two or more capture probes.

In another aspect, the label probes are captured to the capture probes indirectly, for example, through binding of preamplifiers and/or amplifiers. In one class of embodiments in which amplifiers are employed, a capture probe and a plurality of the label probes are provided. An amplifier is hybridized to the capture probe and to the plurality of label probes. In one related class of embodiments, a capture probe, a plurality of the label probes and multiple amplifiers are provided. The amplifiers are hybridized to the capture probe and to the plurality of label probes. In another class of embodiments, two or more capture probes, a plurality of the label probes are provided. The two or more capture probes are hybridized to the nucleic acid target. A single amplifier is hybridized to each of the capture probes, and the plurality of label probes is hybridized to the amplifier. In yet another class of embodiments, two or more capture probes, a plurality of the label probes and a plurality of the amplifier are provided. The two or more capture probes are hybridized to the nucleic acid target. Every single amplifier is hybridized to each of the capture probes, and the plurality of label probes is hybridized to the amplifiers.

In one class of embodiments in which preamplifiers are employed, a capture probe, a plurality of label probes and a plurality of amplifiers are provided. A preamplifier is hybridized to the capture probe, a plurality of amplifiers is hybridized to the preamplifier, and the plurality of label probes is hybridized to the amplifiers. In one related class of embodiments, a capture probe, a plurality of label probes, a plurality of amplifiers and a plurality of preamplifiers are provided. Multiple preamplifiers are hybridized to a single capture probe, a plurality of amplifiers is hybridized to the preamplifier, and the plurality of label probes is hybridized to the amplifiers. In another class of embodiments, two or more capture probes, a plurality of the label probes are provided. The two or more capture probes are hybridized to the nucleic acid target. A preamplifier is hybridized to each of the two or more capture probes, a plurality of amplifiers is hybridized to each of the preamplifiers, and the plurality of label probes is hybridized to the amplifiers. In yet another class of embodiments, two or more capture probes, a plurality of the label probes, a plurality of amplifiers and a plurality of preamplifiers are provided. The two or more capture probes are hybridized to the nucleic acid target. Every preamplifier are hybridized to each of the two or more capture probes, a plurality of amplifiers is hybridized to each of the preamplifiers, and the plurality of label probes is hybridized to the amplifiers.

In embodiments in which two or more capture probes are employed, the capture probes preferably hybridize to nonoverlapping polynucleotide sequences in their respective nucleic acid target. The capture probes can, but need not, cover a contiguous region of the nucleic acid target. Blocking probes, polynucleotides which hybridize to regions of the nucleic acid target not occupied by capture probes, are optionally provided and hybridized to the target. For a given nucleic acid target, the corresponding capture probes and blocking probes are preferably complementary to physically distinct, nonoverlapping sequences in the nucleic acid target, which nonoverlapping sequences are preferably, but not necessarily, contiguous. Having the capture probes and optional blocking probes be contiguous with each other can in some embodiments enhance hybridization strength, remove secondary structure, and ensure more consistent and reproducible signal.

In many embodiments, such as those above, enzymatic manipulation is not required to capture the label probes to the capture probes. In other embodiments, however, enzymatic manipulation, particularly amplification of nucleic acids intermediate between the capture probes and the label probes, facilitates detection of the nucleic acid targets. For example, in one class of embodiments, a plurality of the label probes is provided. A amplified polynucleotide is produced by rolling circle amplification of a circular polynucleotide hybridized to the capture probe. The circular polynucleotide comprises at least one copy of a polynucleotide sequence identical to a polynucleotide sequence in the label probe, and the amplified polynucleotide thus comprises a plurality of copies of a polynucleotide sequence complementary to the polynucleotide sequence in the label probe. The plurality of the label probes is then hybridized to the amplified polynucleotide. The amplified polynucleotides remain associated (e.g., covalently) with the capture probe(s), and the label probes are thus captured to the nucleic acid target. A circular polynucleotide can be provided and hybridized to the capture probe, or a linear polynucleotide that is circularized by ligation after it binds to the capture probe (e.g., a padlock probe) can be employed. Techniques for rolling circle amplification, including use of padlock probes, are well known in the art. See, e.g., Larsson et al. (2004) "In situ genotyping individual DNA molecules by target-primed rolling-circle amplification of padlock probes" Nat Methods. 1(3):227-32, Nilsson et al. (1994) Science 265:2085-2088, and Antson et al. (2000) "PCR-generated padlock probes detect single nucleotide variation in genomic DNA" Nucl Acids Res 28(12):E58.

Potential capture probe sequences are optionally examined for possible interactions with non-corresponding nucleic acid targets, the preamplifiers, the amplifiers, the label probes, and/or any relevant genomic sequences, for example. Sequences expected to cross-hybridize with undesired nucleic acids are typically not selected for use in the capture probes (but may be employed as blocking probes). Examination can be, e.g., visual (e.g., visual examination for complementarity), computational (e.g., a BLAST search of the relevant genomic database, or computation and comparison of binding free energies), and/or experimental (e.g., cross-hybridization experiments). Repetitive sequences are generally avoided. Label probe sequences are preferably similarly examined, to help minimize potential undesirable cross-hybridization.

A capture probe, preamplifier, amplifier, and/or label probe optionally comprises at least one non-natural nucleotide. For example, a capture probe and a preamplifier (or amplifier or label probe) that hybridizes to it optionally comprise, at complementary positions, at least one pair of non-natural nucleotides that base pair with each other but that do not Watson-Crick base pair with the bases typical to biological DNA or RNA (i.e., A, C, G, T, or U). Examples of nonnatural nucleotides include, but are not limited to, Locked NucleicAcidTM nucleotides (available from Exiqon A/S, www (dot) exiqon (dot) com; see, e.g., SantaLucia Jr. (1998) Proc Natl Acad Sci 95:1460-1465) and isoG, isoC, and other nucleotides used in the AEGIS system (Artificially Expanded Genetic Information System, available from EraGen Biosciences, www (dot) eragen (dot) com; see, e.g., USPN 6,001,983, USPN 6,037,120, and USPN 6,140,496). Use of such non-natural base pairs (e.g., isoG-isoC base pairs) in the probes can, for example, reduce background and/or simplify probe design by decreasing cross hybridization, or it can permit use of shorter probes when the non-natural base pairs have higher binding affinities than do natural base pairs.

### Multiplex detection of nucleic acids

As noted, the methods of the invention employ multiplex nucleic acid assays in single cells as defined in the claims. Thus, one general class of embodiments includes methods of detecting two or more nucleic acid targets in an individual cell. In the methods, a sample comprising the cell is provided. The cell comprises, or is suspected of comprising, a first nucleic acid target and a second nucleic acid target. A first label probe comprising a first label and a second label probe comprising a second label are provided. At least a first capture probe and at least a second capture probe are also provided.

The first capture probe is hybridized, in the cell, to the first nucleic acid target (when the first nucleic acid target is present in the cell), and the second capture probe is hybridized, in the cell, to the second nucleic acid target (when the second nucleic acid target is present in the cell). The first label probe is captured to the first capture probe and the second label probe is captured to the second capture probe, thereby capturing the first label probe to the first nucleic acid target and the second label probe to the second nucleic acid target. The first signal from the first label and the second signal from the second label are then detected. Since the first and second labels are associated with their respective nucleic acid targets through the capture probes, presence of the label(s) in the cell indicates the presence of the corresponding nucleic acid target(s) in the cell. The methods are optionally quantitative. Thus, an intensity of the first signal and an intensity of the second signal can be measured, and the intensity of the first signal can be correlated with a quantity of the first nucleic acid target in the cell while the intensity of the second signal is correlated with a quantity of the second nucleic acid target in the cell. As another example, a signal spot can be counted for each copy of the first and second nucleic acid targets to quantitate them, as described in greater detail below.

In one aspect, the label probes bind directly to the capture probes. For example, in one class of embodiments, a single first capture probe, a single second capture probe, a plurality of first label probes and a plurality of second label probes are provided, multiple first label probes is hybridized to the first capture probe, and multiple second label probe is hybridized to the second capture probe. In a related class of embodiments, two or more first capture probes and two or more second capture probes are provided, as are a plurality of the first label probes (e.g., two or more identical first label probes) and a plurality of the second label probes (e.g., two or more identical second label probes). The two or more first capture probes are hybridized to the first nucleic acid target, and the two or more second capture probes are hybridized to the second nucleic acid target. A single first label probe is hybridized to each of the first capture probes, and a single second label probe is hybridized to each of the second capture probes.

In another aspect, the label probes are captured to the capture probes indirectly, for example, through binding of preamplifiers and/or amplifiers. Use of amplifiers and preamplifiers can be advantageous in increasing signal strength, since they can facilitate binding of large numbers of label probes to each nucleic acid target.

In one class of embodiments in which amplifiers are employed, a single first capture probe, a single second capture probe, a plurality of the first label probes, and a plurality of the second label probes are provided. A first amplifier is hybridized to the first capture probe and to the plurality of first label probes, and a second amplifier is hybridized to the second capture probe and to the plurality of second label probes. In another class of embodiments, two or more first capture probes, two or more second capture probes, a plurality of the first label probes, and a plurality of the second label probes are provided. The two or more first capture probes are hybridized to the first nucleic acid target, and the two or more second capture probes are hybridized to the second nucleic acid target. A first amplifier is hybridized to each of the first capture probes, and the plurality of first label probes is hybridized to the first amplifiers. A second amplifier is hybridized to each of the second capture probes, and the plurality of second label probes is hybridized to the second amplifiers.

In one class of embodiments in which preamplifiers are employed, a single first capture probe, a single second capture probe, a plurality of the first label probes, and a plurality of the second label probes are provided. A first preamplifier is hybridized to the first capture probe, a plurality of first amplifiers is hybridized to the first preamplifier, and the plurality of first label probes is hybridized to the first amplifiers. A second preamplifier is hybridized to the second capture probe, a plurality of second amplifiers is hybridized to the second preamplifier, and the plurality of second label probes is hybridized to the second amplifiers. In another class of embodiments, two or more first capture probes, two or more second capture probes, a plurality of the first label probes, and a plurality of the second label probes are provided. The two or more first capture probes are hybridized to the first nucleic acid target, and the two or more second capture probes are hybridized to the second nucleic acid target. A first preamplifier is hybridized to each of the first capture probes, a plurality of first amplifiers is hybridized to each of the first preamplifiers, and the plurality of first label probes is hybridized to the first amplifiers. A second preamplifier is hybridized to each of the second capture probes, a plurality of second amplifiers is hybridized to each of the second preamplifiers, and the plurality of second label probes is hybridized to the second amplifiers. Optionally, additional preamplifiers can be used as intermediates between a preamplifier hybridized to the capture probe(s) and the amplifiers.

In the above classes of embodiments, one capture probe hybridizes to each label probe, amplifier, or preamplifier. In alternative classes of related embodiments, two or more capture probes hybridize to the label probe, amplifier, or preamplifier. See, e.g., the section below entitled "Implementation, applications, and advantages."

In embodiments in which two or more first capture probes and/or two or more second capture probes are employed, the capture probes preferably hybridize to nonoverlapping polynucleotide sequences in their respective nucleic acid target. The capture probes can, but need not, cover a contiguous region of the nucleic acid target. Blocking probes, polynucleotides which hybridize to regions of the nucleic acid target not occupied by capture probes, are optionally provided and hybridized to the target. For a given nucleic acid target, the corresponding capture probes and blocking probes are preferably complementary to physically distinct, nonoverlapping sequences in the nucleic acid target, which nonoverlapping sequences are preferably, but not necessarily, contiguous. Having the capture probes and optional blocking probes be contiguous with each other can in some embodiments enhance hybridization strength, remove secondary structure, and ensure more consistent and reproducible signal.

In many embodiments, such as those above, enzymatic manipulation is not required to capture the label probes to the capture probes. In other embodiments, however, enzymatic manipulation, particularly amplification of nucleic acids intermediate between the capture probes and the label probes, facilitates detection of the nucleic acid targets. For example, in one class of embodiments, a plurality of the first label probes and a plurality of the second label probes are provided. A first amplified polynucleotide is produced by rolling circle amplification of a first circular polynucleotide hybridized to the first capture probe. The first circular polynucleotide comprises at least one copy of a polynucleotide sequence identical to a polynucleotide sequence in the first label probe, and the first amplified polynucleotide thus comprises a plurality of copies of a polynucleotide sequence complementary to the polynucleotide sequence in the first label probe. The plurality of first label probes is then hybridized to the first amplified polynucleotide. Similarly, a second amplified polynucleotide is produced by rolling circle amplification of a second circular polynucleotide hybridized to the second capture probe (preferably, at the same time the first amplified polynucleotide is produced). The second circular polynucleotide comprises at least one copy of a polynucleotide sequence identical to a polynucleotide sequence in the second label probe, and the second amplified polynucleotide thus comprises a plurality of copies of a polynucleotide sequence complementary to the polynucleotide sequence in the second label probe. The plurality of second label probes is then hybridized to the second amplified polynucleotide. The amplified polynucleotides remain associated (e.g., covalently) with the capture probe(s), and the label probes are thus captured to the nucleic acid targets. A circular polynucleotide can be provided and hybridized to the capture probe, or a linear polynucleotide that is circularized by ligation after it binds to the capture probe (e.g., a padlock probe) can be employed. Techniques for rolling circle amplification, including use of padlock probes, are well known in the art. See, e.g., Larsson et al. (2004) "In situ genotyping individual DNA molecules by target-primed rolling-circle amplification of padlock probes" Nat Methods. 1(3):227-32, Nilsson et al. (1994) Science 265:2085-2088, and Antson et al. (2000) "PCR-generated padlock probes detect single nucleotide variation in genomic DNA" Nucl Acids Res 28(12):E58.

Potential capture probe sequences are optionally examined for possible interactions with non-corresponding nucleic acid targets, the preamplifiers, the amplifiers, the label probes, and/or any relevant genomic sequences, for example. Sequences expected to cross-hybridize with undesired nucleic acids are typically not selected for use in the capture probes (but may be employed as blocking probes). Examination can be, e.g., visual (e.g., visual examination for complementarity), computational (e.g., a BLAST search of the relevant genomic database, or computation and comparison of binding free energies), and/or experimental (e.g., cross-hybridization experiments). Repetitive sequences are generally avoided. Label probe sequences are preferably similarly examined, to help minimize potential undesirable cross-hybridization.

A capture probe, preamplifier, amplifier, and/or label probe optionally comprises at least one non-natural nucleotide. For example, a capture probe and a preamplifier (or amplifier or label probe) that hybridizes to it optionally comprise, at complementary positions, at least one pair of non-natural nucleotides that base pair with each other but that do not Watson-Crick base pair with the bases typical to biological DNA or RNA (i.e., A, C, G, T, or U). Examples of nonnatural nucleotides include, but are not limited to, Locked NucleicAcidTM nucleotides (available from Exiqon A/S, www (dot) exiqon (dot) com; see, e.g., SantaLucia Jr. (1998) Proc Natl Acad Sci 95:1460-1465) and isoG, isoC, and other nucleotides used in the AEGIS system (Artificially Expanded Genetic Information System, available from EraGen Biosciences, www (dot) eragen (dot) com; see, e.g., USPN 6,001,983, USPN 6,037,120, and USPN 6,140,496). Use of such non-natural base pairs (e.g., isoG-isoC base pairs) in the probes can, for example, reduce background and/or simplify probe design by decreasing cross hybridization, or it can permit use of shorter probes when the non-natural base pairs have higher binding affinities than do natural base pairs.

As noted, the methods are useful for multiplex detection of nucleic acids, including simultaneous detection of more than two nucleic acid targets. Thus, the cell optionally comprises or is suspected of comprising a third nucleic acid target, and the methods optionally include: providing a third label probe comprising a third label, wherein a third signal from the third label is distinguishable from the first and second signals, providing at least a third capture probe, hybridizing in the cell the third capture probe to the third nucleic acid target (when the third target is present in the cell), capturing the third label probe to the third capture probe, and detecting the third signal from the third label. Fourth, fifth, sixth, etc. nucleic acid targets are similarly simultaneously detected in the cell if desired.

A nucleic acid target can be essentially any nucleic acid that is desirably detected in the cell. For example, a nucleic acid target can be a DNA, a chromosomal DNA, an RNA (e.g., a cytoplasmic RNA), an mRNA, a microRNA, a ribosomal RNA, or the like. The nucleic acid target can be a nucleic acid endogenous to the cell. As another example, the target can be a nucleic acid introduced to or expressed in the cell by infection of the cell with a pathogen, for example, a viral or bacterial genomic RNA or DNA, a plasmid, a viral or bacterial mRNA, or the like.

The first and second (and/or optional third, fourth, etc.) nucleic acid targets can be part of a single nucleic acid molecule, or they can be separate molecules. Various advantages and applications of both approaches are discussed in greater detail below and in the section entitled "Implementation, applications, and advantages." In one class of embodiments, the first nucleic acid target is a first mRNA and the second nucleic acid target is a second mRNA. Alternatively, the first nucleic acid target comprises a first region of an mRNA and the second nucleic acid target comprises a second region of the same mRNA; this approach can increase specificity of detection of the mRNA. In another class of embodiments, the first nucleic acid target comprises a first chromosomal DNA polynucleotide sequence and the second nucleic acid target comprises a second chromosomal DNA polynucleotide sequence. The first and second chromosomal DNA polynucleotide sequences are optionally located on the same chromosome, e.g., within the same gene, or on different chromosomes.

The methods permit detection of even low or single copy number targets. Thus, in one class of embodiments, about 1000 copies or less of the first nucleic acid target and/or about 1000 copies or less of the second nucleic acid target are present in the cell (e.g., about 100 copies or less, about 50 copies or less, about 10 copies or less, about 5 copies or less, or even a single copy).

In one aspect, the signal(s) from nucleic acid target(s) are normalized. For example, the second nucleic acid target comprises a reference nucleic acid, and the method includes normalizing the first signal to the second signal. The reference nucleic acid is a nucleic acid selected as a standard of comparison. It will be evident that choice of the reference nucleic acid can depend on the desired application. For example, for gene expression analysis, where the first and optional third, fourth, etc. nucleic acid targets are mRNAs whose expression levels are to be determined, the reference nucleic acid can be an mRNA transcribed from a housekeeping gene. As another example, the first nucleic acid target can be an mRNA whose expression is altered in a pathological state, e.g., an mRNA expressed in a tumor cell and not a normal cell or expressed at a higher level in a tumor cell than in a normal cell, while the second nucleic acid target is an mRNA expressed from a housekeeping gene or similar gene whose expression is not altered in the pathological state. As yet another example, the first nucleic acid target can be a chromosomal DNA sequence that is amplified or deleted in a tumor cell, while the second nucleic acid target is another chromosomal DNA sequence that is maintained at its normal copy number in the tumor cell. Exemplary reference nucleic acids are described herein, and many more are well known in the art.

Optionally, results from the cell are compared with results from a reference cell. That is, the first and second targets are also detected in a reference cell, for example, a non-tumor, uninfected, or other healthy normal cell, chosen as a standard of comparison depending on the desired application. The signals can be normalized to a reference nucleic acid as noted above. As just one example, the first nucleic acid target can be the Her-2 gene, with the goal of measuring Her-2 gene amplification. Signal from Her-2 can be normalized to that from a reference gene, whose copy number is stably maintained in the genomic DNA. The normalized signal for the Her-2 gene from a target cell (e.g., a tumor cell or suspected tumor cell) can be compared to the normalized signal from a reference cell (e.g., a normal cell), to determine copy number in the cancer cell in comparison to normal cells.

The label (first, second, third, etc.) can be essentially any convenient label that directly or indirectly provides a detectable signal. In one aspect, the first label is a first fluorescent label and the second label is a second fluorescent label. Detecting the signal from the labels thus comprises detecting fluorescent signals from the labels. A variety of fluorescent labels whose signals can be distinguished from each other are known, including, e.g., fluorophores and quantum dots. As other examples, the label can be a luminescent label, a light-scattering label (e.g., colloidal gold particles), or an enzyme (e.g., alkaline phosphatase or horseradish peroxidase).

The methods can be used to detect the presence of the nucleic acid targets in cells from essentially any type of sample. For example, the sample can be derived from a bodily fluid, a bodily waste, blood, bone marrow, sputum, urine, lymph node, stool, vaginal secretions, cervical pap smear, oral swab or other swab or smear, spinal fluid, saliva, sputum, ejaculatory fluid, semen, lymph fluid, an intercellular fluid, a tissue (e.g., a tissue homogenate or tissue section), a biopsy, and/or a tumor. The sample and/or the cell can be derived from one or more of a human, an animal, a plant, and a cultured cell. Samples derived from even relatively large volumes of materials such as bodily fluid or bodily waste can be screened in the methods of the invention, and removal of such materials is relatively non-invasive. Samples are optionally taken from a patient, following standard laboratory methods after informed consent.

The methods for detecting nucleic acid targets in cells can be used to identify the cells. For example, a cell can be identified as being of a desired type based on which nucleic acids, and in what levels, it contains. Thus, in one class of embodiments, the methods include identifying the cell as a desired target cell based on detection of the first and second signals (and optional third, fourth, etc. signals) from within the cell. The cell can be identified on the basis of the presence of the nucleic acid targets. Similarly, the cell can be identified on the basis of the relative signal strength from or expression level of one or more of the nucleic acid targets. Signals are optionally normalized as noted above and/or compared to those from a reference cell.

The methods can be applied to detection and identification of even rare cell types. Thus, the sample including the cell can be a mixture of desired target cells and other, nontarget cells, which can be present in excess of the target cells. For example, the ratio of target cells to cells of all other type(s) in the sample is optionally less than 1:1x104, less than 1:1x105, less than 1:1x106, less than 1:1x107, less than 1:1x108, or even less than 1:1x109.

Essentially any type of cell that can be differentiated based on its nucleic acid content (presence, absence, expression level or copy number of one or more nucleic acids) can be detected and identified using the methods and a suitable choice of nucleic acid targets. As just a few examples, the cell can be a circulating tumor cell or other tumor cell, a virally infected cell, a fetal cell in maternal blood, a bacterial cell or other microorganism in a biological sample (e.g., blood or other body fluid), an endothelial cell, precursor endothelial cell, or myocardial cell in blood, a stem cell, or a T-cell. Rare cell types can be enriched prior to performing the methods, if necessary, by methods known in the art (e.g., lysis of red blood cells, isolation of peripheral blood mononuclear cells, further enrichment of rare target cells through magnetic-activated cell separation (MACS), etc.). The methods are optionally combined with other techniques, such as DAPI staining for nuclear DNA or analysis of cellular morphology. It will be evident that a variety of different types of nucleic acid markers are optionally detected simultaneously by the methods and used to identify the cell. For example, a cell can be identified based on the presence or relative expression level of one nucleic acid target in the cell and the absence of another nucleic acid target from the cell; e.g., a circulating tumor cell can be identified by the presence or level of one or more markers found in the tumor cell and not found (or found at different levels) in blood cells, and its identity can be confirmed by the absence of one or more markers present in blood cells and not circulating tumor cells. The principle may be extended to using any other type of markers such as protein based markers in single cells.

The cell is typically fixed and permeabilized before hybridization of the capture probes, to retain the nucleic acid targets in the cell and to permit the capture probes, label probes, etc. to enter the cell. The cell is optionally washed to remove materials not captured to one of the nucleic acid targets. The cell can be washed after any of various steps, for example, after hybridization of the capture probes to the nucleic acid targets to remove unbound capture probes, after hybridization of the preamplifiers, amplifiers, and/or label probes to the capture probes, and/or the like.

The various capture and hybridization steps can be performed simultaneously or sequentially, in essentially any convenient order. Preferably, a given hybridization step is accomplished for all of the nucleic acid targets at the same time. For example, all the capture probes (first, second, etc.) can be added to the cell at once and permitted to hybridize to their corresponding targets, the cell can be washed, amplifiers (first, second, etc.) can be hybridized to the corresponding capture probes, the cell can be washed, the label probes (first, second, etc.) can be hybridized to the corresponding amplifiers, and the cell can then be washed again prior to detection of the labels. As another example, the capture probes can be hybridized to the targets, the cell can be washed, amplifiers and label probes can be added together and hybridized, and the cell can then be washed prior to detection. It will be evident that double-stranded nucleic acid target(s) are preferably denatured, e.g., by heat, prior to hybridization of the corresponding capture probe(s) to the target(s).

In some embodiments, the cell is in suspension for all or most of the steps of the method, for ease of handling. However, the methods are also applicable to cells in solid tissue samples (e.g., tissue sections) and/or cells immobilized on a substrate (e.g., a slide or other surface). Thus, in one class of embodiments, the cell is in suspension in the sample comprising the cell, and/or the cell is in suspension during the hybridizing, capturing, and/or detecting steps. For example, the cell can be in suspension in the sample and during the hybridization, capture, optional washing, and detection steps. In other embodiments, the cell is in suspension in the sample comprising the cell, and the cell is fixed on a substrate during the hybridizing, capturing, and/or detecting steps. For example, the cell can be in suspension during the hybridization, capture, and optional washing steps and immobilized on a substrate during the detection step. In other embodiments, the sample comprises a tissue section.

Signals from the labels can be detected, and their intensities optionally measured, by any of a variety of techniques well known in the art. For example, in embodiments in which the cell is in suspension, the first and second (and optional third, etc.) signals can be conveniently detected by flow cytometry. In embodiments in which cells are immobilized on a substrate, the first and second (and optional third etc.) signals can be detected, for example, by laser scanner or microscope, e.g., a fluorescent or automated scanning microscope. As noted, detection is at the level of individual, single cells. Signals from the labels are typically detected in a single operation (e.g., a single flow cytometry run or a single microscopy or scanning session), rather than sequentially in separate operations for each label. Such a single detection operation can, for example, involve changing optical filters between detection of the different labels, but it does not involve detection of the first label followed by capture of the second label and then detection of the second label. In some embodiments, the first and second (and optional third etc.) labels are captured to their respective targets simultaneously but are detected in separate detection steps or operations.

Additional features described herein, e.g., in the section below entitled "Implementation, applications, and advantages," can be applied to the methods, as relevant. For example, as described in greater detail below, a label probe can include more than one label, identical or distinct. Signal strength is optionally adjusted between targets depending on their expected copy numbers, if desired; for example, the signal for an mRNA expressed at low levels can be amplified to a greater degree (e.g., by use of more labels per label probe and/or use of preamplifiers and amplifiers to capture more label probes per copy of the target) than the signal for a highly expressed mRNA.

In another aspect of the disclosure, two or more nucleic acids are detected by PCR amplification of the nucleic acids in situ in individual cells. To prevent leakage of the resulting amplicons out of the cells, a water-oil emulsion can be made as mentioned in Li et al. (2006) "BEAMing up for detection and quantification of rare sequence variants" Nature Methods 3(2):95-7 that separates single cells into different compartments.

### Detection of nucleic acid targets using distinguishable labels or indistinguishable labels

As noted, the methods of the invention employ multiplex nucleic acid assays in single cells as defined in the claims. One general class of embodiments includes methods of detecting two or more nucleic acid targets in an individual cell. In the methods, a sample comprising the cell is provided. The cell comprises, or is suspected of comprising, a first nucleic acid target and a second nucleic acid target. A first label probe comprising a first label and a second label probe comprising a second label are provided. At least a first capture probe and at least a second capture probe are also provided.

For example, the first signal from the first label is distinguishable from a second signal from the second label, so that the first nucleic acid target and the second nucleic acid target can be detected independently. In one embodiment, the first signal from the first label is indistinguishable from a second signal from the second label. This embodiment is beneficial in many different applications. In one example application, the first and second nucleic acid target always co-exist in certain cells and the signal produced by the two targets in combination is much stronger than that by one of the targets. In another example application, it is desirable to detect the presence of the first and the second nucleic acid target in cell. Using the same or indistinguishable label enables such detection without increasing the complexity of the readout instrument. In yet another example application, it is desirable to detect or identify a specific type of cell among a mixed cell population based on the presence or quantity of some unique nucleic acid targets in the cell. Since the quantity or even presence of a single nucleic acid target may not be stable within the target cell, using a single nucleic acid target as the marker will affect the reliability of the cell identification. Two or more targets with indistinguishable label can produce much more stable signal, leading to more reliable identification. In a more specific example, circulating tumor cells (CTC) are to be detected from a blood sample.

Cytokeratins 19 (CK19) mRNA is a commonly used as a marker to distinguish CTC from other normal blood cells. However, we have discovered that the expression level of CK19 is not stable in all CTCs. Since CTC are so rare, this fluctuation will affect the reliability of CTC detection. By adopting multiple RNA targets in CK family as the CTC marker and label them with the same label, the marker signal can be much more stable and reliability of CTC detection greatly improved. In a specific embodiment of this particular application, CK8, 18 and 19 RNA are used as a "pan-CK" target group and labeled with the same label for CTC detection in body fluids.

This use of labels producing indistinguishable signals described above has many more specific applications. Two exemplary embodiments are described below:

### Detecting a desired group of human papillomavirus (HPV) sub-types

HPV is a family of more than 100 virus sub-types. One group of these sub-types is regarded as "high risk" in causing cervical cancer, which includes PV-16, 18, 26, 31, 33, 35, 39, 45, 51, 52, 53, 56, 58, 59, 66, 68, 73, and 82. It has been recently discovered that another group is high risk in causing head and neck cancer, which includes HPV-16, 18, 31, 33, 35, 52, and 58. It is clinically valuable for a diagnostic test to detect the presence of these high risk (HR) groups of HPV in clinical sample in order to access the risk of progressing towards cancer. One approach to develop such a diagnostic test is to detect each specific sub-type individually, which will be very costly and requires a large amount of specimens. A second approach would be to find a common sequence among members of the group and design a probe set targeting this common sequence. The problem with the second approach is such common sequence may be too short. In addition, there are many situations where members of this high risk group may change. For example, a new HR sub-type may emerge. In addition, the HR group for different cancers, i.e. cervical cancer and head & neck cancer, has different members. Often it is clinically desirable to further split the HR group into highest risk group and the rest. The way to split the HR group may be different depending on the regions in different parts of the world. For example, in US, HPV-16 is definitely the highest risk member of the HR group for cervical cancer. But in Asia, HPV-16 and 45 are both very prominent. Using the second approach, the probe set and possibly the assay conditions have to be re-design and re-optimized to accommodate any of the above described changes. This application uses a new and different approach: probe sets are designed to specifically detect each individual high-risk sub-types. Labels in each probe set generate the same, indistinguishable signal. Depending on the membership of the group to be detected, their corresponding probe sets are pooled together to become the probe set for the group. If any member of the group is present in the specimen, the presence of the group will be detected. One of the advantages of this design is that the composition of the members of the group can be changed without redesigning the probe set or the assay parameters.

### Providing a more stable positive control

Most diagnostic tests require to perform a parallel positive control test, which provides an indication of assay success. For in situ RNA detection, in particular, there is always the concern of RNA degradation in the specimen. A well designed positive control test can also provide a measure of RNA quality in the specimen. The target of a positive control test is normally a house keeping gene, such as Ubc. An ideal housekeeping gene would be a stable median expressor in all types of organs in a particular species such as human. In reality, however, the level of expression of any one house keeping gene changes from organ to organ. In some organs, the expression can be too low to be useful. This invention solves this problem by using a pool of multiple housekeeping genes labels with indistinguishable signals. A more stable positive control can be achieved across a wider range of organs.

### Detection of relative levels by normalization to reference nucleic acids

As discussed briefly above, the signal detected for a nucleic acid of interest can be normalized to that of a reference nucleic acid. Disclosed herein are methods of assaying a relative level of one or more target nucleic acids in an individual cell. In the methods, a sample comprising the cell is provided. The cell comprises or is suspected of comprising a first, target nucleic acid, and it comprises a second, reference nucleic acid. A first label probe comprising a first label and a second label probe comprising a second label, wherein a first signal from the first label is distinguishable from a second signal from the second label, are also provided. In the cell, the first label probe is captured to the first, target nucleic acid (when the first, target nucleic acid is present in the cell) and the second label probe is captured to the second, reference nucleic acid. The first signal from the first label and the second signal from the second label are then detected in the individual cell, and the intensity of each signal is measured. The intensity of the first signal is normalized to the intensity of the second (reference) signal. The level of the first, target nucleic acid relative to the level of the second, reference nucleic acid in the cell is thereby assayed, since the first and second labels are associated with their respective nucleic acids. The methods are optionally quantitative, permitting measurement of the amount of the first, target nucleic acid relative to the amount of the second, reference nucleic acid in the cell. Thus, the intensity of the first signal normalized to that of the second signal can be correlated with a quantity of the first, target nucleic acid present in the cell. It is highly desirable for the reference nucleic acid to be stable across different cells and/or different types of cells. However, it is difficult to find such a single nucleic acid. As we have discussed before, the second nucleic acid can be a group of many nucleic acids and the probe sets for each member of the group carry indistinguishable signal. The signal of the group can then be more stable across different cells and/or cell types.

The label probes can bind directly to the nucleic acids. For example, the first label probe can hybridize to the first, target nucleic acid and/or the second label probe can hybridize to the second, reference nucleic acid. Alternatively, some or all of the label probes can be indirectly bound to their corresponding nucleic acids, e.g., through capture probes. For example, the first and second label probes can bind directly to the nucleic acids, or one can bind directly while the other binds indirectly, or both can bind indirectly.

The label probes are optionally captured to the nucleic acids via capture probes. In one class of embodiments, at least a first capture probe and at least a second capture probe are provided. In the cell, the first capture probe is hybridized to the first, target nucleic acid and the second capture probe is hybridized to the second, reference nucleic acid. The first label probe is captured to the first capture probe and the second label probe is captured to the second capture probe, thereby capturing the first label probe to the first, target nucleic acid and the second label probe to the second, reference nucleic acid. The features described for the methods above apply to these embodiments as well, with respect to configuration and number of the label and capture probes, optional use of preamplifiers and/or amplifiers, rolling circle amplification of circular polynucleotides, and the like.

The methods can be used for multiplex detection of nucleic acids, including simultaneous detection of two or more target nucleic acids. Thus, the cell optionally comprises or is suspected of comprising a third, target nucleic acid, and the methods optionally include: providing a third label probe comprising a third label, wherein a third signal from the third label is distinguishable from the first and second signals; capturing, in the cell, the third label probe to the third, target nucleic acid (when present in the cell); detecting the third signal from the third label, which detecting comprises measuring an intensity of the third signal; and normalizing the intensity of the third signal to the intensity of the second signal. Alternatively, the third signal can be normalized to that from a different reference nucleic acid. Fourth, fifth, sixth, etc. nucleic acids are similarly simultaneously detected in the cell if desired. The third, fourth, fifth, etc. label probes are optionally hybridized directly to their corresponding nucleic acid, or they can be captured indirectly via capture probes as described for the first and second label probes.

The methods can be used for gene expression analysis, detection of gene amplification or deletion, or detection or diagnosis of disease, as just a few examples. A target nucleic acid can be essentially any nucleic acid that is desirably detected in the cell. For example, a target nucleic acid can be a DNA, a chromosomal DNA, an RNA, an mRNA, a microRNA, a ribosomal RNA, or the like. The target nucleic acid can be a nucleic acid endogenous to the cell, or as another example, the target can be a nucleic acid introduced to or expressed in the cell by infection of the cell with a pathogen, for example, a viral or bacterial genomic RNA or DNA, a plasmid, a viral or bacterial mRNA, or the like. The reference nucleic acid can similarly be a DNA, an mRNA, a chromosomal DNA, an mRNA, an RNA endogenous to the cell, or the like.

As described above, choice of the reference nucleic acid can depend on the desired application. For example, for gene expression analysis, where the first and optional third, fourth, etc. target nucleic acids are mRNAs whose expression levels are to be determined, the reference nucleic acid can be an mRNA transcribed from a housekeeping gene. As another example, the first, target nucleic acid can be an mRNA whose expression is altered in a pathological state, e.g., an mRNA expressed in a tumor cell and not a normal cell or expressed at a higher level in a tumor cell than in a normal cell, while the reference nucleic acid is an mRNA expressed from a housekeeping gene or similar gene whose expression is not altered in the pathological state. In a similar example, the target nucleic acid can be a viral or bacterial nucleic acid while the reference nucleic acid is endogenous to the cell. As yet another example, the first, target nucleic acid can be a chromosomal DNA sequence that is amplified or deleted in a tumor cell, while the reference nucleic acid is another chromosomal DNA sequence that is maintained at its normal copy number in the tumor cell. Exemplary reference nucleic acids are described herein, and many more are well known in the art.

In one class of embodiments, the first, target nucleic acid is a first mRNA and the second, reference nucleic acid is a second mRNA. In another class of embodiments, the first, target nucleic acid comprises a first chromosomal DNA polynucleotide sequence and the second, reference nucleic acid comprises a second chromosomal DNA polynucleotide sequence. The first and second chromosomal DNA polynucleotide sequences are optionally located on the same chromosome or on different chromosomes.

Optionally, normalized results from the cell are compared with normalized results from a reference cell. That is, the target and reference nucleic acids are also detected in a reference cell, for example, a non-tumor, uninfected, or other healthy normal cell, chosen as a standard of comparison depending on the desired application. As just one example, the first, target nucleic acid can be the Her-2 gene, with the goal of measuring Her-2 gene amplification. Signal from Her-2 can be normalized to that from a reference gene whose copy number is stably maintained in the genomic DNA. The normalized signal for the Her-2 gene from a target cell (e.g., a tumor cell or suspected tumor cell) can be compared to the normalized signal from a reference cell (e.g., a normal cell), to determine copy number in the cancer cell in comparison to normal cells.

Signal strength is optionally adjusted between the target and reference nucleic acids depending on their expected copy numbers, if desired. For example, the signal for a target mRNA expressed at low levels can be amplified to a greater degree (e.g., by use of more labels per label probe and/or use of capture probes, preamplifiers and amplifiers to capture more label probes per copy of the target) than the signal for a highly expressed mRNA (which can, e.g., be detected by direct binding of the label probe to the reference nucleic acid, by use of capture probes and amplifier without a preamplifier, or the like).

The methods for assaying relative levels of target nucleic acids in cells can be used to identify the cells. For example, a cell can be identified as being of a desired type based on which nucleic acids, and in what levels, it contains. Thus, in one class of embodiments, the methods include identifying the cell as a desired target cell based on the normalized first signal (and optional normalized third, fourth, etc. signals). As described herein, the cell can be identified on the basis of the presence or absence of one or more of the target nucleic acids. Similarly, the cell can be identified on the basis of the relative signal strength from or expression level of one or more target nucleic acids. Signals are optionally compared to those from a reference cell.

The methods can be applied to detection and identification of even rare cell types. Thus, the sample including the cell can be a mixture of desired target cells and other, nontarget cells, which can be present in excess of the target cells. For example, the ratio of target cells to cells of all other type(s) in the sample is optionally less than 1:1x104, less than 1:1x105, less than 1:1x106, less than 1:1x107, less than 1:1x108, or even less than 1:1x109.

Essentially any type of cell that can be differentiated based on its nucleic acid content (presence, absence, or copy number of one or more nucleic acids) can be detected and identified using the methods and a suitable choice of target and reference nucleic acids. As just a few examples, the cell can be a circulating tumor cell or other tumor cell, a virally infected cell, a fetal cell in maternal blood, a bacterial cell or other microorganism in a biological sample (e.g., blood or other body fluid), or an endothelial cell, precursor endothelial cell, or myocardial cell in blood. Rare cell types can be enriched prior to performing the methods, if necessary, by methods known in the art (e.g., lysis of red blood cells, isolation of peripheral blood mononuclear cells, etc.). The methods are optionally combined with other techniques, such as DAPI staining for nuclear DNA. It will be evident that a variety of different types of nucleic acid markers are optionally detected simultaneously by the methods and used to identify the cell. For example, a cell can be identified based on the presence or relative expression level of one target nucleic acid in the cell and the absence of another target nucleic acid from the cell; e.g., a circulating tumor cell can be identified by the presence or level of one or more markers found in the tumor cell and not found (or found at different levels) in blood cells, and by the absence of one or more markers present in blood cells and not circulating tumor cells. The principle may be extended to using any other type of markers such as protein based markers in single cells.

Essentially all of the features noted for the methods above apply to these embodiments as well, as relevant; for example, with respect to source of sample, fixation and permeabilization of the cell, washing the cell, denaturation of double-stranded target and reference nucleic acids, type of labels, use of optional blocking probes, detection of signals, detection (and intensity measurement or spot counting) by flow cytometry or microscopy, presence of the cell in suspension, immobilized on a substrate, or in a tissue, and/or the like. Also, additional features described herein, e.g., in the section entitled "Implementation, applications, and advantages," can be applied to the methods, as relevant.

The methods of the invention can be used for gene expression analysis in single cells. Currently, gene expression analysis deals with heterogeneous cell populations such as blood or tumor specimens. Blood contains various subtypes of leukocytes, and when changes in gene expression of whole blood or RNA isolated from blood are measured, it is not known what subtype of blood cells actually changed their gene expression. It is possible that gene expression of only a certain subtype of blood cells is affected in a disease state or by drug treatment, for example. Technology that can measure gene expression in single cells, so changes of gene expression in single cells can be examined, is thus desirable. Similarly, a tumor specimen contains a heterogeneous cell population including tumor cells, normal cells, stromal cells, immune cells, etc. Current technology looks at the sum of the expression of all those cells through total RNA or cell lysate. However, the overall expression change may not be representative of that in target tumor cells. So again, it would be useful to look at the expression changes in single cells so that the target tumor cells can be examined specifically, to see how the target cells change in gene expression and how they respond to drug treatment, for example.

In one aspect, the present invention can be used for gene expression analysis in single cells. Single cell gene expression analysis can be accomplished by measuring expression of a target gene and normalizing against the expression of a housekeeping gene, as described above. As just a couple of examples, the normalized expression in a disease state can be compared to that in the normal state, or the expression in a drug treated state can be compared to that in the normal state. The change of expression level in single cells may have biological significance indicating disease progression, drug therapeutic efficacy and/or toxicity, tumor staging and classification, etc.

Accordingly, also disclosed herein are methods of performing comparative gene expression analysis in single cells. In the methods, a first mixed cell population comprising one or more cells of a specified type is provided. A second mixed cell population comprising one or more cells of the specified type is also provided. An expression level of one or more target nucleic acids relative to a reference nucleic acid is measured in the cells of the specified type of the first population, to provide a first expression profile. An expression level of the one or more target nucleic acids relative to the reference nucleic acid is measured in the cells of the specified type of the second population, to provide a second expression profile. The first and second expression profiles are compared.

For example, the one or more target nucleic acids are one or more mRNAs, e.g., two or more, three or more, four or more, etc. mRNAs. The expression level of each mRNA can be determined relative to that of a housekeeping gene whose mRNA serves as the reference nucleic acid.

The first and/or second mixed cell population contains at least one other type of cell in addition to the specified type, more typically at least two or more other types of cells, and optionally several to many other types of cells (e.g., as is found in whole blood, a tumor, or other complex biological sample). The ratio of cells of the specified type to cells of all other type(s) in the first or second mixed cell population is optionally less than 1:1x104, less than 1:1x105, less than 1:1x106, less than 1:1x107, less than 1:1x108, or even less than 1:1x109.

As will be evident, a change in gene expression profile between the two populations may indicate a disease state or progression, a drug response, a therapeutic efficacy, etc. Thus, for example, the first mixed cell population can be from a patient who has been diagnosed or who is to be diagnosed with a particular disease or disorder, while the second mixed population is from a healthy individual. Similarly, the first and second mixed populations can be from a single individual but taken at different time points, for example, to follow disease progression or to assess response to drug treatment. Accordingly, the first mixed cell population can be taken from an individual (e.g., a human) before treatment is initiated with a drug or other compound, while the second population is taken at a specified time after treatment is initiated. As another example, the first mixed population can be from a treated individual while the second mixed population is from an untreated individual.

Essentially all of the features noted for the methods above apply to these embodiments as well, as relevant; for example, with respect to type of target and reference nucleic acids, cell type, source of sample, fixation and permeabilization of the cell, washing the cell, denaturation of double-stranded target and reference nucleic acids, type of labels, use and configuration of label probes, capture probes, preamplifiers and/or amplifiers, use of optional blocking probes, detection of signals, detection (and intensity measurement or spot counting) by flow cytometry or microscopy, presence of the cell in suspension, immobilized on a substrate, or in a tissue, and/or the like. Exemplary target and reference nucleic acids are described herein.

In another aspect, the methods can be used to compare copy number in single cells from a first population (e.g., tumor cells) with copy number in single cells from a second population (e.g., normal cells used as a reference). The nucleic acid target(s) can be transcripts or genomic DNA, where, for example, the degree of amplification or deletion of genes such as her-2 can correlate with tumor progression. In another aspect, the methods can be applied to gene expression analysis in single cells in even a single population, including, for example, cells of the same type but at different stages of the cell cycle.

### Label density

The methods of the invention permit far more labels to be captured to small regions of target nucleic acids than do currently existing techniques. For example, standard FISH techniques typically use probes that cover 20 kb or more, and a probe typically has fluorophores chemically conjugated at a density of approximately one fluorescent molecule per seven nucleotides of the probe. When molecular beacon target detection is employed, one label pair is captured to the target in the region covered by the beacon, typically about 40 nucleotides. For additional discussion of exemplary current techniques, see, e.g., U.S. patent application publications 2004/0091880 and 2005/0181463, USPN 6,645,731, and international patent application publications WO 95/09245 and 03/019141.

Methods described herein, in comparison, readily permit capture of hundreds of labels (e.g., 400 or more) to the region of the target covered by a single capture probe, e.g., 20-25 nucleotides or more. The theoretical degree of amplification achieved from a single capture probe is readily calculated for any given configuration of capture probes, amplifiers, etc; for example, the theoretical degree of amplification achieved from a single capture probe, and thus the number of labels per length in nucleotides of the capture probe, can be equal to the number of preamplifiers bound to the capture probe times the number of amplifiers that bind each preamplifier times the number of label probes that bind each preamplifier times the number of labels per label probe.

Thus, in one aspect, the disclosure provides methods that facilitate association of a high density of labels to target nucleic acids in cells. One general class of embodiments provides methods of detecting two or more nucleic acid targets in an individual cell. In the methods, a sample comprising the cell is provided. The cell comprises or is suspected of comprising a first nucleic acid target and a second nucleic acid target. In the cell, a first label is captured to the first nucleic acid target (when present in the cell) and a second label is captured to the second nucleic acid target (when present in the cell). A first signal from the first label is distinguishable from a second signal from the second label. As noted, the labels are captured at high density. Thus, an average of at least one copy of the first label per nucleotide of the first nucleic acid target is captured to the first nucleic acid target over a region that spans at least 20 contiguous nucleotides of the first nucleic acid target, and an average of at least one copy of the second label per nucleotide of the second nucleic acid target is captured to the second nucleic acid target over a region that spans at least 20 contiguous nucleotides of the second nucleic acid target. The first signal from the first label and the second signal from the second label are detected.

In one class of embodiments, an average of at least four, eight, or twelve copies of the first label per nucleotide of the first nucleic acid target are captured to the first nucleic acid target over a region that spans at least 20 contiguous nucleotides of the first nucleic acid target, and an average of at least four, eight, or twelve copies of the second label per nucleotide of the second nucleic acid target are captured to the second nucleic acid target over a region that spans at least 20 contiguous nucleotides of the second nucleic acid target. In one embodiment, an average of at least sixteen copies of the first label per nucleotide of the first nucleic acid target are captured to the first nucleic acid target over a region that spans at least 20 contiguous nucleotides of the first nucleic acid target, and an average of at least sixteen copies of the second label per nucleotide of the second nucleic acid target are captured to the second nucleic acid target over a region that spans at least 20 contiguous nucleotides of the second nucleic acid target.

Essentially all of the features noted for the methods above apply to these embodiments as well, as relevant, for example, with respect to type of labels, detection of signals, type, treatment, and suspension of the cell, and/or the like. The regions of the first and second nucleic acid targets optionally span at least 25, 50, 100, 200, or more contiguous nucleotides and/or at most 2000, 1000, 500, 200, 100, 50, or fewer nucleotides. A like density of third, fourth, fifth, sixth, etc. labels is optionally present for (e.g., captured to) third, fourth, fifth, sixth, etc. nucleic acid targets.

If the target is short, conventional FISH (or other direct label in situ methods) cannot attain sufficient signal to achieve detection of the target. The methods described herein, however, enable in situ, high sensitivity detection of even short targets (e.g., a short nucleic acid molecule or a short region of polynucleotide sequence within a longer nucleic acid molecule), including, e.g., target sections of longer sequences and target molecules less than 1 kb. Accordingly, one general class of embodiments provides methods of detecting one or more nucleic acid targets in an individual cell that include: providing a sample comprising the cell, which cell comprises or is suspected of comprising a first nucleic acid target; providing a first label probe comprising a first label; providing a set of one or more first capture probes; hybridizing, in the cell, the first capture probes to the first nucleic acid target, when present in the cell, wherein the set of first capture probes hybridizes to a region of the first nucleic acid target (including, e.g., the entire target molecule or a portion thereof) that is 1000 nucleotides or less in length (e.g., 500 nucleotides or less in length); capturing the first label probe to the first capture probes, thereby capturing the first label probe to the first nucleic acid target; and detecting a first signal from the first label. For example, the set of first capture probes can hybridize to a region of the first nucleic acid target that is 200 nucleotides or less in length, 100 nucleotides or less in length, 50 nucleotides or less in length, or even 25 nucleotides or less in length, thus permitting detection of target nucleic acids as small as microRNAs, for example. Other exemplary targets include, but are not limited to, short or short regions of DNAs, chromosomal DNAs, RNAs, mRNAs, and ribosomal RNAs.

As for the embodiments above, the methods are useful for multiplex detection of nucleic acids, including simultaneous detection of two or more nucleic acid targets (e.g., short targets, or a combination of short and longer targets). Thus, the cell optionally comprises or is suspected of comprising a second nucleic acid target, and the methods optionally include: providing a second label probe comprising a second label, wherein a second signal from the second label is distinguishable from the first signal, providing a set of one or more second capture probes, hybridizing in the cell the second capture probes to the second nucleic acid target, when present in the cell, capturing the second label probe to the second capture probes, and detecting the second signal from the second label. Third, fourth, fifth, sixth, etc. nucleic acid targets are similarly simultaneously detected in the cell if desired. Each hybridization or capture step is preferably accomplished for all of the nucleic acid targets at the same time.

Essentially all of the features noted for the methods above apply to these embodiments as well, as relevant; for example, with respect to type of nucleic acid targets, copy number, cell type, source of sample, fixation and permeabilization of the cell, washing the cell, denaturation of double-stranded nucleic acids, type of labels, use and configuration of label probes, capture probes, preamplifiers and/or amplifiers (including, e.g., hybridization of two capture probes to a single label probe, preamplifier, or amplifier molecule), use of optional blocking probes, detection of signals, detection (and intensity measurement) of signals from the individual cell by flow cytometry or microscopy, presence of the cell in suspension, immobilized on a substrate, or in a tissue, and/or the like.

### Detection of target cells

As described above, cells can be detected and identified by detecting their constituent nucleic acids. In a general class of embodiments, multiple targets with indistinguishable labels are used for the identification of a specific cell type. The unique probe set configuration described above in this invention significantly improves the sensitivity and specificity of the nucleic acid target detection, which, in turn, would enhanced the sensitivity and specificity in the identification of specific cells from a mixed cell population.

One particular application of indistinguishable labels for detection and identification of a specific target cells in a mixed cell population is for the detection, identification and enumeration of CTCs. Research shows that there are different types of CTCs, some of them epithelial-like, some have gone through epithelial to mesenchymal transition (EMT). We have discussed in the above the approach for using a "pan-CK" marker comprising CK8, CK18, CK19 and other CK mRNAs to detect epithelial-like CTCs. The probe set for the pan-CK marker is a pool of probe sets with indistinguishable labels, each specifically targeting one of the CKs in the group. However, other types of CTCs, such as EMT CTCs which have different molecular phenotype, may not express these markers. Because CTCs are so rare, it is important to detect and enumerate all CTCs in the specimen. We have therefore expanded the "pan-CK" marker into a "pan-CTC" marker, which comprises CK8, CK14, CK17, CK18, CK19, CK20, EpCAM, Muc1, EGFR, Twist, N-Cadherin and Fibronectin. We have conducted experiments to demonstrate that each member in the group is specifically expressed in certain type of CTCs, but not in blood cells. Again, the probe set of this pan-CTC marker group is a pool of probes with indistinguishable labels, with each probe specifically targeting one member of the group. Thus, both epithelial-like and EMT CTCs can be detected and accounted for in a sample, if present.

As disclosed herein, two or more nucleic acid targets are used for the identification of a specific cell type; each target is an independent marker producing distinguishable signals. In yet another general class of embodiments, two or more groups of targets are used for the identification of a specific cell type; each group is an independent marker so that each target member of the same group is labeled to generate indistinguishable signals and different group are labeled to produce different, distinguishable signals. For certain applications, for example, detection of rare cells from large heterogeneous mixtures of cells, such a scheme to detect multiple, redundant markers is advantageous. The following hypothetical example illustrates one advantage of detecting redundant markers.

Say that circulating tumor cells (CTC) are to be detected from a blood sample in which the CTC concentration is one in 106 normal white blood cells. If a single marker for the CTC (e.g., a nucleic acid whose presence or copy number can uniquely and sufficiently distinguish the cell from the rest of the cell population) has a detection specificity of 1 in 103, 1000 cells will be mistakenly identified as "CTC" when 106 cells are counted. (Such false positives can result from random background signal generated by nonspecific binding of the relevant probe(s) or from similar factors.) If an additional independent marker is included which, on its own, also has a detection specificity of 1 in 103, and if a cell is identified as a CTC only if both markers are positive, the combined detection specificity is now theoretically dramatically increased, to 1 in 103x103=106. This specificity is sufficient for direct CTC detection in normal white blood cells under these assumptions. Similarly, if three independent redundant markers are used for identification of CTC, the detection specificity can be boosted to 1 in 109. Use of two or more redundant markers thus reduces the number of false positives and facilitates detection of even rare cells from complex samples.

Accordingly, one general class of aspects discloses methods of detecting an individual cell of a specified type. In the methods, a sample comprising a mixture of cell types including at least one cell of the specified type is provided. A first label probe comprising a first label and a second label probe comprising a second label, wherein a first signal from the first label is distinguishable from a second signal from the second label, are provided. In the cell, the first label probe is captured to a first nucleic acid target (when the first nucleic acid target is present in the cell) and the second label probe is captured to a second nucleic acid target (when the second nucleic acid target is present in the cell). The first signal from the first label and the second signal from the second label are detected and correlated with the presence, absence, or amount of the corresponding, first and second nucleic acid targets in the cell. The cell is identified as being of the specified type based on detection of the presence, absence, or amount (e.g., a non-zero amount) of both the first and second nucleic acid targets within the cell, where the specified type of cell is distinguishable from the other cell type(s) in the mixture on the basis of either the presence, absence, or amount of the first nucleic acid target or the presence, absence, or amount of the second nucleic acid target in the cell (that is, the nucleic acid targets are redundant markers for the specified cell type). An intensity of the first signal and an intensity of the second signal are optionally measured and correlated with a quantity of the corresponding nucleic acid present in the cell. As another example, a signal spot can be counted for each copy of the first and second nucleic acid targets to quantitate them, as described in greater detail below.

Each nucleic acid target that serves as a marker for the specified cell type can distinguish the cell type by its presence in the cell, by its amount (copy number, e.g., its genomic copy number or its transcript expression level), or by its absence from the cell (a negative marker). A set of nucleic acid targets can include different types of such markers; that is, one nucleic acid target can serve as a positive marker, distinguishing the cell by its presence or non-zero amount in the cell, while another serves as a negative marker, distinguishing the cell by its absence from the cell. For example, the cell comprises a first nucleic acid target and a second nucleic acid target, and the cell is identified as being of the specified type based on detection of the presence or amount of both the first and second nucleic acid targets within the cell, where the specified type of cell is distinguishable from the other cell type(s) in the mixture on the basis of either the presence or amount of the first nucleic acid target or the presence or amount of the second nucleic acid target in the cell.

The label probes can bind directly to the nucleic acid targets. For example, the first label probe can hybridize to the first nucleic acid target and/or the second label probe can hybridize to the second nucleic acid target. Alternatively, some or all of the label probes can be indirectly bound to their corresponding nucleic acid targets, e.g., through capture probes. For example, the first and second label probes can bind directly to the nucleic acid targets, or one can bind directly while the other binds indirectly, or both can bind indirectly.

The label probes are optionally captured to the nucleic acid targets via capture probes. In one class of embodiments, at least a first capture probe and at least a second capture probe are provided. In the cell, the first capture probe is hybridized to the first nucleic acid target and the second capture probe is hybridized to the second nucleic acid target. The first label probe is captured to the first capture probe and the second label probe is captured to the second capture probe, thereby capturing the first label probe to the first nucleic acid target and the second label probe to the second nucleic acid target. The features described for the methods above apply to these embodiments as well, with respect to configuration and number of the label and capture probes, optional use of preamplifiers and/or amplifiers, rolling circle amplification of circular polynucleotides, and the like.

Third, fourth, fifth, etc. nucleic acid targets are optionally detected in the cell. For example, the method optionally includes: providing a third label probe comprising a third label, wherein a third signal from the third label is distinguishable from the first and second signals, capturing in the cell the third label probe to a third nucleic acid target (when present in the cell), and detecting the third signal from the third label. The third, fourth, fifth, etc. label probes are optionally hybridized directly to their corresponding nucleic acid, or they can be captured indirectly via capture probes as described for the first and second label probes.

The additional markers can be used in any of a variety of ways. For example, the cell can comprise the third nucleic acid target, and the first and/or second signal can be normalized to the third signal. The methods can include identifying the cell as being of the specified type based on the normalized first and/or second signal, e.g., in embodiments in which the target cell type is distinguishable from the other cell type(s) in the mixture based on the copy number of the first and/or second nucleic acid targets, rather than purely on their presence in the target cell type and not in the other cell type(s). Examples include cells detectable based on a pattern of differential gene expression, CTC or other tumor cells detectable by overexpression of one or more specific mRNAs, and CTC or other tumor cells detectable by amplification or deletion of one or more specific chromosomal regions.

As another example, the third nucleic acid target can serve as a third redundant marker for the target cell type, e.g., to improve specificity of the assay for the desired cell type. Thus, the methods may include correlating the third signal detected from the cell with the presence, absence, or amount of the third nucleic acid target in the cell, and identifying the cell as being of the specified type based on detection of the presence, absence, or amount of the first, second, and third nucleic acid targets within the cell, wherein the specified type of cell is distinguishable from the other cell type(s) in the mixture on the basis of either presence, absence, or amount of the first nucleic acid target, presence, absence, or amount of the second nucleic acid target, or presence, absence, or amount of the third nucleic acid target in the cell.

As yet another example, the additional markers can assist in identifying the cell type. For example, the presence, absence, or amount of the first and third markers may suffice to identify the cell type, as could the presence, absence, or amount of the second and fourth markers; all four markers could be detected to provide two redundant sets of markers and therefore increased specificity of detection. As another example, one or more additional markers can be used in negative selection against undesired cell types; for example, identity of a cell as a CTC can be further verified by the absence from the cell of one or more markers present in blood cells and not circulating tumor cells.

Detection of additional nucleic acid targets can also provide further information useful in diagnosis, outcome prediction or the like, regardless of whether the targets serve as markers for the particular cell type. For example, additional nucleic acid targets can include markers for proliferating potential, apoptosis, or other metastatic, genetic, or epigenetic changes.

Signals from the additional targets are optionally normalized to a reference nucleic acid as described above. Signal strength is optionally adjusted between targets depending on their expected copy numbers, if desired. Signals from the target nucleic acids in the cell are optionally compared to those from a reference cell, as noted above.

A nucleic acid target can be essentially any nucleic acid that is desirably detected in the cell. For example, a nucleic acid target can be a DNA, a chromosomal DNA, an RNA, an mRNA, a microRNA, a ribosomal RNA, or the like. The nucleic acid target can be a nucleic acid endogenous to the cell. As another example, the target can be a nucleic acid introduced to or expressed in the cell by infection of the cell with a pathogen, for example, a viral or bacterial genomic RNA or DNA, a plasmid, a viral or bacterial mRNA, or the like.

The first and second (and/or optional third, fourth, etc.) nucleic acid targets can be part of a single nucleic acid molecule, or they can be separate molecules. Various advantages and applications of both approaches are discussed in greater detail below, e.g., in the section entitled "Implementation, applications, and advantages." In one class of embodiments, the first nucleic acid target is a first mRNA and the second nucleic acid target is a second mRNA. Alternatively, the first nucleic acid target comprises a first region of an mRNA and the second nucleic acid target comprises a second region of the same mRNA. In another class of embodiments, the first nucleic acid target comprises a first chromosomal DNA polynucleotide sequence and the second nucleic acid target comprises a second chromosomal DNA polynucleotide sequence. The first and second chromosomal DNA polynucleotide sequences are optionally located on the same chromosome, e.g., within the same gene, or on different chromosomes.

The methods can be applied to detection and identification of even rare cell types. For example, the ratio of cells of the specified type to cells of all other type(s) in the mixture is optionally less than 1:1x104, less than 1:1x105, less than 1:1x106, less than 1:1x107, less than 1:1x108, or even less than 1:1x109.

Essentially any type of cell that can be differentiated based on suitable markers (or redundant regions of a single marker, e.g., a single mRNA or amplified/deleted chromosomal region) can be detected and identified using the methods. As just a few examples, the cell can be a circulating tumor cell or other tumor cell, a virally infected cell, a fetal cell in maternal blood, a bacterial cell or other microorganism in a biological sample (e.g., blood or other body fluid), an endothelial cell, precursor endothelial cell, or myocardial cell in blood, stem cell, or T-cell. Rare cell types can be enriched prior to performing the methods, if necessary, by methods known in the art (e.g., lysis of red blood cells, isolation of peripheral blood mononuclear cells, etc.).

Essentially all of the features noted for the methods above apply to these embodiments as well, as relevant; for example, with respect to source of sample, fixation and permeabilization of the cell, washing the cell, denaturation of double-stranded nucleic acids, type of labels, use of optional blocking probes, detection of signals, detection (and intensity measurement or spot counting) of signals from the individual cell by flow cytometry or microscopy, presence of the cell in suspension, immobilized on a substrate, or in a tissue, and/or the like. Also, additional features described herein, e.g., in the section entitled "Implementation, applications, and advantages," can be applied to the methods, as relevant.

In another aspect, detection of individual cells of a specified type is performed as described above, but the first and second nucleic acid targets need not be redundant markers for that cell type. The nucleic acid targets can be essentially any desired nucleic acids, including, for example, redundant and/or non-redundant markers for the cell type.

### Detection of nucleic acids in cells in suspension

Another aspect of the invention provides methods for detection of nucleic acids in cells in suspension, for example, rapid detection by flow cytometry. Accordingly, disclosed herein are methods of detecting one or more nucleic acid targets in an individual cell that include: providing a sample comprising the cell, which cell comprises or is suspected of comprising a first nucleic acid target; providing a first label probe comprising a first label; providing at least a first capture probe; hybridizing, in the cell, the first capture probe to the first nucleic acid target, when present in the cell; capturing the first label probe to the first capture probe, thereby capturing the first label probe to the first nucleic acid target; and detecting, while the cell is in suspension, a first signal from the first label. For example, the signal can be conveniently detected by performing flow cytometry.

The methods are useful for multiplex detection of nucleic acids, including simultaneous detection of two or more nucleic acid targets. Thus, the cell optionally comprises or is suspected of comprising a second nucleic acid target, and the methods optionally include: providing a second label probe comprising a second label, wherein a second signal from the second label is distinguishable from the first signal, providing at least a second capture probe, hybridizing in the cell the second capture probe to the second nucleic acid target, when present in the cell, capturing the second label probe to the second capture probe, and detecting the second signal from the second label. Third, fourth, fifth, sixth, etc. nucleic acid targets are similarly simultaneously detected in the cell if desired. Each hybridization or capture step is preferably accomplished for all of the nucleic acid targets at the same time.

The methods permit detection of even low or single copy number targets. Thus, in one class of embodiments, about 1000 copies or less of the first nucleic acid target are present in the cell (e.g., about 100 copies or less, about 50 copies or less, about 10 copies or less, about 5 copies or less, or even a single copy).

Essentially all of the features noted for the methods above apply to these embodiments as well, as relevant; for example, with respect to type of nucleic acid targets, cell type, source of sample, fixation and permeabilization of the cell, washing the cell, denaturation of double-stranded nucleic acids, type of labels, use and configuration of label probes, capture probes, preamplifiers and/or amplifiers (including, e.g., hybridization of two capture probes to a single label probe, preamplifier, or amplifier molecule), use of optional blocking probes, detection of signals, detection (and intensity measurement) of signals from the individual cell by flow cytometry or microscopy, presence of the cell in suspension or immobilized on a substrate, and/or the like.

### Quantifying mRNA in individual cells through imaging and spot counting

In existing DNA FISH assays, the copy numbers of a target DNA sequence are usually visualized and counted on a "one spot per locus" basis either manually or using imaging processing software. However, it has been difficult to employ the same approach to quantify the copy number of mRNA transcripts in individual cells because mRNA, usually around 1000 nucleotides in length, is much shorter than the length of probes required to detect DNA (100,000 nucleotides). This leads to difficulty in the visualization of single RNA molecules. Most existing labeling methodologies cannot attach enough fluorescent label molecules onto an mRNA to generate sufficient signal intensity to visualize a single RNA molecule. Certain aspects of the application described herein, however, employ a probe set system comprising preamplifiers and amplifiers, which significantly increases the number of label molecules that can be attached to a single RNA molecule and enables it to be observed using a normal microscope. Because an RNA molecule is so small in size, it produces a diffraction-limited spot, which is sharp and well-rounded and can be distinguished from background spots by its unique spatial features. In addition, some aspects of the application employ a "cooperative hybridization" capture probe design that effectively reduces background noise caused by non-specific hybridization. The combination of these two factors means each copy of an RNA can be observed under an normal microscope as a sharp, bright spot clearly distinguishable from surrounding background. (See, e.g., Example 1 hereinbelow.) This enables truly reliable quantification of RNA copy number, of even endogenous RNAs, by spot counting either manually or automatically utilizing simple image processing software. Since capture probes can be designed against essentially any RNA, even endogenous RNAs can be quantitated, without need for creation of recombinant reporter constructs that include repetitive probe binding sites. For diagnostic applications in particular, since most human genes express less than 50 copies of their RNA per cell, spot counting is an effective and useful tool for the quantification of gene expression level. While the techniques are particularly useful for quantitating RNA in situ, as discussed in greater detail below they can also be applied to RNA that is not inside any cell.

Disclosed herein are methods of quantitating a target nucleic acid (e.g., an RNA). In the methods, a sample comprising one or more copies of the target nucleic acid is provided. Typically, the target nucleic acid is endogenous to a cell. A plurality of copies of an optically detectable label are captured to each of the one or more copies of the target nucleic acid (e.g., a fluorescent label or an enzyme that is optically detectable, e.g., with fast red substrate). The copies of the label are optically detected. An optical signal focus (or, equivalently, punctum, spot, or dot) is observable for each of the one or more copies of the target nucleic acid, and the one or more resulting foci are counted, thereby quantitating the target nucleic acid.

As noted, the target nucleic acid can be an RNA, e.g., an mRNA, a microRNA, a ribosomal RNA, or the like. The methods can be applied, e.g., to RNA in situ in a cell or free of any cell. Thus, in one class of examples, the sample comprises a cell lysate or other solution comprising the RNA. In another class of examples, the sample comprises the cell to which the target RNA is endogenous, and the capturing, detecting, and counting steps are performed in the cell. Optionally, the RNA is located in the cytoplasm of the cell.

The methods are particularly useful for quantitation of low abundance nucleic acids (e.g., RNAs). Thus, in one embodiment, about 100 copies or less of the target nucleic acid are present in the cell, cell lysate, etc., for example, about 10 copies or less, about 5 copies or less, or even a single copy. As noted, a large number of labels are captured to each molecule. For example, at least about 400 copies of the label can be captured to each of the one or more copies of the target nucleic acid, e.g., at least about 1000 copies, at least about 2000 copies, at least about 4000 copies, or at least about 8000 copies. The label can be, e.g., a fluorescent label or an enzyme (e.g., an enzyme optically detectable using a fluorogenic or chromogenic substrate, e.g., fast red).

The label can be captured to the nucleic acid directly or indirectly. Optionally, the label is provided by providing one or more copies of a label probe, the label probe comprising one or more copies of the label. The label probe can be hybridized directly to the target nucleic acid. Preferably, however, the label probe is indirectly captured, e.g., by providing one or more capture probes, hybridizing a copy of each of the one or more capture probes to each of the one or more copies of the target nucleic acid, and capturing the one or more copies of the label probe to the one or more capture probes. As for the examples above, the label probe can bind directly to the capture probe, or more typically an amplifier or a preamplifier and amplifier serve as intermediates. Optionally, two or more capture probes bind each label probe, amplifier, or preamplifier.

Essentially all of the features noted for the methods above apply to these examples as well, as relevant; for example, with respect to cell type, type of target (including size), source of sample, fixation and permeabilization of the cell, washing the cell, denaturation of double-stranded nucleic acids, type of labels, configuration of label probes, capture probes, preamplifiers and/or amplifiers, label density, use of optional blocking probes, and/or the like.

A related general class of examples provides methods of quantitating a target RNA. In the methods, a sample comprising one or more copies of the target RNA is provided. The target RNA is generally endogenous to a cell. (That is, the RNA is a naturally occurring RNA, as opposed to an RNA produced by human intervention, e.g., using recombinant DNA techniques to insert probe binding sites into an RNA to create a reporter RNA for the purpose of monitoring its presence, location, or quantity in the cell.) A plurality of copies of a fluorescent label are captured to each of the one or more copies of the target RNA. The copies of the label are exposed to excitation light (of an appropriate wavelength for the label), whereupon the copies of the label fluoresce, thereby providing a florescent focus (or, equivalently, punctum, spot, or dot) for each of the one or more copies of the target RNA. The one or more resulting fluorescent foci are counted, thereby quantitating the target RNA. The target RNA can be an mRNA, a microRNA, a ribosomal RNA, a nuclear RNA, a cytoplasmic RNA, or the like.

The methods can be applied, e.g., to RNA in situ in a cell or free of any cell. Thus, in one class of examples, the sample comprises a cell lysate or other solution comprising the RNA. The RNA is optionally bound to a solid support, e.g., before or after capture of the label to the RNA. The RNA can be directly bound to the support, or it can be bound to a moiety that is in turn directly or indirectly bound to the support, e.g., an oligonucleotide or oligonucleotides; see, e.g., the section entitled "Non-specific capture" hereinbelow and U.S. patent application publications 2006/0286583 and 2006/0263769. In another class of examples, the sample comprises the cell to which the target RNA is endogenous, and the capturing, exposing, and counting steps are performed in the cell.

The methods are particularly useful for quantitation of low abundance RNAs. Thus, in one examples, about 100 copies or less of the target RNA are present in the cell, cell lysate, etc., for example, about 10 copies or less, about 5 copies or less, or even a single copy. As noted, a large number of labels are captured to each molecule. For example, at least about 400 copies of the label can be captured to each of the one or more copies of the target RNA, e.g., at least about 1000 copies, at least about 2000 copies, at least about 4000 copies, or at least about 8000 copies.

The label can be captured to the RNA directly or indirectly. Optionally, the label is provided by providing one or more copies of a label probe, the label probe comprising one or more copies of the label. The label probe can be hybridized directly to the target RNA. Preferably, however, the label probe is indirectly captured, e.g., by providing one or more capture probes, hybridizing a copy of each of the one or more capture probes to each of the one or more copies of the target RNA, and capturing the one or more copies of the label probe to the one or more capture probes. As for the examples above, the label probe can bind directly to the capture probe, or more typically an amplifier or a preamplifier and amplifier serve as intermediates. Optionally, two or more capture probes bind each label probe, amplifier, or preamplifier. Counting of the foci can be manual (e.g., involving visual inspection through a microscope) or it can be automated; see, e.g., Raj et al. (2006) "Stochastic MRNA synthesis in mammalian cells" PLoS Biology 4(10) e309 1707-1719 and Vargas et al. (2005) "Mechanism of RNA transport in the nucleus" Proc Natl Acad Sci 102:17008-17013.

Essentially all of the features noted for the methods above apply to these examples as well, as relevant; for example, with respect to cell type, type of target (including size), source of sample, fixation and permeabilization of the cell, washing the cell, denaturation of double-stranded nucleic acids, type of labels, configuration of label probes, capture probes, preamplifiers and/or amplifiers, label density, use of optional blocking probes, and/or the like.

### Detection of nucleic acid splicing in individual cells

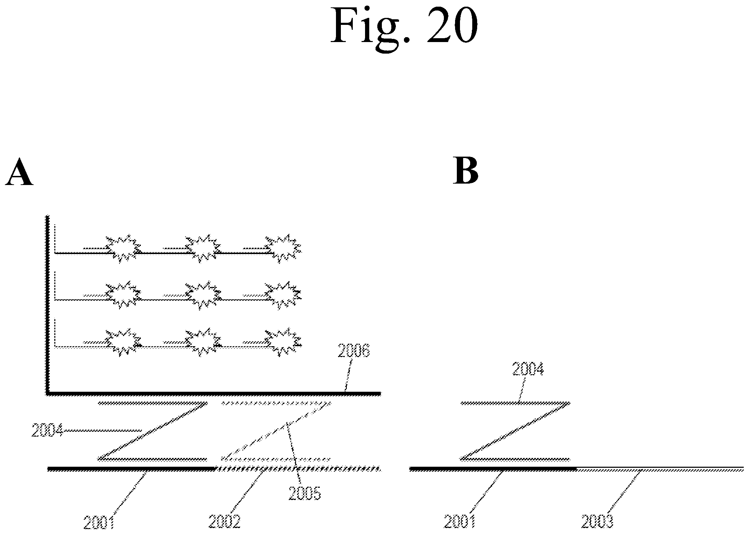

In one aspect, splicing of specific nucleic acid sequences can be detected using the instant technology. In one exemplary embodiment illustrated in **Figure 20** **Panel A**, capture probes 2004 and 2005 are designed to hybridize to a first splice variant. Capture probes 2004 and 2005 are complementary to sequences of the target nucleic acid (the first splice variant) on each side of the splice junction (sequences 2001 and 2002, respectively, e.g., a first exon and a second exon). If the splice has been formed (as in **Figure 20** **Panel A**), the two capture probes align side by side in the hybridization, which provides sufficient hybridization strength in the assay to maintain the attachment of preamplifier 2006, to which are hybridized multiple amplifiers and label probes. (It will be evident that the capture probes could instead hybridize, e.g., to an amplifier or label probe as described elsewhere herein.) Signal is then generated. If the splice is not formed or a different splice has been formed, the two capture probes will not be aligned side by side and there won't be sufficient hybridization strength to maintain the attachment of the preamplifier (or amplifier or label probe) and no signal will be generated. See **Figure 20** **Panel B,** which illustrates a second splice variant that includes sequences 2001 and 2003 (e.g., the first exon and a third exon). Capture probe 2004 but not 2005 can hybridize to the second splice variant. The hybridization of only capture probe 2004 is insufficient to capture preamplifier 2006, and thus the amplifier and label probe, to the second splice variant.

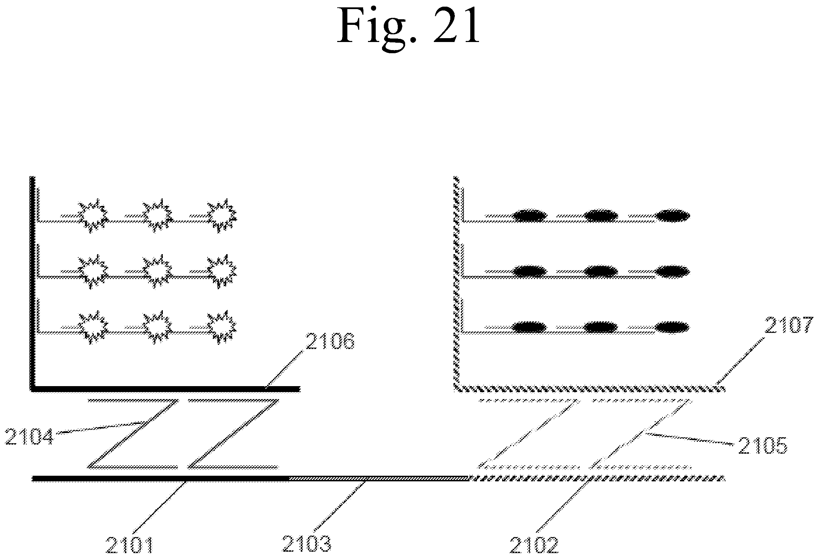

In another example, different regions of the splice variant to be detected are tagged with different labels. This approach can be particularly useful for detection of a specific splice variant where the variant does not include a unique sequence (e.g., where other splice variants of the RNA include the same exons but in different combinations). In the embodiment shown in **Figure 21****,** the target splice variant includes sequences 2101 and 2102 (e.g., two exons present in the target splice variant but not present in combination in other splice variants of the mRNA) separated by sequence 2103. Capture probes 2104 capture preamplifier 2106, to which is hybridized a first amplifier and a first label probe. Capture probes 2105 capture preamplifier 2107, to which is hybridized a second preamplifier and a second label probe. The first and second labels emit different signals. If the splice is formed, the signals generated by the corresponding labels will spatially collocate at a single spot, yielding one new color; other variants that include either 2101 or 2102 but not both will bind only one of the two labels, therefore forming different spots of the two original colors.

In yet another example, one of the capture probes can be complementary to a region of the target splice variant that includes the splice junction, e.g., for variants in which the sequence at the splice junction is unique.

It will be evident that either exemplary configuration can be applied to singleplex or multiplex detection of splice variants.

### Applications to "whole-sample" analysis

This invention concerns in situ detection of nucleic acids in individual cells. However, many features disclosed herein, including, but not limited to, probe set design, multiplexing, detection and quantification, can also be used in whole-sample nucleic acid detection applications. This section described several specific examples of such applications, which are, however, not part of the invention.

### Non-specific capture

In existing hybridization-based assays, such as bDNA, only the target nucleic acid molecules are captured on a solid substrate while other nucleic acids are washed away. Such a measure reduces background noise and thus improves detection specificity. Techniques described herein, however, facilitate detection of a target nucleic acid (singleplex or multiplex) where essentially all nucleic acids in a given sample are immobilized non-specifically. Specific capture probes are designed to attach label molecules onto the target nucleic acid. As a result, only the target nucleic acid will produce signal. Any potential increase of background noise due to non-specific binding of nucleic acids can be more than compensated for by the noise reduction effect of the probe design, e.g., a double-Z design or other approach in which two or more capture probes are used to capture a preamplifier, amplifier, or label probe (see, e.g., the section entitled "Probe selection and design" hereinbelow). Such a probe set design scheme has the advantage of reduced probe set complexity, assay step simplification and cost reduction.

In *in situ* detection applications, nucleic acids are immobilized in cells through a cell fix step employing cross linking chemistry. In whole-sample detection applications, the nucleic acid molecules are released into solution from individual cells. They can be immobilized on solid substrates using any one of the existing nucleic acid immobilization methods, which include, but are not limited to, immobilization on nitrocellulose membranes or silica beads, attachment of poly-T oligo to a substrate surface, which in turn captures the poly-A section of RNA molecules to the substrate, and attachment of a long, random sequence nucleic acid on a substrate surface, which can provide affinity for RNA or DNA molecules.

### Quantification of gene expression level through imaging and spot counting

In existing whole-sample detection technologies, the expression level of a particular gene is quantified by measuring the intensity of the label attached to the target nucleic acid. The detection sensitivity is limited by the noise floor, which is produced by non-specific binding of label molecules or auto-fluorescence. When applying techniques described herein to whole-sample nucleic acid detection, the cells are lysed to release essentially all of the cellular nucleic acid molecules into a sample solution. Then the target nucleic acid molecules can be immobilized on solid substrate either specifically or non-specifically together with other nucleic acids. As described in previous sections, a large number of label probes can be attached to a single target nucleic acid molecule, which produces sufficient signal for each target nucleic acid molecule to be visualized as a spot under a normal microscope. Noise produced by non-specific label attachment or auto-fluorescence appears as larger patches with lower intensity, which are easily distinguishable from the real signal. As a result, the copy number of one or more target nucleic acid can be quantified by spot counting either manually or using simple image processing software. This quantification methodology is especially useful when the total number of target molecules in the sample is very small and the required detection accuracy is high.

### Detection of nucleic acid splicing in whole sample solution

The splicing of nucleic acid molecules resulting in a either specific or non-specific sequence can be detected in similar ways to those described for detection in individual cells, except the nucleic acid molecules are released from cells into sample solutions and are typically immobilized on a substrate before detection.

### COMPOSITIONS AND KITS

The application also describes compositions useful in practicing or produced by the methods. One exemplary class of examples provides a composition that includes a fixed and permeabilized cell, which cell comprises or is suspected of comprising a first nucleic acid target and a second nucleic acid target, at least a first capture probe capable of hybridizing to the first nucleic acid target, at least a second capture probe capable of hybridizing to the second nucleic acid target, a first label probe comprising a first label, and a second label probe comprising a second label. A first signal from the first label is distinguishable from a second signal from the second label. The cell optionally comprises the first and second capture probes and label probes. The first and second capture probes are optionally hybridized to their respective nucleic acid targets in the cell.

The features described for the methods above for indirect capture of the label probes to the nucleic acid targets apply to these examples as well. For example, the label probes can hybridize to the capture probes. In one class of examples, the composition includes a single first capture probe and a single second capture probe, where the first label probe is capable of hybridizing to the first capture probe and the second label probe is capable of hybridizing to the second capture probe. In another class of examples, the composition includes two or more first capture probes, two or more second capture probes, a plurality of the first label probes, and a plurality of the second label probes. A single first label probe is capable of hybridizing to each of the first capture probes, and a single second label probe is capable of hybridizing to each of the second capture probes.

In another aspect, amplifiers can be employed to increase the number of label probes captured to each target. For example, in one class of examples, the composition includes a single first capture probe, a single second capture probe, a plurality of the first label probes, a plurality of the second label probes, a first amplifier, and a second amplifier. The first amplifier is capable of hybridizing to the first capture probe and to the plurality of first label probes, and the second amplifier is capable of hybridizing to the second capture probe and to the plurality of second label probes. In another class of examples, the composition includes two or more first capture probes, two or more second capture probes, a multiplicity of the first label probes, a multiplicity of the second label probes, a first amplifier, and a second amplifier. The first amplifier is capable of hybridizing to one of the first capture probes and to a plurality of first label probes, and the second amplifier is capable of hybridizing to one of the second capture probes and to a plurality of second label probes.

In another aspect, preamplifiers and amplifiers are employed to capture the label probes to the targets. In one class of examples, the composition includes a single first capture probe, a single second capture probe, a multiplicity of the first label probes, a multiplicity of the second label probes, a plurality of first amplifiers, a plurality of second amplifiers, a first preamplifier, and a second preamplifier. The first preamplifier is capable of hybridizing to the first capture probe and to the plurality of first amplifiers, and the second preamplifier is capable of hybridizing to the second capture probe and to the plurality of second amplifiers. The first amplifier is capable of hybridizing to the first preamplifier and to a plurality of first label probes, and the second amplifier is capable of hybridizing to the second preamplifier and to a plurality of second label probes. In a related class of examples, the composition includes two or more first capture probes, two or more second capture probes, a multiplicity of the first label probes, a multiplicity of the second label probes, a multiplicity of first amplifiers, a multiplicity of second amplifiers, a plurality of first preamplifiers, and a plurality of second preamplifiers. The first preamplifier is capable of hybridizing to one of the first capture probes and to a plurality of first amplifiers, the second preamplifier is capable of hybridizing to one of the second capture probes and to a plurality of second amplifiers, the first amplifier is capable of hybridizing to the first preamplifier and to a plurality of first label probes, and the second amplifier is capable of hybridizing to the second preamplifier and to a plurality of second label probes. Optionally, additional preamplifiers can be used as intermediates between a preamplifier hybridized to the capture probe(s) and the amplifiers.

In the above classes of examples, one capture probe hybridizes to each label probe, amplifier, or preamplifier. In alternative classes of related examples, two or more capture probes hybridize to the label probe, amplifier, or preamplifier.

In one class of examples, the composition comprises a plurality of the first label probes, a plurality of the second label probes, a first amplified polynucleotide produced by rolling circle amplification of a first circular polynucleotide hybridized to the first capture probe, and a second amplified polynucleotide produced by rolling circle amplification of a second circular polynucleotide hybridized to the second capture probe. The first circular polynucleotide comprises at least one copy of a polynucleotide sequence identical to a polynucleotide sequence in the first label probe, and the first amplified polynucleotide comprises a plurality of copies of a polynucleotide sequence complementary to the polynucleotide sequence in the first label probe (and can thus hybridize to a plurality of the label probes). The second circular polynucleotide comprises at least one copy of a polynucleotide sequence identical to a polynucleotide sequence in the second label probe, and the second amplified polynucleotide comprises a plurality of copies of a polynucleotide sequence complementary to the polynucleotide sequence in the second label probe. The composition can also include reagents necessary for producing the amplified polynucleotides, for example, an exogenously supplied nucleic acid polymerase, an exogenously supplied nucleic acid ligase, and/or exogenously supplied nucleoside triphosphates (e.g., dNTPs).

The cell optionally includes additional nucleic acid targets, and the composition (and cell) can include reagents for detecting these targets. For example, the cell can comprise or be suspected of comprising a third nucleic acid target, and the composition can include at least a third capture probe capable of hybridizing to the third nucleic acid target and a third label probe comprising a third label. A third signal from the third label is distinguishable from the first and second signals. The cell optionally includes fourth, fifth, sixth, etc. nucleic acid targets, and the composition optionally includes fourth, fifth, sixth, etc. label probes and capture probes.

Essentially all of the features noted for the methods above apply to these embodiments as well, as relevant; for example, with respect to type of nucleic acid target, location of various targets on a single molecule or on different molecules, type of labels, inclusion of optional blocking probes, and/or the like. For example, it is worth noting that the second nucleic acid target optionally comprises a reference nucleic acid. In other embodiments, the first and second nucleic acid targets serve as markers for a specified cell type, e.g., redundant markers.

The cell can be essentially any type of cell from any source, particularly a cell that can be differentiated based on its nucleic acid content (presence, absence, or copy number of one or more nucleic acids). As just a few examples, the cell can be a circulating tumor cell or other tumor cell, a virally infected cell, a fetal cell in maternal blood, a bacterial cell or other microorganism in a biological sample (e.g., blood or other body fluid), or an endothelial cell, precursor endothelial cell, or myocardial cell in blood. For example, the cell can be derived from a bodily fluid, blood, bone marrow, sputum, urine, lymph node, stool, cervical pap smear, oral swab or other swab or smear, spinal fluid, saliva, sputum, semen, lymph fluid, an intercellular fluid, a tissue (e.g., a tissue homogenate), a biopsy, and/or a tumor. The cell is optionally in a tissue, e.g., a tissue section (e.g., an FFPE section) or other solid tissue sample. The cell can be derived from one or more of a human, an animal, a plant, and a cultured cell.

The cell can be present in a mixture of cells, for example, a complex heterogeneous mixture. In one class of examples, the cell is of a specified type, and the composition comprises one or more other types of cells. These other cells can be present in excess, even large excess, of the cell. For example, the ratio of cells of the specified type to cells of all other type(s) in the composition is optionally less than 1:1x104, less than 1:1x105, less than 1:1x106, less than 1:1x107, less than 1:1x108, or even less than 1:1x109.

The cell is optionally immobilized on a substrate, present in a tissue section, or the like. In certain examples, however, the cell is in suspension in the composition. The composition can be contained in a flow cytometer or similar instrument. Additional features described herein, e.g., in the section entitled "Implementation, applications, and advantages," can be applied to the compositions, as relevant.

Another aspect of the applilcaton provides compositions in which a large number of labels are correlated with each target nucleic acid. One general class of examples thus provides a composition comprising a cell, which cell includes a first nucleic acid target, a second nucleic acid target, a first label whose presence in the cell is indicative of the presence of the first nucleic acid target in the cell, and a second label whose presence in the cell is indicative of the presence of the second nucleic acid target in the cell, wherein a first signal from the first label is distinguishable from a second signal from the second label. An average of at least one copy of the first label is present in the cell per nucleotide of the first nucleic acid target over a region that spans at least 20 contiguous nucleotides of the first nucleic acid target, and an average of at least one copy of the second label is present in the cell per nucleotide of the second nucleic acid target over a region that spans at least 20 contiguous nucleotides of the second nucleic acid target.

In one class of embodiments, the copies of the first label are physically associated with the first nucleic acid target, and the copies of the second label are physically associated with the second nucleic acid target. For example, the first label can be part of a first label probe and the second label part of a second label probe, where the label probes are captured to the target nucleic acids.

In one class of embodiments, an average of at least four, eight, or twelve copies of the first label are present in the cell per nucleotide of the first nucleic acid target over a region that spans at least 20 contiguous nucleotides of the first nucleic acid target, and an average of at least four, eight, or twelve copies of the second label are present in the cell per nucleotide of the second nucleic acid target over a region that spans at least 20 contiguous nucleotides of the second nucleic acid target. In one embodiment, an average of at least sixteen copies of the first label are present in the cell per nucleotide of the first nucleic acid target over a region that spans at least 20 contiguous nucleotides of the first nucleic acid target, and an average of at least sixteen copies of the second label are present in the cell per nucleotide of the second nucleic acid target over a region that spans at least 20 contiguous nucleotides of the second nucleic acid target.

Essentially all of the features noted for the embodiments above apply to these embodiments as well, as relevant, for example, with respect to type of labels, suspension of the cell or presence of the cell in a tissue section, and/or the like. The regions of the first and second nucleic acid targets are typically regions covered by a probe, primer, or similar polynucleotide employed to detect the respective target. The regions of the first and second nucleic acid targets optionally span at least 25, 50, 100, 200, or more contiguous nucleotides and/or at most 2000, 1000, 500, 200, 100, 50, or fewer nucleotides. A like density of labels is optionally captured to third, fourth, fifth, sixth, etc. nucleic acid targets. The composition optionally includes PCR primers, a thermostable polymerase, and/or the like, in embodiments in which the targets are detected by multiplex in situ PCR.

Another aspect of the application provides kits useful for practicing the methods. One general class of examples provides a kit for detecting a first nucleic acid target and a second nucleic acid target in an individual cell. The kit includes at least one reagent for fixing and/or permeabilizing the cell, at least a first capture probe capable of hybridizing to the first nucleic acid target, at least a second capture probe capable of hybridizing to the second nucleic acid target, a first label probe comprising a first label, and a second label probe comprising a second label, wherein a first signal from the first label is distinguishable from a second signal from the second label, packaged in one or more containers. Essentially all of the features noted for the embodiments above apply to these examples as well, as relevant; for example, with respect to number of nucleic acid targets, configuration and number of the label and capture probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like. The kit optionally also includes instructions for detecting the nucleic acid targets in the cell and/or identifying the cell as being of a specified type, one or more buffered solutions (e.g., diluent, hybridization buffer, and/or wash buffer), reference cell(s) comprising one or more of the nucleic acid targets, and/or the like.

Disclosed herein is a kit for detecting an individual cell of a specified type from a mixture of cell types by detecting a first nucleic acid target and a second nucleic acid target. The kit includes at least one reagent for fixing and/or permeabilizing the cell, a first label probe comprising a first label (for detection of the first nucleic acid target), and a second label probe comprising a second label (for detection of the second nucleic acid target), wherein a first signal from the first label is distinguishable from a second signal from the second label, packaged in one or more containers. The specified type of cell is distinguishable from the other cell type(s) in the mixture by presence, absence, or amount of the first nucleic acid target in the cell or by presence, absence, or amount of the second nucleic acid target in the cell (that is, the two targets are redundant markers for the specified cell type).

Essentially all of the features noted for the embodiments above apply to these examples as well, as relevant; for example, with respect to number of nucleic acid targets, inclusion of capture probes, configuration and number of the label and/or capture probes, inclusion of preamplifiers and/or amplifiers, inclusion of blocking probes, inclusion of amplification reagents, type of nucleic acid target, location of various targets on a single molecule or on different molecules, type of labels, inclusion of optional blocking probes, and/or the like. The kit optionally also includes instructions for identifying the cell as being of the specified type, one or more buffered solutions (e.g., diluent, hybridization buffer, and/or wash buffer), reference cell(s) comprising one or more of the nucleic acid targets, and/or the like.

### IMPLEMENTATION, APPLICATIONS, AND ADVANTAGES

Various aspects of the invention are described in additional detail below. Exemplary embodiments and applications are also described.

The new technology (methods, compositions, systems, and kits), QMAGEX (Quantitative Multiplex Analysis of Gene Expression in Single Cell), disclosed herein is capable of detection and quantification of multiple nucleic acids within individual cells. The technology is significantly different from existing ISH technology in several aspects, although they both can measure mRNA expression in individual cells. First, cells optionally remain in suspension status during all or at least most of the assay steps in the assays of the present invention, which greatly improves assay hybridization kinetics, resulting in better reproducibility and shorter assay time. Second, the instant technology has the capability for analyzing the expression of multiple mRNA transcripts within cells simultaneously and quantitatively. This is highly desirable, since, for example, detection of multiple tumor marker genes could greatly improve the accuracy of CTC identification (Mocellin et al., 2004) and greatly reduce the false positive rate. Quantitative analysis of gene expression level could not only further aid in discriminating the CTC from other types of cells but also could help in distinguishing the type and source of primary tumors as well as the stages of tumor progression. Third, the instant technology enables the use of a flow cytometer as the base for detection, which, compared with microscope-based detection instruments, offers higher throughput. In addition, the flow cytometer is capable of sorting out cells, e.g., tumor cells, for further study. Subsequent to the detection and quantification of mRNA expression, isolation of the CTC or other cells may be advantageous for further identity confirmation or for additional cytological and molecular analysis. Fourth, the instant technology has vastly improved detection sensitivity and reproducibility, and is capable of single copy gene detection and quantification. In addition, the instant technology uses a standard, generic set of probe labeling and detection technology (e.g., the same set of preamplifiers, amplifiers, and label probes can be used to detect multiple different sets of nucleic acid targets, requiring only synthesis of a new set of capture probes for each new set of nucleic acid targets), and optionally uses standardized procedures for cell fixation and permeation and for hybridization and washing. Furthermore, the technology can include built-in internal controls for assay specificity and efficiency.

The instant technology can be used not only for the detection and enumeration of rare CTC in blood samples or other body fluids, but also for any type of rare cell identification and enumeration events. Applications include, but are not limited to: detection of minimal residual disease in leukemia and lymphoma; recurrence monitoring after chemotherapy treatment (Hess et al.); detection of other pre-cancerous cells, such as the detection of HPV-containing cervical cells in body fluids; detection of viral or bacterial nucleic acid in an infected cell; detection of fetal cells in maternal blood; detection of micro-tumor lesions during early stage of tumor growth; or detection of residual tumor cells after surgery for margin management. In all of these cases, target cell specific gene expression is likely to be buried in the background of large numbers of heterogeneous cell populations. As a result, microarray or RT-PCR based expression analysis, which require the isolation of mRNA from a large population of cells, will have difficulty detecting the presence of those rare cell events accurately or reliably, whereas the invented technology can readily be applied.

It should also be noted that although single cell detection and quantification of multiple mRNA transcripts is illustrated here as the main application, such technology is equally applicable to detection of other rare cell events that include changes in chromosomal DNA or cellular nucleic acid content. Examples include, but are not limited to, detection of her-2/neu gene amplification, detection of Rb gene deletion, detection of somatic mutations, detection of chromosome translocation such as in chronic myelogenous leukemia (BCR-ABL), or detection of HPV insertion to chromosomal DNA of cervical cancer cells.

Finally, the probe design, multiplexing and amplification aspects of the instant technology can be applied in quantitative, multiplex gene expression analysis and in measuring chromosomal DNA changes at a single cell level in solid tissue sections, such as formalin-fixed, paraffin embedded (FFPE) tissue samples.

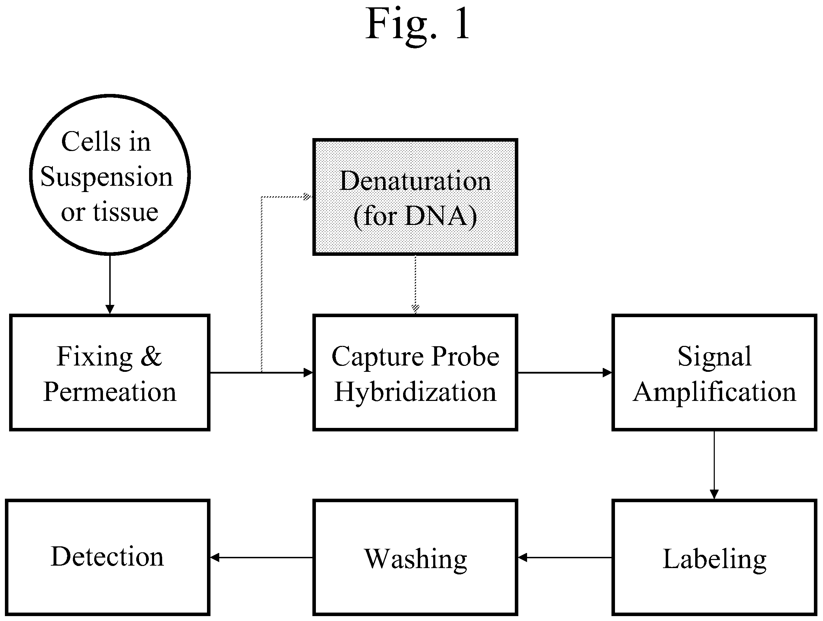

The QMAGEX technology comprises an assay and optional associated apparatus to implement the assay in an automated fashion. **Figure 1** illustrates major elements of the QMAGEX assay work flow, which, for one exemplary embodiment in which the cells are in suspension and amplifiers are employed, include:

Fixation and Permeation: Cells in the sample are fixed and permeated (permeabilized) in suspension. The fixation step immobilizes nucleic acids (e.g., mRNA or chromosomal DNA) and cross-links them to the cellular structure. Then the cell membrane is permeabilized so that target-specific nucleic acid probes and signal-generating particles, such as fluorescently labeled nucleic acid probes, can enter the cell and bind to the target.

Denaturation: If the detection target is double-stranded chromosomal DNA, a denaturation step is added to convert the double-stranded target into single-stranded DNA, ready to be bound with the target-specific probes.

Capture Probe Hybridization: Carefully selected target-specific capture probes or probe sets are hybridized to the target nucleic acids. The capture probes serve to link the target molecules specifically to signal-generating particles. The technology enables multiple target genes in the cell to be recognized by different probe sets simultaneously and with a high degree of specificity.

Signal Amplification: Signals from target molecules are amplified by binding a large scaffold molecule, an amplifier, to the capture probes or probe sets. Each scaffold has multiple locations to accept label probes and signal-generating particles. In a multiplex assay, multiple distinct amplifiers are used.

Labeling: Label probes, to which signal generating particles (labels) are attached, hybridize to the amplifier in this step. In a multiplex assay, multiple distinct label probes are used.

Washing: The excess probes or signal generating particles that are not bound or that are nonspecifically bound to the cells are removed through a washing step, which reduces background noise and improves the detection signal to noise ratio. Additional washing steps may be added during the capture probe hybridization or signal amplification steps to further enhance the assay performance.

Detection: The labeled suspension cells are detected using Fluorescent Activated Cell Sorting (FACS) or a flow cytometer, or are immobilized on a solid surface and detected using a microscope or scanner based instrument.

In the following section, major elements of the QMAGEX technology will be described in detail. In the following, the term label probe refers to an entity that binds to the target molecule, directly or indirectly, and enables the target to be detected by a readout instrument. The label probe, in general, comprises a nucleic acid or modified nucleic acid molecule that binds to the target, directly or indirectly, and one or more "signal generating particle" (i.e., label) that produces the signal recognizable by the readout instrument. Preferably, the label probe comprises a relatively short, single strand oligo with the label attached to one end of the oligo strand. In another embodiment, the label probe can also be a relatively larger molecule, which can be a longer, single strand oligo or a molecule of branched structure, enabling the attachment of multiple labels. In indirect mode, the label probe can either be attached to the target molecule through binding to a capture probe directly or through binding to an amplifier that is in turn linked to a capture probe. Exemplary signal-generating particles (labels) include, but are not limited to, fluorescent molecules, nano-particles, radioactive isotopes, chemiluminescent molecules (e.g., digoxigenin, dinitrophenyl). Fluorescent molecules include, but are not limited to, fluorescein (FITC), cy3, cy5, alexa dyes, phycoerythrin, etc. Nano-particles include, but are not limited to, fluorescent quantum dots, scattering particles, etc. The term capture probe refers to a nucleic acid or a modified nucleic acid that links the target to a specific type of label probe, directly or indirectly. The capture probe has one section of sequence complimentary to the target and one or multiple sections of sequences complementary to label probes or amplifiers or preamplifiers. The term "capture probe set" refers to multiple nucleic acids or modified nucleic acids that link a target to a specific type of label probe, directly or indirectly, for increased assay sensitivity. The term amplifier refers to a large scaffold molecule(s) that binds to one or more capture probes or to a preamplifier on one side and to multiple label probes on another side.

### Fixation

In this step, the nucleic acids are immobilized within cells by cross-linking them within the cellular structure. There are a variety of well known methods to fix cells in suspension with a fixative reagent and to block the endogenous RNase activities, which can be adapted for use in the present invention. Fixative reagents include formalin (formaldehyde), paraformaldehyde, gluteraldehyde, ethanol, methanol, etc. One common fixative solution for tissue sections includes 0.25% gluteraldehyde and 4% paraformaldehyde in phosphate buffer. Another common fixative solution for tissue sections includes 50% ethanol, 10% formalin (containing 37% formaldehyde), and 5% acetic acid. Different combinations of the fixative reagents at various concentrations are optionally tested to find the optimal composition for fixing cells in suspension, using techniques well known in the art. Duration of the fixing treatment can also be optimized. A number of different RNase inhibitors can be included in the fixative solution, such as RNAlater (Ambion), citric acid or LiCl, etc.

### Permeation

Fixation results in cross-linking of the target nucleic acids with proteins or other cellular components within cells, which may hinder or prevent infiltration of the capture probes into the cells and mask the target molecules for hybridization. The assays of the invention thus typically include a follow-on permeation step to enable in-cell hybridization. One technique involves the application of heat for varying lengths of time to break the cross-linking. This has been demonstrated to increase the accessibility of the mRNA in the cells for hybridization. Detergents (e.g., Triton X-100 or SDS) and Proteinase K can also be used to increase the permeability of the fixed cells. Detergent treatment, usually with Triton X-100 or SDS, is frequently used to permeate the membranes by extracting the lipids. Proteinase K is a nonspecific protease that is active over a wide pH range and is not easily inactivated. It is used to digest proteins that surround the target mRNA. Again, optimal concentrations and duration of treatment can be experimentally determined as is well known in the art. A cell washing step can follow, to remove the dissolved materials produced in the permeation step.

Optionally, prior to fixation and permeation, cells in suspension are collected and treated to inactivate RNase and/or to reduce autofluorescence. DEPC treatment (e.g. Braissant and Wahli (1988) "A simplified in situ hybridization protocol using non-radioactively labeled probes to detect abundant and rare mRNAs on tissue sections" Biochemica 1:10-16) and RNAlater (Ambion, Inc.) have been demonstrated to be effective in stabilizing and protecting cellular RNA. Sodium borohydride and high heat have also been shown to preserve the integrity of RNA and to reduce autofluorescence, facilitating the detection of genes expressed at a low level (Capodieci et al. (2005) "Gene expression profiling in single cells within tissue" Nat Methods 2(9):663-5). Other methods of reducing cellular autofluorescence such as trypan blue (Mosiman et al. (1997) "Reducing cellular autofluorescence in flow cytometry: an in situ method" Cytometry 30(3):151-6) or singly labeled quencher oligonucleotide probe (Nolan et al. (2003) "A simple quenching method for fluorescence background reduction and its application to the direct, quantitative detection of specific mRNA" Anal Chem. 2003 75(22):6236-43) are optionally employed.

### Capture Probe Hybridization

In this assay step, the capture probe or capture probe set binds to the intended target molecule by hybridization. One indicator for a successful target hybridization is specificity, i.e. the capture probes or probe sets should substantially only link the label probes to the specific target molecule of interest, not to any other molecules. Probe selection and design are important in achieving specific hybridization.

### Probe Selection and Design

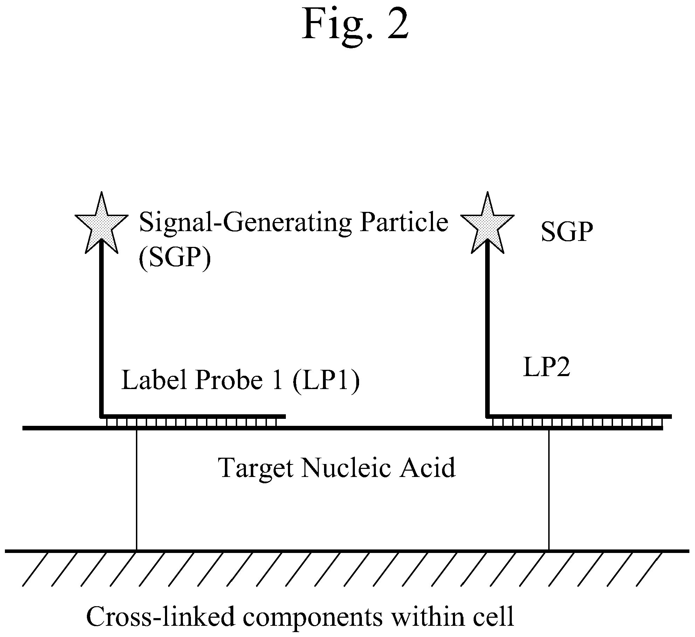

The assays may employ two types of approaches in probe design to link the target nucleic acids in cells to signal generating particles: "direct labeling" and "indirect labeling". In the direct labeling approach, the target molecule hybridizes to or captures one or more label probes (LP) directly. The LPs contain the signal-generating particles (SGP), as shown in **Figure 2****.** A different LP needs to be used to attach additional SGP at different positions on the target molecule. In order to ensure hybridization specificity, the label probe is preferably stringently selected to ensure that it does not cross-hybridize with nonspecific nucleic acid sequences.

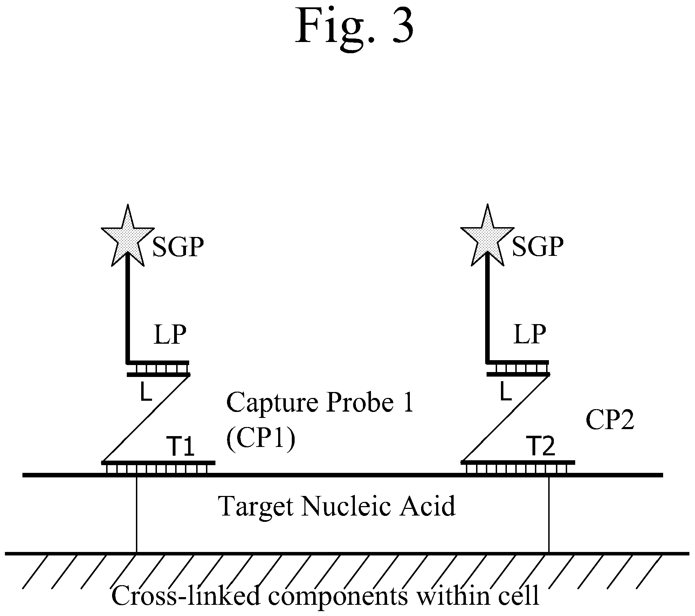

In the indirect labeling approach, an additional capture probe (CP) is employed. An example is shown in **Figure 3****.** The target molecule captures the label probe through the capture probe. In each capture probe, there is at least one section, T, complementary to a section on the target molecule, and another section, L, complementary to a section on the label probe. The T and L sections are connected by a section C. To attach more SGPs to different positions on the same target molecule, different capture probes are needed, but the label probe can remain the same. The sequence of L is carefully selected to ensure that it does not cross-hybridize substantially with any sequences in the nucleic acids in cells. In a further embodiment, the L portion of the capture probe and the label probe contain chemically modified or nonnatural nucleotides that do not hybridize with natural nucleotides in cells. In another embodiment, L and the label probe (or a portion thereof) are not even nucleic acid sequences. For example, L can be a weak affinity binding antibody that recognizes the signal-generating probe, which in this case is or includes an antigen; L can be covalently conjugated to an oligonucleotide that comprises the T section of the capture probe. Optionally, for two adjacent capture probes, the T sections hybridize to the target and two of the low affinity binding antibody binds to the antigen on the label probe at the same time, which results in strong affinity binding of the antigen. The capture and label probes are specific for a target gene of interest. Multiple capture probes (probe set) can be bound to the same target gene of interest in order to attach more signal-generating particles for higher detection sensitivity. In this situation, the probe set for the same target gene can share the same label probe.

Although both approaches can be used in the instant technology, the indirect capture approach is preferred because it enables the label probe to be target independent and further disclosure will show that it can offer better specificity and sensitivity.

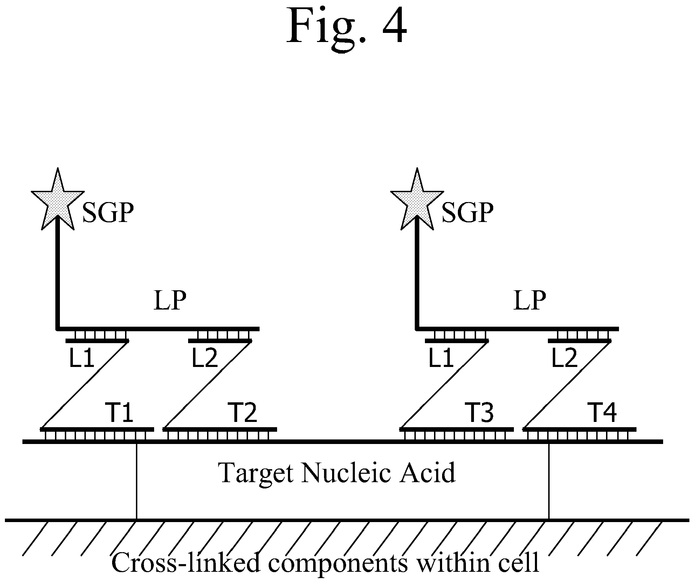

In a further indirect capture embodiment shown in **Figure 4****,** two adjacent capture probes are incorporated in a probe set targeting a gene of interest. T1 and T2 are designed to be complementary to two unique and adjacent sections on the target nucleic acid. L1 and L2, which can be different or the same, are complementary to two adjacent sections on the label probe. Their binding sections, T, L or both, are designed so that the linkage between the label probe and the target is unstable and tends to fall off at hybridization temperature when only one of the capture probes is in place. Such a design should enable exceptional specificity because the signal-generating label probe can only be attached to the target gene of interest when two independent capture probes both recognize the target and bind to the adjacent sequences or in very close proximity of the target gene. In one embodiment, the melting temperature, Tm, of the T sections of the two capture probes are designed to be significantly above the hybridization temperature while the Tm of the L sections is below the hybridization temperature. As a result, T sections bind to the target molecule strongly and stably during hybridization, while L sections bind to the label probe weakly and unstably if only one of the capture probes is present. However, if both capture probes are present, the combination of L1 and L2 holds the label probe strongly and stably during hybridization. For example, the T sections can be 20-30 nucleotides in length while the L sections are 13-15 nucleotides in length; C can be 0 to 10 nucleotides in length, e.g., 5 nucleotides. In another embodiment, Tm of the T sections is below hybridization temperature while Tm of the L sections is substantially above. In the same way, the linkage between the label probe and the target can only survive the hybridization when both capture probes are hybridized to the target in a cooperative fashion. See Example 1 hereinbelow and U.S. patent application publication 2007/0015188 entitled "Multiplex detection of nucleic acids" by Luo et al. for additional details on design of capture probes.

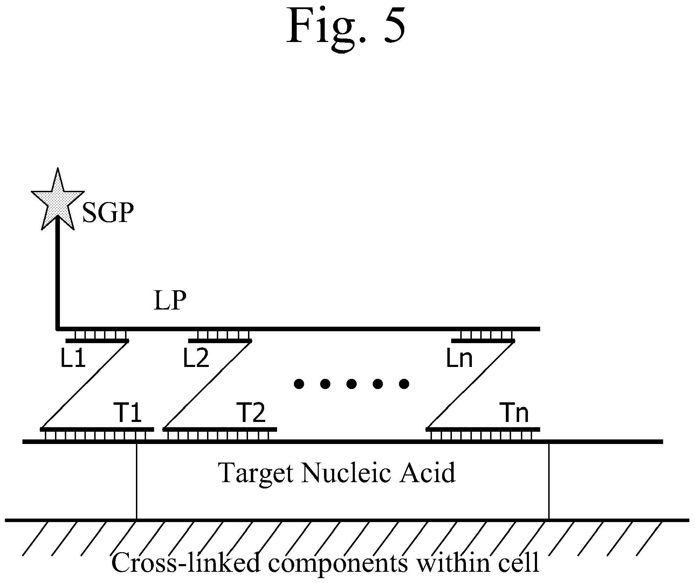

In another embodiment, three or more of the target nucleic acid specific, neighboring capture probes are used for the stable capture of one label probe within cells **(****Figure 5****).** The basic design of the probes is the same as discussed above, but the capture of one signal-generating probe should have even higher specificity than when two neighboring probes are used since now three independent probes have to bind to the same target molecule of interest in neighboring positions in order to generate signal.

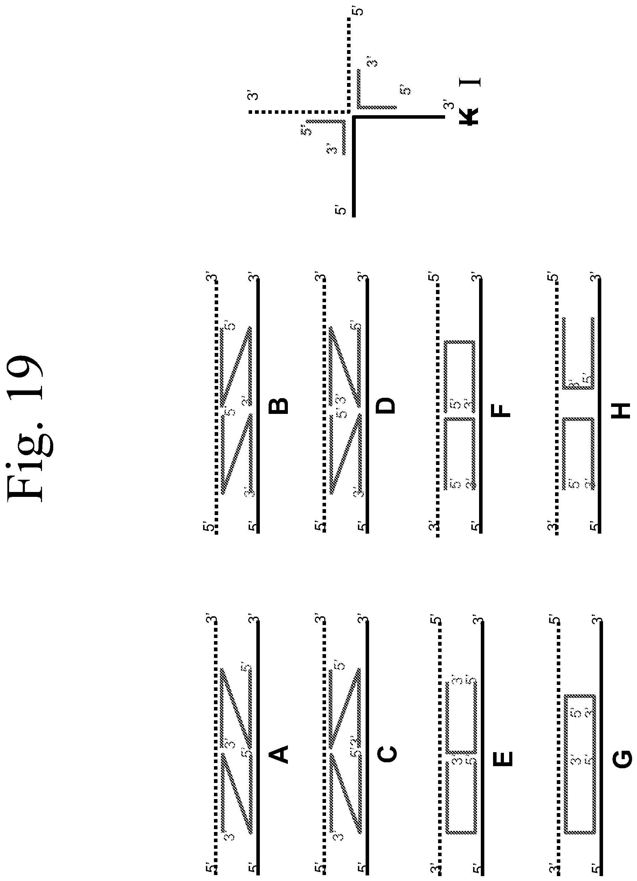

It will be evident that, while the embodiments above are described in terms of capture probe configurations such as those shown in **Figures 3-5** and **Figure 19** **Panels A-B,** other capture probe configurations can readily be employed. Additional exemplary capture probe configurations that can be adapted to the practice of the present invention are illustrated in **Figure 19** **Panels C-I.** As for the embodiments above, two, three, or more such capture probes can bind to a single label probe, amplifier, or preamplifier. Also as described above, optionally sections T, L, or both are designed such that stable capture of the label probe, amplifier, or preamplifier requires binding of more than one of the capture probes. For example, the T sections can be 20-30 nucleotides in length while the L sections are 13-15 nucleotides in length; C can be 0-10 nucleotides in length, e.g., 5 nucleotides. It is worth noting that, in certain configurations, the ends of adjacent capture probes can optionally be ligated to each other when the capture probes are bound to the target nucleic acid and/or the label probe, amplifier, or preamplifier; see **Figure 19** **Panels C, D and G.**

### Multiplexing

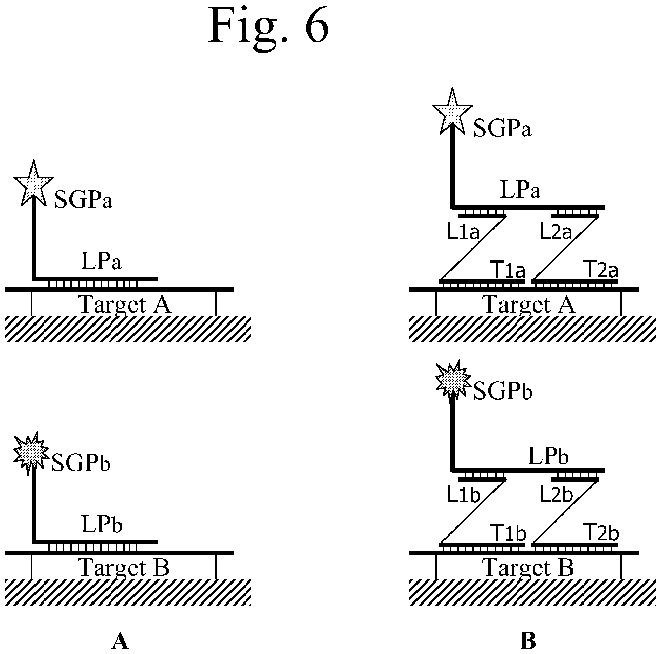

To perform multiplexed detection for more than one target gene, e.g., as shown in Figure 6, each target gene has to be specifically bound by different capture and label probes. In addition, the signal generating particle (the label) attached to the label probe should provide distinctively different signals for each target that can be read by the detection instrument. In the direct labeling approach (e.g., **Figure 6** **Panel A**), suitable label probes with minimal cross-hybridization can be harder to find because each label probe has to be able to bind to the target strongly but not cross-hybridize to any other nucleic acid molecules in the system. For this approach to provide optimal results, the target binding portion of the label probe should be judiciously designed so that it does not substantially cross-hybridize with nonspecific sequences. In the indirect labeling approach (e.g., **Figure 6** **Panel B**), because of the unique multiple capture probe design approach, even when one capture probe binds to a nonspecific target, it will not result in the binding of the label probe to the nonspecific target. The assay specificity can be greatly improved. Thus the capture probe design illustrated in **Figure 4** and **Figure 5** is typically preferred in some multiplex assay applications. For example, the signal-generating particles attached to different target genes are different fluorescent molecules with distinctive emission spectra.

The capacity of the instant technology to measure more than one parameter simultaneously can enable detection of rare cells in a large heterogeneous cell population. As noted above, the concentration of CTC is estimated to be in the range of one tumor cell among every 106-107 normal blood cells. In existing FACS based immunoassays, on the other hand, random dye aggregation in cells may produce one false positive cell count in every ten thousand cells. Such an assay can thus not be used for CTC detection due to the unacceptably high false positive rates. This problem can be solved elegantly using the instant technology. Particularly, expression of more than one tumor genes are used as the targets for multiplex detection. Only cells that express all the target genes are counted as tumor cells. In this way, the false positive rate of the CTC detection can be dramatically reduced. For example, since dye aggregation in cells is a random event, if the false positive rate of a single color detection is 10-4, the false positive rate for two color or three color detection can be as low as 10-8 or 10-12, respectively. In situations where the relative levels of expression of the target genes are known, these relative levels can be measured using the multiplex detection methods disclosed herein and the information can be used to further reduce the false positive rate of the detection.

In another example, schematically illustrated in **Figure 7** **Panel A**, more than one signal-generating particles are linked to the same target nucleic acid. These particles generate distinct signals in the detection instrument. The relative strengths of these signals can be pre-determined by designing the number of each type of particles attached to the target. The number of signal-generating particles on a target can be controlled in probe design by changing the number of probe sets or employing different signal amplification methods, e.g., as described in the following section. The rare cells are identified only when the relative signal strengths of these particles measured by the detection instrument equal the pre-determined values. This embodiment is useful when there are not enough suitable markers or when their expression levels are unknown in a particular type of rare cells. In yet another embodiment, shown in **Figure 7** **Panel B,** the same set of signal-generating particles are attached to more than one target. The relative signal strengths of the particle set are controlled to be the same on all selected targets. This embodiment is useful in situations in which the rare cell is identified when any of the target molecules are present. In yet another embodiment, depicted in **Figure 7** **Panel C**, each target molecule has a set of signal generating particles attached to it, but the particle sets are distinctively different from target to target.

The detection of multiple target nucleic acid species of interest can be applied to quantitative measurement of one target. Due to different sample and experimental conditions, the abundance of a particular target molecule in a cell normally may not be determined quantitatively through the detection of the signal level associated with the target alone in embodiments in which intensity levels are measured. More precise measurement can potentially be accomplished by normalizing the signal of a gene of interest to that of a reference/housekeeping gene. A reference/housekeeping gene is defined as a gene that is generally always present or expressed in cells. The expression of the reference/housekeeping gene is generally constitutive and tends not to change under different biological conditions. 18S, 28S, GAPD, ACTB, PPIB etc. have generally been considered as reference or housekeeping genes, and they have been used in normalizing gene expression data generated from different samples and/or under varying assay conditions.

In another embodiment, a special label probe set can be designed that does not bind to any capture probe or target specifically. The signal associated to this label probe can be used to establish the background of hybridization signal in individual cells. Thus the abundance of a particular target molecule can be quantitatively determined by first subtracting the background hybridization signal, then normalizing against the background subtracted reference/housekeeping gene hybridization signal.

In yet another embodiment, two or more chromosomal DNA sequences of interest can be detected simultaneously in cells. In the detection of multiple DNA sequences in cells, the label probes for the DNA sequences are distinct from each other and they do not cross-hybridize with each other. In embodiments in which cooperative indirect capture is employed, because of the design scheme, even when one probe binds to a nonspecific DNA sequence, it will not result in the capture of the signal-generating probe to the nonspecific DNA sequences.

In yet another example, the detection of multiple target chromosomal DNA sequences of interest enables quantitative analysis of gene amplification, gene deletion, or gene translocations in single cells. This is accomplished by normalizing the signal of a gene of interest to that of a reference gene. The signal ratio of the gene of interest to the reference gene for a particular cell of interest is compared with the ratio in reference cells. A reference gene is defined as a gene that stably maintains its copy numbers in the genomic DNA. A reference cell is defined as a cell that contains the normal copy number of the gene of interest and the reference gene. If the signal ratio is higher in the cells of interest in comparison to the reference cells, gene amplification is detected. If the ratio is lower in the cells of interest in comparison to the reference cells, then gene deletion is detected.

### Signal Amplification & Labeling

The direct labeling approach depicted in **Figure 2** and **Figure 6** Panel A offers only limited sensitivity because only a relatively small number of signal-generating particles (labels) can be attached to each label probe.

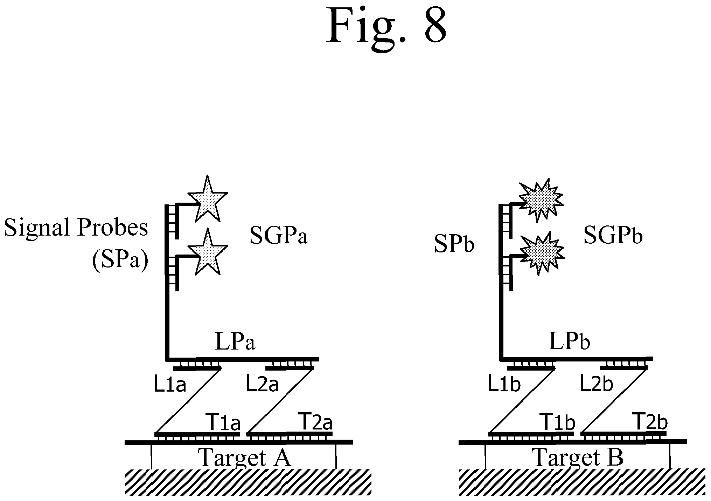

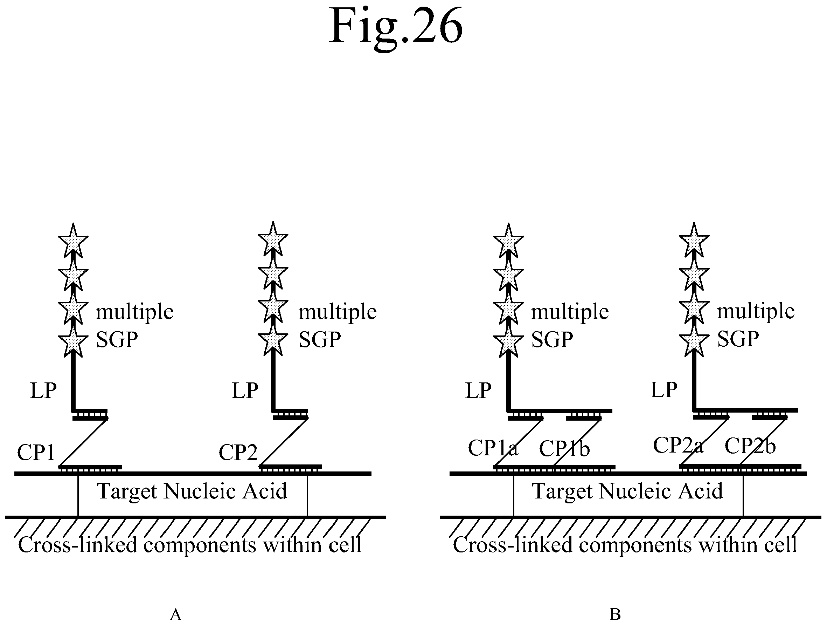

It is evident that the more labels or signal generating particles (SGP) being attached to a copy of the nucleic acid target, stronger will be the signal, more sensitive will be the assay. In one embodiment, the label probe is short, single stranded oligo with a SGP attached to one end of the oligo. In another embodiment, the label probe is short, single stranded oligo with a SGP attached to each end of the oligo, thus effectively doubling the signal strength. In yet another embodiment, shown in **Figure 26****,** the label probe is longer, single stranded oligo with many SGPs attached to the oligo. One way to achieve this is to use in vitro transcribed RNA that incorporates signal-generating particles. Panel A shows a large label probe comprises multiple label particles or molecules captured by a capture probe. Panel B shows a single large label probe captured by each of two or more capture probes. **Figure 8** shows the use of large label probe with an amplifier captured simultaneously to two or more capture probes.

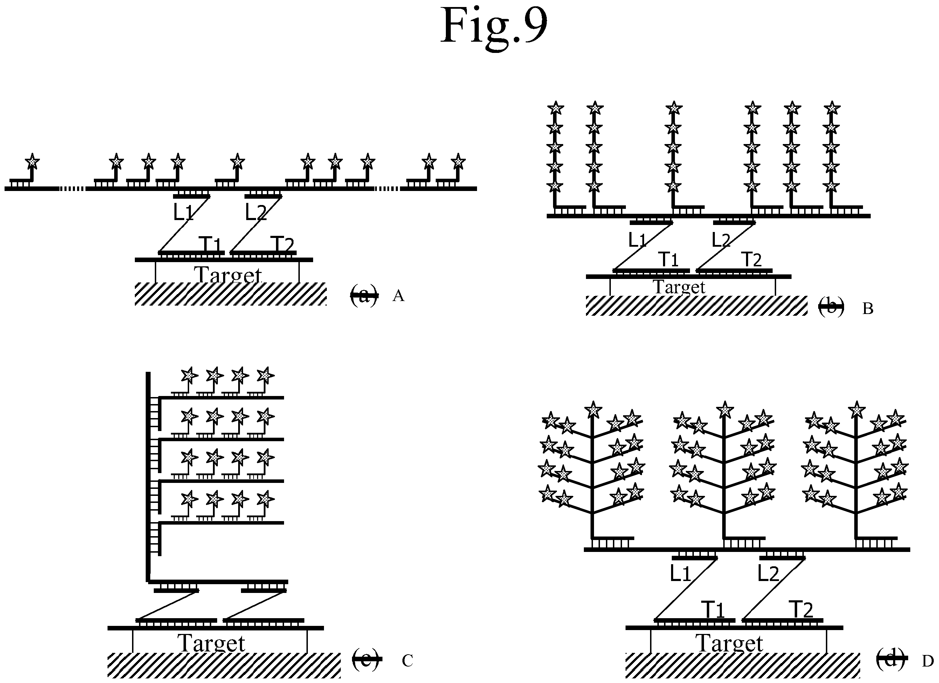

The "indirect labeling" approach not only can improve specificity as described above but also can be used to improve the detection sensitivity. In this approach, the label probe is hybridized or connected to an amplifier molecule, which provides many more attachment locations for label probes. The structure and attachment method of the amplifier can take many forms. **Figure 9** **Panels A-D** show a number of amplification schemes as illustrative examples. In Panel A, multiple singly-labeled label probes bind to the amplifier. In Panel B, multiple multiply-labeled label probes bind to the amplifier. In Panel C, multiple singly-labeled label probes bind to the amplifier, and multiple copies of the amplifier are bound to a preamplifier. In one particular embodiment, the amplifier is one or multiple branched DNA molecules (Panel D). The sequence of the label probe is preferably selected carefully so that it does not substantially cross-hybridize with any endogenous nucleic acids in the cell. In fact, the label probe does not have to be a natural polynucleotide molecule. Chemical modification of the molecule, for example, inclusion of nonnatural nucleotides, can ensure that the label probe only hybridizes to the amplifier and not to nucleic acid molecules naturally occurring in the cells. In multiplex assays, distinct amplifiers and label probes will be designed and used for the different targets.

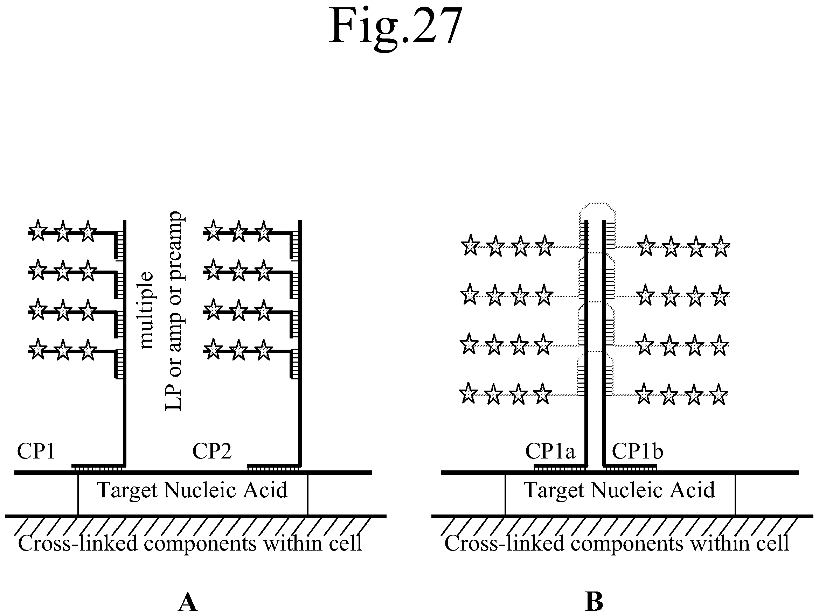

It is also possible to increase the number of SGPs attached to a copy of the nucleic acid target by increase the size of capture probes. In one embodiment, the capture probe has one section that has sequence complementary to that of label probe (or amplifier or preamplifier). Therefore, maximum one label probe or amplifier or preamplifier can be hybridized to the capture probe. In another embodiment, as shown in **Figure 27****,** a larger capture probe is used, which has multiple sections complementary to that of label probe or amplifier or preamplifier. Panel A shows one capture probe captures multiple label probes, or amplifiers or preamplifiers.

The desire to improve assay sensitivity may lead to attempts to employ larger and larger label probe molecules with ever increased number of labels attached to the probe. However, it is more difficult to remove large molecules, especially those with complex branched structure, that are not specifically bound to target from cells in an in situ detection assay. Those nonspecifically bound label probes will increase background which could lead to high false positives and reduced assay specificity. This instant invention uses a probe set that comprises multiple elements including label probe, capture probe and optionally preamplifier and amplifier. The invented assay builds a large structure of molecules in situ that enables a large number of labels (or SGPs) to attach to each copy of the target while at the same time, keep each component molecule relatively small and simple. The "collaborative hybridization" technique described above is employed to build the large "scaffold" of molecules. The key feature of this technique is to that the condition of forming such a scaffold has to be very stringent and specific; once such a structure is formed, it should be very stable. Figure 27 Panel B shows a unique scaffold structure where two or more capture probes collaboratively capture multiple labels, or amplifiers or preamplifiers.

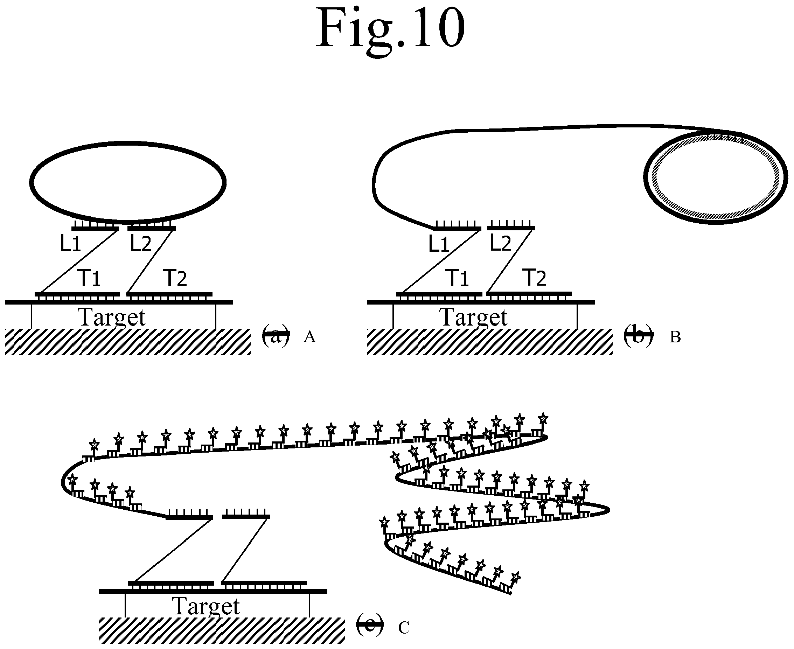

In one embodiment, as schematically illustrated in **Figure 10****,** a circular polynucleotide molecule is captured by the capture probe set. Along the circle, there can be one sequence or more than one repeat of the same sequence that binds to label probe (**Figure 10** **Panel A**). In the signal amplification step of the assay, a rolling circle amplification procedure (Larsson et al, 2004) is carried out. As the result of this procedure, a long chain polynucleotide molecule attached to the capture probes is produced (**Figure 10** **Panel B).** There are many repeating sequences along the chain, on which label probes can be attached by hybridization (**Figure 10** **Panel C**). In multiplex assays, distinct capture probes, rolling circles, and label probes will be designed and used.

In one embodiment, a portion of the signal-generating probe can be PCR-amplified. In another embodiment, each portion of multiple signal-generating probes can be PCR-amplified simultaneously.

Although a specific capture approach (indirect labeling with capture probe pairs) has been used to illustrate the labeling and amplification schemes in **Figures 8** **and** **9****,** it is important to note that any other probe capture approaches, direct or indirect, described in previous sections can be used in combination with the labeling and amplification schemes described in these sections. The capture probe, labeling methods, and amplifier configurations described above are independent of each other and can be used in any combination in a particular assay design, e.g., in in situ or whole sample detection.

### Hybridization Conditions

The composition of the hybridization solution can affect efficiency of the hybridization process. Hybridization typically depends on the ability of the oligonucleotide to anneal to a complementary mRNA strand below its melting point (Tₘ). The value of the Tm is the temperature at which half of the oligonucleotide duplex is present in a single stranded form. The factors that influence the hybridization of the oligonucleotide probes to the target nucleic acids can include temperature, pH, monovalent cation concentration, presence of organic solvents, etc. A typical hybridization solution can contain some or all of the following reagents, e.g., dextran sulfate, formamide, DTT (dithiothreitol), SSC (NaCl plus sodium citrate), EDTA, etc. Other components can also be added to decrease the chance of nonspecific binding of the oligonucleotide probes, including, e.g., single-stranded DNA, tRNA acting as a carrier RNA, polyA, Denhardt's solution, etc. Exemplary hybridization conditions can be found in the art and/or determined empirically as well known in the art. See, e.g., U.S. patent application publication 2002/0172950, Player et al. (2001) J. Histochem. Cytochem. 49:603-611, and Kenny et al. (2002) J. Histochem. Cytochem. 50:1219-1227, which also describe fixation, permeabilization, and washing.

An additional prehybridization is optionally carried out to reduce background staining. Prehybridization involves incubating the fixed tissue or cells with a solution that is composed of all the elements of the hybridization solution, minus the probe.

### Washing

Following the labeling step, the cells are preferably washed to remove unbound probes or probes which have loosely bound to imperfectly matched sequences. Washing is generally started with a low stringency wash buffer such as 2 X SSC + 1 mM EDTA (1 X SSC is 0.15M NaCl, 0.015M Na-citrate), then followed by washing with higher stringency wash buffer such as 0.2 X SSC + 1 mM EDTA or 0.1 X SSC + 1 mM EDTA.

Washing is important in reducing background noise, improving signal to noise ratio of and quantification with the assay. Established washing procedures can be found, e.g., in Bauman and Bentvelzen (1988) "Flow cytometric detection of ribosomal RNA in suspended cells by fluorescent in situ hybridization" Cytometry 9(6):517-24 and Yu et al. (1992) "Sensitive detection of RNAs in single cells by flow cytometry" Nucleic Acids Res. 20(1):83-8.

Washing can be accomplished by executing a suitable number of washing cycles, i.e., one or more. Each cycle in general includes the following steps: mixing the cells with a suitable buffer solution, detaching non-specifically bound materials from the cells, and removing the buffer together with the waste. Each step is described in more detail below.

Mix the cells with wash buffer: In some assays, the cells are immobilized on the surface of a substrate before being washed. In such cases, the washing buffer is mixed together with the substrate surface. In many other embodiments, the cells to be washed are free-floating. The washing buffer is added to cell pellets or to the solution in which the cells are floating.

Detach non-specifically bound materials from cells: Any of a number of techniques can be employed here to reduce nonspecific binding after cell permeability treatment and probe hybridization to encourage non-specifically bound probes to detach from the cells and dissolve into the wash buffer. These include raising the temperature to somewhere just below the melting temperature of the specifically bound probes and employing agitation using a magnetic or mechanical stirrer or perturbation with sonic or ultrasonic waves. Agitation of the mixture can also be achieved by shaking the container with a rocking or vortex motion.

Remove buffer together with waste: Any convenient method can be employed to separate and remove the washing buffer and waste from the target cells in the sample. For example, the floating cells or substrates that the cells bound to are separated from the buffer and waste through centrifugation. After the spin, the cells or substrates form a pellet at the bottom of the container. The buffer and waste are decanted from the top.

As another example, the mixture is optionally transferred to (or formed in) a container the bottom of which is made of a porous membrane. The pore size of the membrane is chosen to be smaller than the target cells or the substrates that the cells are bound to but large enough to allow for debris and other waste materials to pass through. To remove the waste, the air or liquid pressure is optionally adjusted such that the pressure is higher inside the container than outside, thus driving the buffer and waste out of the container while the membrane retains the target cells inside. The waste can also be removed, e.g.,, by filtering the buffer and waste through the membrane driven by the force of gravity or by centrifugal force.

As yet another example, the cells can be immobilized on the surface of a large substrate, for example, a slide or the bottom of a container, through cell fixing or affinity attachment utilizing surface proteins. The buffer and waste can be removed directly by either using a vacuum to decant from the top or by turning the container upside down. As yet another example, the cells are optionally immobilized on magnetic beads, e.g., by either chemical fixing or surface protein affinity attachment. The beads can then be immobilized on the container by attaching a magnetic field on the container. The buffer and waste can then be removed directly without the loss of cells the same way as described in the previous example. As yet another example, the cells are optionally immobilized on beads that are larger than or comparable in size to the target cells, e.g., by either chemical fixing or surface protein affinity attachment. The buffer and waste can then be removed through a porous membrane with pore size smaller than the beads. Alternatively, beads together with cells can be separated from buffer and waste by gravity or centrifugal force with the latter being removed from the top layer. As yet another example, the nonspecifically bound probes within cells are induced to migrate out of the cells by electrophoretic methods while the specifically bound probes remain.

As stated before, a washing cycle is completed by conducting each of the three steps above, and the washing procedure is accomplished by executing one or more (e.g., several) such washing cycles. Different washing buffers, detachment, or waste removal techniques may be used in different washing cycles.

### Detection

In the instant technology, the target cells that have signal-generating particles (labels) specifically hybridized to nucleic acid targets in them can be identified out of a large heterogeneous population after non-specifically bound probes and other wastes are removed through washing. Essentially any convenient method for the detection and identification can be employed.

In one embodiment, the suspension cells are immobilized onto a solid substrate after the labeling or washing step described above. The detection can be achieved using microscope based instruments. Specifically, in cases where the signal generated by the probes is chemiluminescent light, an imaging microscope with a CCD camera or a scanning microscope can be used to convert the light signal into digital information. In cases where the probe carries a label emitting a fluorescent signal, a fluorescent imaging or scanning microscope based instrument can be used for detection. In addition, since the target cells are, in general, rare among a large cell population, automatic event finding algorithms can be used to automatically identify and count the number of target cells in the population. Cells in suspension can be immobilized onto solid surfaces by any of a number of techniques. In one embodiment, a container with large flat bottom surface is used to hold the solution with the suspended cells. The container is then centrifuged to force the floating cells to settle on the bottom. If the surface is sufficiently large in comparison to the concentration of cells in the solution, cells are not likely to overlap on the bottom surface. In most cases, even if the cells overlap, the target cells will not because they are relatively rare in a large population. In another embodiment, suspended cells are cytospun onto a flat surface. After removal of fluids, the cells are immobilized on the surface by surface tension.

In certain embodiments of this invention, cells are floating (in suspension) or are immobilized on floating substrates, such as beads, so that pre-detection procedures, such as hybridization and washing, can be carried out efficiently in solution. There are several methods to detect rare target cells out of a large floating cell population. The preferred method is to use a detection system based on the concept of flow cytometry, where the floating cells or substrates are streamlined and pass in front of excitation and detection optics one by one. The target cells are identified through the optical signal emitted by the probes specifically bound to the nucleic acid targets in the cells. The optical signal can, e.g., be luminescent light or fluorescent light of a specific wavelength.

### Advantages

In summary, the instant QMAGEX technology has a number of unique elements that enable multiplex nucleic acid detection in single cells and detection of target cells. These elements include the following.

Nucleic acid molecules immobilized inside cells are used as markers for the identification of CTC (or other cell types). Compared with protein based markers, nucleic acids are more stable, widely available, and provide better signal to noise ratio in detection. In addition, the detection technique can be readily applied to a wide range of tumors or even other applications related to cell identification or classification. As another advantage, nucleic acid molecules are quantifiably measured at an individual cell level, instead of in a mixed cell population. This feature ensures that the cell as a key functional unit in the biological system is preserved for study. In many applications involving a mixed population of cells, this feature can be very useful in extracting real, useful information out of the assay. (For example, a CTC can be identified based on detection of the presence or expression level(s) of a set of nucleic acid marker(s) in the cell; the presence or copy number of additional nucleic acids in the cell can then provide additional information useful in diagnosis, predicting outcome, or the like.)

Cells optionally remain in suspension or in pellets that can be re-suspended in all steps of the assay before final detection. This feature significantly improves assay kinetics, simplifies the process, enhances the reproducibility, and keeps the cell in its most functional relevant status. On the other hand, significant aspects of the invention, including probe selection and design, multiplexing, amplification and labeling, can be applied directly to in situ hybridization technique for the detection and enumeration of rare cells in tissue samples.

A unique indirect capture probe design approach is optionally employed to achieve exceptional target hybridization specificity, which results in better signal to noise ratio in detection.

The assays enable the detection of multiple target genes or multiple parameters on the same gene simultaneously. This feature benefits the detection of rare cells such as CTC in a number of ways. First, it can reduce the false positive rate, which is essential in cancer diagnostics. Second, it can provide additional, clinically important information related to the detected tumor cell, which may include the progression stage and/or original type and source of the primary tumor.

The invented technology incorporates a signal amplification scheme, which boosts the detection sensitivity and enables the detection of rare cells among a large number of normal cells with high confidence.

Detection can be implemented on FACS or flow cytometer based instruments or on microscope based platforms. The former can be fully automated and provides fast detection and the additional benefit of sorting out identified cells for further study, if desired. The latter platform is more widely available and has the benefit of allowing final manual identification through morphology.

### SYSTEMS

In one aspect, the systems and apparatus are configured to carry out the procedures of the novel assays. The apparatus or system comprises one or more (and preferably all) of at least the following elements.

Fluid handling: The apparatus optionally includes a subsystem that can add reagents, and if required by the assay, decant fluids from the sample container (e.g., a removable or fixed, disposable or reusable container, for example a sample tube, multiwell plate, or the like). The subsystem can be based on a pipette style fluid transfer system where different fluids are handled by one pump head with disposable tips. As an alternative example, each reagent may have its own dedicated fluid channel.

Mixing and agitation: The apparatus optionally includes a device to mix different reagents in the sample solution and encourage any non-specifically bound material to detach from the cells. The device may have a mechanism to introduce a vortex or rocking motion to the holder of the sample container or to couple sound or ultrasound to the container. Alternatively, a magnetic stirrer can be put into the sample container and be driven by rotating magnetic field produced by an element installed in a holder for the container.

Temperature control: The temperature of the sample can be controlled to a level above the room temperature by installing a heater and a temperature probe to the chamber that holds the sample container. A peltier device can be used to control the temperature to a level above or below ambient. Temperature control is important, e.g., for performance of the hybridization and washing procedures in the assays.

Cell and waste fluid separation: The apparatus optionally includes a device that can remove waste fluid from the sample mixture while retaining cells for further analysis. The device may comprise a sample container that has a porous membrane as its bottom. The pore size of the membrane is smaller than the cells (or beads on which the cells are immobilized) but larger than the waste material in the mixed solution. The space below the membrane can be sealed and connected to a vacuum pump. As an alternative example, the space above the membrane can be sealed and connected to a positive pressure source. In a different embodiment, the device can comprise a centrifuge. The container with the membrane bottom is loaded into the centrifuge, which spins to force the waste solution to filter out through the membrane. In another configuration of this device, the sample container has a solid bottom. Cells deposit at the bottom after centrifugation, and the waste solution is decanted from the top by the fluid handling subsystem described above.

This device can also perform a function that prepares the sample for final readout. In embodiments where the readout is by microscopy, the cells are typically deposited and attached to a flat surface. A centrifuge in the device can achieve this if the bottom of the container is flat. In another approach, a flat plate can spin within its plane, and the system can employ the fluid handling device to drop the solution containing the cells at the center of the spin. The cells will be evenly spun on the plate surface.

Detection: The detection element of the invented apparatus can be integrated with the rest of the system, or alternatively it can be separate from the rest of the subsystems described above. (For example, for FFPE sections assay steps can be performed in an automated ISH station such as those commercially available from Ventana Medical Systems Inc. or Leica Microsystems, then detection can be performed on a separate microscope.) In one embodiment, the readout device is based on a microscope, which may be an imaging or scanning microscope. In another embodiment, the device is based on a fluorescent imaging or scanning microscope with multiple excitation and readout wavelengths for different probes. In a preferred embodiment when the cells are in suspension, the readout device is based on flow cytometry. The cytometry approach is preferred because it can read floating cells directly out of fluid at multiple wavelengths thus greatly improving the efficiency of the assay.

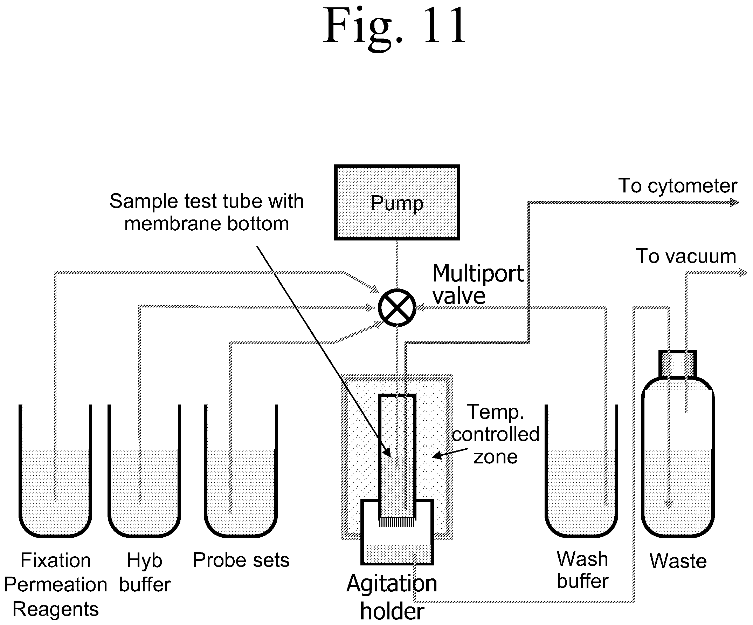

All of the above elements can be integrated into one instrument. Alternatively, these elements may be included in a number of instruments, which work together as a system to perform the assay. Figure 11 illustrates one particular exemplary embodiment of the instrument configuration. In this particular configuration, the sample is held in a container (sample test tube) with a membrane bottom. Reagents are added from the top of the tube using a pump through a multiport valve. Waste is removed from bottom by vacuum. The holder for the sample container is fixed on an agitation table and the space around the sample is temperature controlled (temp controlled zone) by the temperature controller. The fluid handling element can introduce reagents (fixation and permeation reagents, hybridization buffer, probes sets, and wash buffer) into the sample tube, remove waste into a waste container, and feed cells to a flow cytometer for detection.

Disclosed herein is a system comprising a holder configured to accept a sample container; a temperature controller configured to maintain the sample container at a selected temperature (e.g., a temperature selected by a user of the system or a preset temperature, different temperatures are optionally selected for different steps in an assay procedure); a fluid handling element fluidly connected to the sample container and configured to add fluid to and/or remove fluid from the sample container; a mixing element configured to mix (e.g., stir or agitate) contents of the sample container; and a detector for detecting one or more signals from within individual cells, wherein the detector is optionally fluidly connected to the sample container. One of more fluid reservoirs (e.g., for fixation or permeabilization reagents, wash buffer, probe sets, and/or waste) are optionally fluidly connected to the sample container.

A system optionally includes a computer. The computer can include appropriate software for receiving user instructions, either in the form of user input into a set of parameter fields, e.g., in a GUI, or in the form of preprogrammed instructions, e.g., preprogrammed for a variety of different specific operations. As just one example, the software can be preprogrammed for one or more operation such as sample handling, slide handling, de-paraffinization, de-crosslinking, hybridization, washing, etc. as described herein. The software optionally converts these instructions to appropriate language for controlling the operation of components of the system (e.g., for controlling a fluid handling element and/or laser). The computer can also receive data from other components of the system, e.g., from a detector, and can interpret the data, provide it to a user in a human readable format, or use that data to initiate further operations, in accordance with any programming by the user.

### NUCLEIC ACID TARGETS

As noted, a nucleic acid target can be essentially any nucleic acid that is desirably detected in a cell. Choice of targets will obviously depend on the desired application, e.g., expression analysis, disease diagnosis, staging, or prognosis, target identification or validation, pathway analysis, drug screening, drug efficacy studies, or any of many other applications. Large numbers of suitable targets have been described in the art, and many more can be identified using standard techniques.

For detection of CTC, as just one example, a variety of suitable nucleic acid targets are known. For example, a multiplex panel of markers for CTC detection could include one or more of the following markers: epithelial cell-specific (e.g. CK19, Muc1, EpCAM), blood cell-specific as negative selection (e.g. CD45), tumor origin-specific (e.g. PSA, PSMA, HPN for prostate cancer and mam, mamB, her-2 for breast cancer), proliferating potential-specific (e.g. Ki-67, CEA, CA15-3), apoptosis markers (e.g. BCL-2, BCL-XL), and other markers for metastatic, genetic and epigenetic changes. As another example, targets can include HOXB13 and IL17BR mRNAs, whose ratio in primary tumor has been shown to predict clinical outcome of breast cancer patients treated with tamoxifen (Ma et al. (2004) "A two-gene expression ratio predicts clinical outcome in breast cancer patients treated with tamoxifen" Cancer Cell 5(6):607-16 and Goetz et al. (2006) "A Two-Gene Expression Ratio of Homeobox 13 and Interleukin-17B Receptor for Prediction of Recurrence and Survival in Women Receiving Adjuvant Tamoxifen" Clin Cancer Res 12:2080-2087). See also, e.g., Gewanter, R. M., A. E. Katz, et al. (2003) "RT-PCR for PSA as a prognostic factor for patients with clinically localized prostate cancer treated with radiotherapy" Urology 61(5):967-71; Giatromanolaki et al. (2004) "Assessment of highly angiogenic and disseminated in the peripheral blood disease in breast cancer patients predicts for resistance to adjuvant chemotherapy and early relapse" Int J Cancer 108(4):620-7; Halabi et al. (2003) "Prognostic significance of reverse transcriptase polymerase chain reaction for prostate-specific antigen in metastatic prostate cancer: a nested study within CALGB 9583" J Clin Oncol 21(3):490-5; Hardingham et al. (2000) "Molecular detection of blood-borne epithelial cells in colorectal cancer patients and in patients with benign bowel disease" Int J Cancer 89(1):8-13; Hayes et al. (2002) "Monitoring expression of HER-2 on circulating epithelial cells in patients with advanced breast cancer" Int J Oncol 21(5):1111-7; Jotsuka, et al. (2004) "Persistent evidence of circulating tumor cells detected by means of RT-PCR for CEA mRNA predicts early relapse: a prospective study in node-negative breast cancer" Surgery 135(4):419-26; Allen-Mersh T et al. (2003) "Colorectal cancer recurrence is predicted by RT-PCR detection of circulating cancer cells at 24 hours after primary excision" ASCO meeting, Chicago, May 2003; Shariat et al. (2003) "Early postoperative peripheral blood reverse transcription PCR assay for prostate-specific antigen is associated with prostate cancer progression in patients undergoing radical prostatectomy" Cancer Res 63(18):5874-8; Smith et al. (2000) "Response of circulating tumor cells to systemic therapy in patients with metastatic breast cancer: comparison of quantitative polymerase chain reaction and immunocytochemical techniques" J Clin Oncol 18(7):1432-9; Stathopoulou et al. (2002) "Molecular detection of cytokeratin-19-positive cells in the peripheral blood of patients with operable breast cancer: evaluation of their prognostic significance" J Clin Oncol 20(16):3404-12; and Xenidis et al. (2003) "Peripheral blood circulating cytokeratin-19 mRNA-positive cells after the completion of adjuvant chemotherapy in patients with operable breast cancer" Ann Oncol 14(6):849-55.

One preferred class of nucleic acid targets to be detected in the methods herein are those involved in cancer. Any nucleic acid that is associated with cancer can be detected in the methods of the invention, e.g., those that encode over expressed or mutated polypeptide growth factors (e.g., sis), overexpressed or mutated growth factor receptors (e.g., erb-B1), over expressed or mutated signal transduction proteins such as G-proteins (e.g., Ras) or non-receptor tyrosine kinases (e.g., abl), over expressed or mutated regulatory proteins (e.g., myc, myb, jun, fos, etc.) and/or the like. In general, cancer can often be linked to signal transduction molecules and corresponding oncogene products, e.g., nucleic acids encoding Mos, Ras, Raf, and Met; and transcriptional activators and suppressors, e.g., p53, Tat, Fos, Myc, Jun, Myb, Rel, and/or nuclear receptors. p53, colloquially referred to as the "molecular policeman" of the cell, is of particular relevance, as about 50% of all known cancers can be traced to one or more genetic lesion in p53. Additional exemplary markers useful for detection of breast cancer cells include, but are not limited to, uPA (urokinase-type plasminogen activator), PAI-1 (plasminogen activator inhibitor-1), PAI-2, and/or uPAR (urokinase-type plasminogen activator receptor). Other additional exemplary markers include, but are not limited to, CK18, CK20, C-met, EGFR, and ERCC1 (a marker for resistance to cisplatin; patients with completely resected NSCLC and ERCC1-negative tumors are helped by cisplatin-based chemotherapy, while in contrast, patients with ERCC1-positive tumors may endure the toxicities of therapy with little benefit).

Taking one class of genes that are relevant to cancer as an example for discussion, many nuclear hormone receptors have been described in detail and the mechanisms by which these receptors can be modified to confer oncogenic activity have been worked out. For example, the physiological and molecular basis of thyroid hormone action is reviewed in Yen (2001) "Physiological and Molecular Basis of Thyroid Hormone Action" Physiological Reviews 81(3):1097-1142, and the references cited therein. Known and well characterized nuclear receptors include those for glucocorticoids (GRs), androgens (ARs), mineralocorticoids (MRs), progestins (PRs), estrogens (ERs), thyroid hormones (TRs), vitamin D (VDRs), retinoids (RARs and RXRs), and the peroxisome proliferator activated receptors (PPARs) that bind eicosanoids. The so called "orphan nuclear receptors" are also part of the nuclear receptor superfamily, and are structurally homologous to classic nuclear receptors, such as steroid and thyroid receptors. Nucleic acids that encode any of these receptors, or oncogenic forms thereof, can be detected in the methods of the invention. About 40% of all pharmaceutical treatments currently available are agonists or antagonists of nuclear receptors and/or oncogenic forms thereof, underscoring the relative importance of these receptors (and their coding nucleic acids) as targets for analysis by the methods of the invention.

One exemplary class of target nucleic acids are those that are diagnostic of colon cancer, e.g., in samples derived from stool. Colon cancer is a common disease that can be sporadic or inherited. The molecular basis of various patterns of colon cancer is known in some detail. In general, germline mutations are the basis of inherited colon cancer syndromes, while an accumulation of somatic mutations is the basis of sporadic colon cancer. In Ashkenazi Jews, a mutation that was previously thought to be a polymorphism may cause familial colon cancer. Mutations of at least three different classes of genes have been described in colon cancer etiology: oncogenes, suppressor genes, and mismatch repair genes. One example nucleic acid encodes DCC (deleted in colon cancer), a cell adhesion molecule with homology to fibronectin. An additional form of colon cancer is an autosomal dominant gene, hMSH2, that comprises a lesion. Familial adenomatous polyposis is another form of colon cancer with a lesion in the MCC locus on chromosome number 5. For additional details on colon cancer, see, Calvert et al. (2002) "The Genetics of Colorectal Cancer" Annals of Internal Medicine 137 (7):603-612 and the references cited therein. For a variety of colon cancers and colon cancer markers that can be detected in stool, see, e.g., Boland (2002) "Advances in Colorectal Cancer Screening: Molecular Basis for Stool-Based DNA Tests for Colorectal Cancer: A Primer for Clinicans" Reviews In Gastroenterological Disorders Volume 2, Supp. 1 and the references cited therein. As with other cancers, mutations in a variety of other genes that correlate with cancer, such as Ras and p53, are useful diagnostic indicators for cancer.

Cervical cancer is another exemplary target for detection, e.g., by detection of nucleic acids that are diagnostic of such cancer in samples obtained from vaginal secretions. Cervical cancer can be caused by the papova virus (e.g., human papilloma virus) and has two oncogenes, E6 and E7. E6 binds to and removes p53 and E7 binds to and removes PRB. The loss of p53 and uncontrolled action of E2F/DP growth factors without the regulation of pRB is one mechanism that leads to cervical cancer. E6 and/or E7 (e.g., from specific HPV strains, particularly high risk strains such as HPV16 and HPV18) can thus be used as markers for detection of cervical cancer. Other useful markers include, but are not limited to, factors involved in cell cycle control and/or DNA replication that are aberrantly expressed in cervical cancer such as p16INK4a, topoisomerase II alpha (TOP IIA), and mini-chromosome maintenance 2 (Mdm2).

Another exemplary target for detection by the methods of the invention is retinoblastoma, e.g., in samples derived from tears. Retinoblastoma is a tumor of the eyes which results from inactivation of the pRB gene. It has been found to transmit heritably when a parent has a mutated pRB gene (and, of course, somatic mutation can cause non-heritable forms of the cancer).

Neurofibromatosis Type 1 can be detected in the methods of the invention. The NF1 gene is inactivated, which activates the GTPase activity of the ras oncogene. If NF1 is missing, ras is overactive and causes neural tumors. The methods of the invention can be used to detect Neurofibromatosis Type 1 in CSF or via tissue sampling.

Many other forms of cancer are known and can be found by detecting associated genetic lesions using the methods of the invention. Cancers that can be detected by detecting appropriate lesions include cancers of the lymph, blood, stomach, gut, colon, testicles, pancreas, bladder, cervix, uterus, skin, and essentially all others for which a known genetic lesion exists. For a review of the topic, see, e.g., The Molecular Basis of Human Cancer Coleman and Tsongalis (Eds) Humana Press; ISBN: 0896036340; 1st edition (August 2001).

Similarly, nucleic acids from pathogenic or infectious organisms can be detected by the methods of the invention, e.g., for infectious fungi, e.g., Aspergillus, or Candida species; bacteria, particularly E. coli, which serves a model for pathogenic bacteria (and, of course certain strains of which are pathogenic), as well as medically important bacteria such as Staphylococci (e.g., aureus), or Streptococci (e.g., pneumoniae); protozoa such as sporozoa (e.g., Plasmodia), rhizopods (e.g., Entamoeba) and flagellates (Trypanosoma, Leishmania, Trichomonas, Giardia, etc.); viruses such as (+) RNA viruses (examples include Poxviruses e.g., vaccinia; Picornaviruses, e.g. polio; Togaviruses, e.g., rubella; Flaviviruses, e.g., HCV; and Coronaviruses), (-) RNA viruses (e.g., Rhabdoviruses, e.g., VSV; Paramyxovimses, e.g., RSV; Orthomyxovimses, e.g., influenza; Bunyaviruses; and Arenaviruses), dsDNA viruses (Reoviruses, for example), RNA to DNA viruses, i.e., Retroviruses, e.g., HIV and HTLV, and certain DNA to RNA viruses such as Hepatitis B.

As noted previously, gene amplification or deletion events can be detected at a chromosomal level using the methods of the invention, as can altered or abnormal expression levels. One preferred class of nucleic acid targets to be detected in the methods herein include oncogenes or tumor suppressor genes subject to such amplification or deletion. Exemplary nucleic acid targets include, but are not limited to, integrin (e.g., deletion), receptor tyrosine kinases (RTKs; e.g., amplification, point mutation, translocation, or increased expression), NF1 (e.g., deletion or point mutation), Akt (e.g., amplification, point mutation, or increased expression), PTEN (e.g., deletion or point mutation), EGFR (amplification), c-met (amplification), MDM2 (e.g., amplification), SOX (e.g., amplification), RAR (e.g., amplification), CDK2 (e.g., amplification or increased expression), Cyclin D (e.g., amplification or translocation), Cyclin E (e.g., amplification), Aurora A (e.g., amplification or increased expression), P53 (e.g., deletion or point mutation), NBS1 (e.g., deletion or point mutation), Gli (e.g., amplification or translocation), Myc (e.g., amplification or point mutation), HPV-E7 (e.g., viral infection), and HPV-E6 (e.g., viral infection).

For embodiments in which a nucleic acid target is used as a reference, suitable reference nucleic acids have similarly been described in the art or can be determined. For example, a variety of genes whose copy number is stably maintained in various tumor cells is known in the art. Housekeeping genes whose transcripts can serve as references in gene expression analyses include, for example, 18S rRNA, 28S rRNA, GAPD, ACTB, and PPIB. Additional similar nucleic acids have been described in the art and can be adapted to the practice of the present invention.

### LABELS

A wide variety of labels are well known in the art and can be adapted to the practice of the present invention. For example, luminescent labels and light-scattering labels (e.g., colloidal gold particles) have been described. See, e.g., Csaki et al. (2002) "Gold nanoparticles as novel label for DNA diagnostics" Expert Rev Mol Diagn 2:187-93.

As another example, a number of fluorescent labels are well known in the art, including but not limited to, hydrophobic fluorophores (e.g., phycoerythrin, rhodamine, Alexa Fluor 488 and fluorescein), green fluorescent protein (GFP) and variants thereof (e.g., cyan fluorescent protein and yellow fluorescent protein), and quantum dots. See e.g., The Handbook: A Guide to Fluorescent Probes and Labeling Technologies, Tenth Edition or Web Edition (2006) from Invitrogen (available on the world wide web at probes (dot) invitrogen (dot) com/handbook), for descriptions of fluorophores emitting at various different wavelengths (including tandem conjugates of fluorophores that can facilitate simultaneous excitation and detection of multiple labeled species). For use of quantum dots as labels for biomolecules, see e.g., Dubertret et al. (2002) Science 298:1759; Nature Biotechnology (2003) 21:41-46; and Nature Biotechnology (2003) 21:47-51.

Labels can be introduced to molecules, e.g. polynucleotides, during synthesis or by postsynthetic reactions by techniques established in the art. For example, kits for fluorescently labeling polynucleotides with various fluorophores are available from Molecular Probes, Inc. (www (dot) molecularprobes (dot) com), and fluorophore-containing phosphoramidites for use in nucleic acid synthesis are commercially available. Similarly, signals from the labels (e.g., absorption by and/or fluorescent emission from a fluorescent label) can be detected by essentially any method known in the art. For example, multicolor detection and the like are well known in the art. Instruments for detection of labels are likewise well known and widely available, e.g., scanners, microscopes, flow cytometers, etc. For example, flow cytometers are widely available, e.g., from Becton-Dickinson (www (dot) bd (dot) com) and Beckman Coulter (www (dot) beckman (dot) com).

### MOLECULAR BIOLOGICAL TECHNIQUES

In practicing the present invention, many conventional techniques in molecular biology, microbiology, and recombinant DNA technology are optionally used. These techniques are well known and are explained in, for example, Berger and Kimmel, Guide to Molecular Cloning Techniques, Methods in Enzymology volume 152 Academic Press, Inc., San Diego, CA; Sambrook et al., Molecular Cloning - A Laboratory Manual (3rd Ed.), Vol. 1-3, Cold Spring Harbor Laboratory, Cold Spring Harbor, New York, 2000 and Current Protocols in Molecular Biology, F.M. Ausubel et al., eds., Current Protocols, a joint venture between Greene Publishing Associates, Inc. and John Wiley & Sons, Inc., (supplemented through 2008). Other useful references, e.g. for cell isolation and culture (e.g., for subsequent nucleic acid isolation) include Freshney (1994) Culture of Animal Cells, a Manual of Basic Technique, third edition, Wiley-Liss, New York and the references cited therein; Payne et al. (1992) Plant Cell and Tissue Culture in Liquid Systems John Wiley & Sons, Inc. New York, NY; Gamborg and Phillips (Eds.) (1995) Plant Cell, Tissue and Organ Culture; Fundamental Methods Springer Lab Manual, Springer-Verlag (Berlin Heidelberg New York) and Atlas and Parks (Eds.) The Handbook of Microbiological Media (1993) CRC Press, Boca Raton, FL.

### Making Polynucleotides

Methods of making nucleic acids (e.g., by in vitro amplification, purification from cells, or chemical synthesis), methods for manipulating nucleic acids (e.g., by restriction enzyme digestion, ligation, etc.) and various vectors, cell lines and the like useful in manipulating and making nucleic acids are described in the above references. In addition, methods of making branched polynucleotides (e.g., amplification multimers) are described in USPN 5,635,352, USPN 5,124,246, USPN 5,710,264, and USPN 5,849,481, as well as in other references mentioned above.

In addition, essentially any polynucleotide (including, e.g., labeled or biotinylated polynucleotides) can be custom or standard ordered from any of a variety of commercial sources, such as The Midland Certified Reagent Company (www (dot) mcrc (dot) com), The Great American Gene Company (www (dot) genco (dot) com), ExpressGen Inc. (www (dot) expressgen (dot) com), Qiagen (oligos (dot) qiagen (dot) com) and many others.

A label, biotin, or other moiety can optionally be introduced to a polynucleotide, either during or after synthesis. For example, a biotin phosphoramidite can be incorporated during chemical synthesis of a polynucleotide. Alternatively, any nucleic acid can be biotinylated using techniques known in the art; suitable reagents are commercially available, e.g., from Pierce Biotechnology (www (dot) piercenet (dot) com). Similarly, any nucleic acid can be fluorescently labeled, for example, by using commercially available kits such as those from Molecular Probes, Inc. (www (dot) molecularprobes (dot) com) or Pierce Biotechnology (www (dot) piercenet (dot) com) or by incorporating a fluorescently labeled phosphoramidite during chemical synthesis of a polynucleotide.

### REFERENCES:

Hess CJ, et al. Gene expression profiling of minimal residual disease in acute myeloid leukaemia by novel multiplex-PCR-based method. Leukemia. 2004 Dec;18(12):1981-8.
Vogel I et al. Detection and prognostic impact of disseminated tumor cells in pancreatic carcinoma. Pancreatology. 2002;2(2):79-88.
Gilbey AM et al. The detection of circulating breast cancer cells in blood. J Clin Pathol. 2004 Sep;57(9):903-11.
Molnar B et al. Molecular detection of circulating cancer cells. Role in diagnosis, prognosis and follow-up of colon cancer patients. Dig Dis. 2003;21(4):320-5.
Vlems FA et al. Detection and clinical relevance of tumor cells in blood and bone marrow of patients with colorectal cancer. Anticancer Res. 2003 Jan-Feb;23(1B):523-30.
Ma PC et al Circulating tumor cells and serum tumor biomarkers in small cell lung cancer. Anticancer Res. 2003 Jan-Feb;23(1A):49-62.
Mocellin S et al (2004) Molecular detection of circulating tumor cells in an independent prognostic factor in patients with high-risk cutaneous melanoma. Int J Cancer 111:741-745
Cristofanilli M. et al., (2004) Circulating tumor cells, disease progression, and survival in metastatic breast cancer. N Engl J Med. 2004 Aug 19;351(8):781-91.
Ito S et al., (2002) Quantitative detection of CEA expressing free tumor cells in the peripheral blood of colorectal cancer patients during surgery with the real-time RT-PCR on a Light Cycler, Cancer Letters, 183:195-203.
Hicks DG et al., In situ hybridization in the pathology laboratory: General principles, automation, and emerging research applications for tissue-based studies of gene expression. J Mol Histol. 2004 Aug;35(6):595-601.
Herzenberg LA et al. The history and future of the fluorescence activated cell sorter and flow cytometry: a view from Stanford. Clin Chem. 2002 Oct;48(10):1819-27.
Timm EA Jr et al. Amplification and detection of a Y-chromosome DNA sequence by fluorescence in situ polymerase chain reaction and flow cytometry using cells in suspension. Cytometry. 1995 Sep 15;22(3):250-5.
Bauman JG, Bentvelzen P. Flow cytometric detection of ribosomal RNA in suspended cells by fluorescent in situ hybridization. Cytometry. 1988 Nov;9(6):517-24.
Timm EA Jr, Stewart CC. Fluorescent in situ hybridization en suspension (FISHES) using digoxigenin-labeled probes and flow cytometry. Biotechniques. 1992 Mar;12(3):362-7.
Bains MA Flow cytometric quantitation of sequence-specific mRNA in hemopoietic cell suspensions by primer-induced in situ (PRINS) fluorescent nucleotide labeling. Exp Cell Res. 1993 Sep;208(1):321-6.
Patterson BK Detection of HIV-1 DNA and messenger RNA in individual cells by PCR-driven in situ hybridization and flow cytometry. Science. 1993 May 14;260(5110):976-9.
Rufer N Telomere length dynamics in human lymphocyte subpopulations measured by flow cytometry. Nat Biotechnol. 1998 Aug;16(8):743-7.
Hultdin M Telomere analysis by fluorescence in situ hybridization and flow cytometry. Nucleic Acids Res. 1998 Aug 15;26(16):3651-6.
Fava TA, et al Ectopic expression of guanylyl cyclase C in CD34+ progenitor cells in peripheral blood. J Clin Oncol. 2001 Oct 1;19(19):3951-9.
Kosman D, Mizutani CM, Lemons D, Cox WG, McGinnis W, Bier E. Multiplex detection of RNA expression in Drosophila embryos. Science. 2004 Aug 6;305(5685):846.
Player AN, Shen LP, Kenny D, Antao VP, Kolberg JA. Single-copy gene detection using branched DNA (bDNA) in situ hybridization. J Histochem Cytochem. 2001 May;49(5):603-12.
Schrock E, du Manoir S, Veldman T, Schoell B, Wienberg J, Ferguson-Smith MA, Ning Y, Ledbetter DH, Bar-Am I, Soenksen D, Garini Y, Ried T. Multicolor spectral karyotyping of human chromosomes. Science. 1996 Jul 26;273(5274):494-7.
Larsson C, Koch J, Nygren A, Janssen G, Raap AK, Landegren U, Nilsson M. In situ genotyping individual DNA molecules by target-primed rolling-circle amplification of padlock probes. Nat Methods. 2004 Dec;1(3):227-32. Epub 2004 Nov 18.
Zhang, L., Zhou, W., Velculescu, V.E., Kern, S.E., Hruban, R.H., Hamilton, S.R., Vogelstein, B., and Kinzler, K.W. (1997). Gene expression profiles in normal and cancer cells. Science (New York, NY 276, 1268-1272.
Pinkel, D., Straume, T., Gray, J.W. Cytogenetic analysis using quantitative, high-sensitivity, fluorescence hybridization. Proc. Natl. Acad. Sci. USA 1986 83:2934-2938.

### EXAMPLES

### EXAMPLE 1: DETECTION OF NUCLEIC ACIDS IN INDIVIDUAL CELLS

The following sets forth a series of experiments that demonstrate in-cell detection of nucleic acid. The results demonstrate, for example, that when staining cells on a glass substrate with QMAGEX, we can obtain a highly specific signal with a sensitivity of detecting a single mRNA molecule. Moreover, we can achieve staining of multiple mRNAs at the same time using a combination of different target probes and amplifiers. These results further demonstrate the feasibility of detecting cancer cells exhibiting transcriptional upregulation within a population of cells with normal gene expression. The results also demonstrate staining of cells in suspension and identification of them using flow cytometry, eliminating need for a solid support for the cells and allowing for rapid detection of stained cells. These results further demonstrate the ability to detect cells exhibiting transcriptional upregulation from those with low basal levels of mRNA expression in a rapid manner using flow cytometry.

### Overview of assay

We have developed an assay for detecting multiple RNA transcripts in situ in individual cells over a large cell population that we have named QMAGEX. The assay can be performed, e.g., on cells attached to a glass substrate and examined using a fluorescent microscope or on cells in suspension and analyzed using a flow cytometer. This assay is analogous in some respects to traditional RNA ISH/FISH but possesses the following unique features: 1) it has the sensitivity to detect a single mRNA transcript; 2) it is easy to conduct multiplex in situ for simultaneous detection of markers that can be correlated with cell morphology; 3) it can provide an internal control staining of a housekeeping gene through its multiplex capability to determine RNA integrity and assay quality (important for regulatory approval); and 4) the signals from QMAGEX are semi-quantitative and/or quantitative.

The basic assay procedure (**Figure 1**) can be done within a day and generally includes the following steps. After being fixed and permeablized, cells either on substrate or in suspension are hybridized to the following series of oligonucleotide probes. First, a set of capture probes is hybridized to the target RNA inside the cells. Next, preamplifier molecules (PreAMP) are hybridized to the capture probes, providing a bridge for the hybridization of amplifier molecules (AMP). Finally, amplification of the signal is accomplished by the binding of, e.g., up to 20 AMPs to each PreAMP, and 20 label probes (LPs) to each AMP, giving a total of 400 fluorescent labels or alkaline phosphatase (AP) labels to each target probe. (It is worth noting that signal intensity can be enhanced further by including more than one label in each LP; as just one example, by conjugating up to three fluorescent molecules per LP instead of one fluorescent molecule per LP.) In the case when AP-conjugated LPs are used in combination with Fast Red substrate, signal amplification is enhanced further due to deposition of red fluorescent precipitate in the vicinity of the target nucleic acid. Signals are detected, e.g., with either a regular fluorescent microscope with appropriate filters or with a multicolor flow cytometer.

Nonspecific hybridization can be prevented or minimized through the "cooperative hybridization" concept (for additional details, see Flagella et al. (2006) "A multiplex branched DNA assay for parallel quantitative gene expression profiling" Anal Biochem. 352(1):50-60 and U.S. patent application publication 2007/0015188 entitled "Multiplex detection of nucleic acids" by Luo et al.). Nonspecific hybridization can be prevented or minimized, for example, by designing probe sets targeting a specific mRNA sequence using a double "Z" probe design. Target double "Z" probes are prescreened against the GenBank database to ensure minimal cross-hybridization with unintended nucleic acid sequences. In the double "Z" design, two neighboring probes each contain a target-hybridizing sequence, e.g., 20 to 30 base in length with a Tm significantly above the assay temperature, and a PreAMP-hybridizing sequence, e.g., only 14 bases in length with a Tm well below the assay temperature (**Figure 9** **Panels C-D**). As a result, a single capture probe is able to bind to target RNA strongly and stably during hybridization, but will bind to the PreAMP weakly and unstably due to the 14 base pair region of homology having a Tm well below the assay temperature. However, when two capture probes are present in neighboring positions, the combined hybridization strength, e.g., of 28 complementary base pairs, holds the PreAMP strongly and stably at the assay temperature, enabling signal amplification to occur. Such a double "Z" design ensures high detection specificity and simplifies probe design for simultaneous detection of multiple targets.

Two signal amplifiers have been tested in the assay, one with 400-fold (400X AMP1) amplification and another with 16-fold (16X AMP2) amplification. The 400X AMP1 is composed of 20 AMP binding site per PreAMP and 20 AP or fluorescent conjugated-LP binding sites per AMP molecule to provide 400 labeling molecule per capture probe pair (20x20=400). The 16X AMP2 is composed of 4 AMP binding sites per PreAMP and 4 AP or fluorescent conjugated-LP binding sites per AMP to give rise to 16 labeling molecules per capture probe pair (4X4=16). The two amplifying systems have been shown experimentally to have no cross reactivity to each other.

### In cell detection of 18S RNA

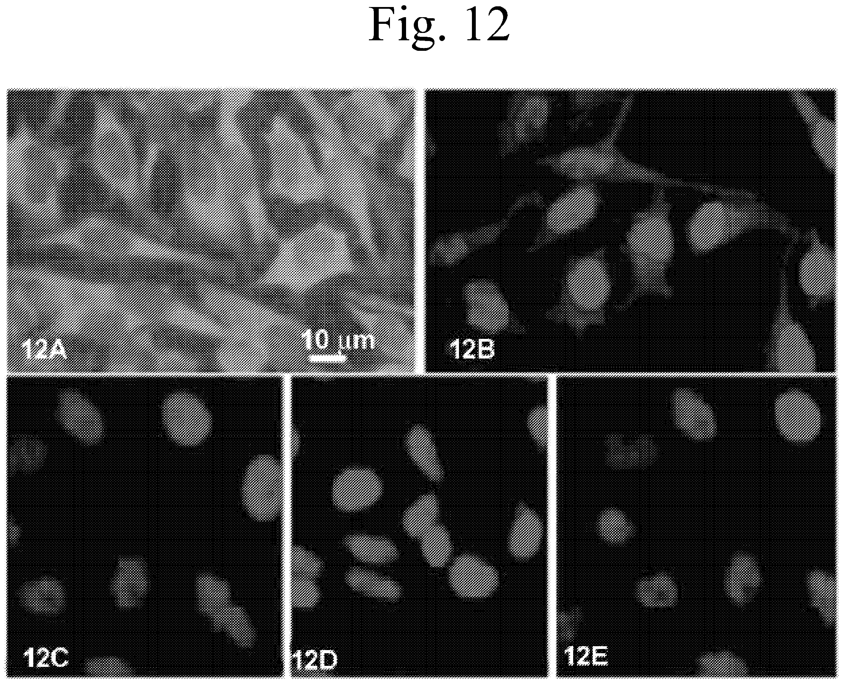

In an initial experiment, 18S capture probes (capture probes complementary to 18S RNA) in combination with 16X AMP2 were used on HeLa cells grown on coverslips. The goal of this initial effort was to identify an assay condition that produces maximal signal-to-background ratio. As will be discussed below, we have achieved a signal-to-background ratio sufficient for single copy mRNA detection. To understand the magnitude of signal enhancement by the amplifiers, we conducted parallel experiments in which the same set of 18S capture probes were used to probe 18S RNA in HeLa cells. One set of capture probes was amplified by 16X AMP2/Alexa 488-LP while the other set was probed with an amplifier designed to have only one PreAMP/AMP and one Alexa 488-LP binding site (1X AMP3). By setting the camera exposure time constant, we captured the 18S signal in cells labeled with 16X AMP2 (**Figure 12** **Panel A**) and 1X AMP3 (**Figure 12** **Panel B**). We reproducibly saw a higher 18S signal in cells labeled with 16X AMP2 than with 1X AMP1, suggesting that signal amplification is necessary to gain a greater signal-to-background ratio. To confirm the specificity of the capture probe design, we used a probe set targeting the anti-sense strand of the 18S intron sequence, and it showed a low to absent background signal (**Figure 12** **Panel C**). We have also found that the 18S signal is completely removed when the cells are pre-treated with RNase or when the cells are incubated with either no capture probe set or with only the tail sequence complementary to the PreAMP (data not shown). These results thus indicate that the fluorescent signal we observed is specific in labeling 18S RNA. The double "Z" capture probe design used in QMAGEX greatly improves the assay specificity. In experiments in which one half or the other of the double "Z" probe set was used, signal is greatly reduced as compared to that when the full probe set is used (**Figure 12** **Panels D and E vs. Panel A**). Based on the above results, we conclude that QMAGEX performs to our intended design principle and the assay is the first of its kind in simultaneous signal amplification (PreAMP/AMP) and background reduction (double Z design) to achieve high signal and great specificity.

### Duplex QMAGEX assay

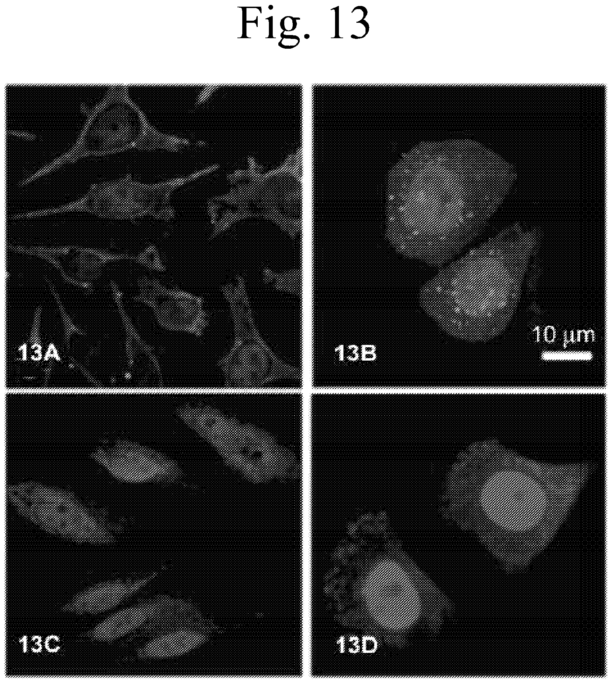

To explore its potential for in situ detection of low copy RNA transcripts and its capability for multiplex detection, we developed a multiplex QMAGEX assay using 18S and Her-2 as the model genes. HeLa and SKBR3 are labeled with DAPI to facilitate the identification of nuclei (blue). Her-2 mRNA was labeled with the 400X AMP1/Alexa 488-LP (green) while 18S RNA was labeled with the 16X AMP2/Alexa 555-LP (red). High 18S expression in HeLa (**Figure 13** **Panels A and C**) and SKBR3 (**Figure 13** **Panels B and D**) resulted in a ubiquitous staining pattern around the entire cells. When labeling Her-2 mRNA (green), signals appeared to be punctate fluorescent dots with SKBR3 cells showing a higher number of dots per cell (**Figure 13** **Panel B**) than HeLa (**Figure 13** **Panel A**), consistent with the fact that SKBR3 is a breast cancer cell line with HER2 gene amplification whereas HeLa has no HER2 amplification. Since a control probe set targeting the anti-sense strand of the Her-2 intron sequence gave rise to no green fluorescent dots in any cells (**Figure 13** **Panels C and D**), we concluded that the capture probes designed for Her-2 mRNA are specific in detecting Her-2 mRNA transcripts. We also noticed the variation of RNA dots in individual HeLa cells. Considering the relative same level of 18S (a housekeeping gene) staining in all HeLa cells, we believe that the variation in dot number seen in HeLa is likely to be an intrinsic property of gene expression, rather than assay variability, and is consistent with previous observations on stochastic expression of mRNA transcripts (e.g. reviewed by Shav-Tal et al. (2004) "Imaging gene expression in single living cells" Nat Rev Mol Cell Biol. 5(10):855-61). Thus we have demonstrated using a Her-2/18S duplex that the QMAGEX assay can be used to detect two RNA transcripts simultaneously and the relative signals can be used to compare gene expression.

### Single copy mRNA detection

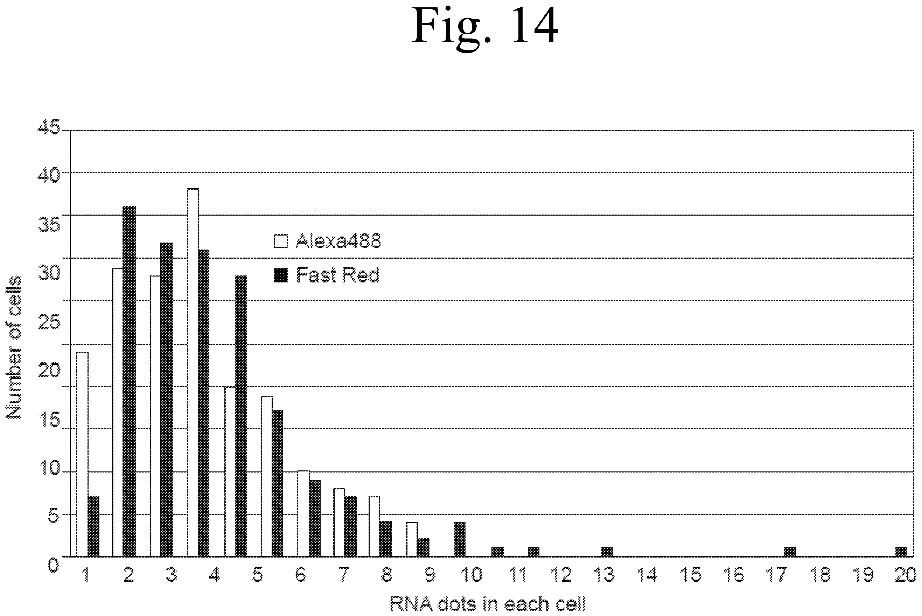

The punctate expression pattern of Her-2 in HeLa and SKBR3 cells detected using QMAGEX suggests that each fluorescent dot is one mRNA; however, we can not exclude the possibility that each puncta represents two or more mRNAs in close proximity to one another. We designed two experiments in order to distinguish between these two possibilities. The first experiment utilized QuantiGene 2.0, an established quantitative assay, to compare the average copy number of transcripts per cell to the number of fluorescent dots seen in QMAGEX. We labeled Her-2 mRNA in HeLa cells with capture probes designed for the Her-2 gene followed by 400XAMP1/Alexa488-LP or 400XAMP1/AP-LP and Fast Red substrate reaction to ensure sensitive and reproducible detection of all RNA dots. In both assays, 200 cells were randomly selected. The number of fluorescent dots in each cell was counted and the average dots per cell were calculated. The histogram of fluorescent dots per cell by both labeling schemes (**Figure 14**) showed a similar stochastic distribution with a median value at 3 copies per cell and an average value of 3.2-3.4 copies per cell. The similar number of dots seen using both fluorescence and Fast Red indicated that the extra signal amplification created by the Fast Red substrate is not necessary to elucidate all of the RNAs present in the cells. Using the QuantiGene 2.0 assay, the same batch of HeLa cells were tested and showed an average of ∼5 Her-2 mRNA transcripts per cell, which is close to our results using the QMAGEX assay (**Table 1**). To further confirm these results, we designed a second experiment in which we measured the fluorescent intensity of each dot for Her-2 mRNA, and compared them with the fluorescent intensity of each dot in HER2 genomic DNA. In this experiment, RNA and DNA QMAGEX assays were run in parallel on the same batch of HeLa cells using the same capture probes. With a constant camera exposure time, pictures were taken from both DNA and RNA QMAGEX assays. The CellProfiler program (www (dot) cellprofiler (dot) org) was utilized to measure fluorescent intensity of each dot. Since we used the same probe set for both RNA and DNA FISH, a similar distribution of fluorescent intensity would be expected if RNA was being measured at a single copy resolution. This is because each fluorescent dot in DNA FISH represents a single gene copy. In our analysis of fluorescent intensity distribution (data not shown), the range of fluorescent intensity from the RNA dots does not exceed the fluorescent intensity from each DNA dot, confirming that each RNA dot is indeed representative of a single copy mRNA. In situ detection of single copy mRNA by routine fluorescent microscopy is a major achievement because this has not been done before. Traditional ISH/FISH assays only have a detection sensitivity around 50 copies per cell, which excludes 95% of the genes which are expressed at a level that is less than 50 transcripts per cell (Zhang et al. (1997) "Gene expression profiles in normal and cancer cells" Science 276(5316):1268-72).

**Table 1. Average mRNA copies/cell determined by QG2.0.**

| Genes | HeLa | | SKBR3 |
|---|---|---|---|
| | Control | Induced | |
| Her-2 | ∼5 | NA | ∼100 |
| IL-6 | ∼2 | ∼5 | NA |
| IL-8 | ∼1 | ∼275 | NA |

### Determination of gene expression changes in single cells

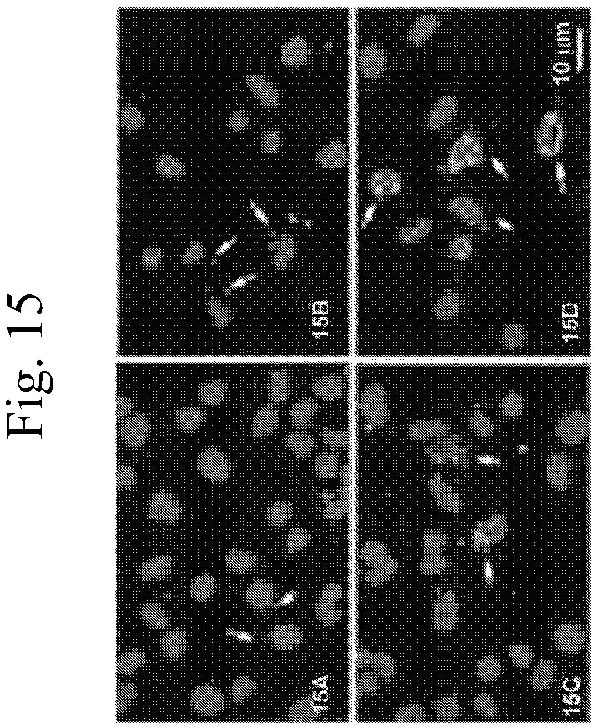

The induction of cytokine gene expression in HeLa cells upon PMA-treatment is a classic model for validation of expression profiling technologies. It has been shown that IL-6 and IL-8 mRNA are expressed at very low levels in resting HeLa cells, but they are induced significantly upon PMA treatment (e.g. Zhang et al. (2005) "Small interfering RNA and gene expression analysis using a multiplex branched DNA assay without RNA purification" J Biomol Screen. 10(6):549-56). Using QuantiGene 2.0, we have determined that, on average, there are only about 1 to 2 copies of IL-8 and IL-6 mRNA per cell in resting HeLa cells and upon PMA induction IL-8 and IL-6 increase to -275 copies and -5 copies per cell, respectively (**Table 1**). Since existing technologies (e.g. microarray, qRT-PCR, QuantiGene 2.0) measure gene expression in purified RNA or cell lysates, the measurement represents an average response of groups of cells in the sample. In contrast, QMAGEX offers a unique opportunity to determine mRNA expression in single cells in response to PMA treatment. Using 400X AMP1 in combination with Alexa 488-label probe, we have determined expression for IL-6 and IL-8 mRNA in resting (**Figure 15** **Panels A and B**) and PMA-treated (**Figure 15** **Panels C and D**) HeLa cells at the single cell level. While very low levels of IL-6 and IL-8 mRNA expression are observed in resting HeLa cells, significant induction of IL-6 and extremely high level of induction of IL-8 are observed in some, but not all of the PMA-treated HeLa cells. Thus, while IL-6 and IL-8 expression measured in single cell by QMAGEX assay are consistent with the average expression response obtained by QuantiGene 2.0, there is a dramatic variation in single cell response as some cells show extremely high levels of induction while other cells remain unchanged (**Figure 15** **Panels C and D**). The dramatic variation in single cell expression profile underscores the heterogeneity in individual cell's response to PMA treatment, even with a supposed homogenous cell line. To our knowledge this is the first study to look at the induction response of native gene expression at the single cell level. The observed heterogeneous expression response underlines the value of studying single cell biology for which QMAGEX can be a valuable tool.

### Detection of cancer cells in mixed cell populations

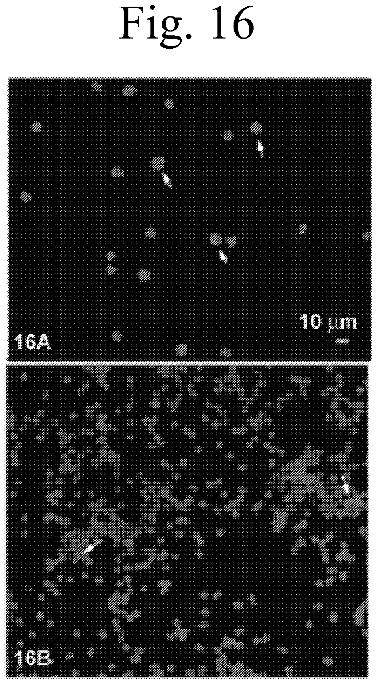

In order to determine the feasibility of QMAGEX in CTC detection, we mixed breast cancer cells into Jurkat cells (T cell origin) or WBCs, and evaluated the capability of QMAGEX to distinguish breast cancer cells from Jurkat cells or WBCs. For example, we mixed SKBR3 cells with Jurkat cells at 1:50 ratio, cultured them for a day, and detected the mRNA expression of the common cancer cell marker CK19 in the mixed cells by QMAGEX. Using capture probes targeting CK19 in combination with 400X AMP1/AP-LP and Fast Red substrate, SKBR3 cells were identified by their high expression of CK19 among CK19 negative Jurkat cells (**Figure 16** **Panel A**). We have also spiked BT474 breast cancer cells into Ficoll-purified blood cells at a 1:1,000 ratio, cytospun the cells onto a slide, and performed QMAGEX with capture probes targeting CK19 in combination with 400X AMP1/AP-LP and Fast Red substrate. Similar to the Jurkat/SKBR3 mix cells, 1 per 1000 cell was labeled with CK19 (**Figure 16** **Panel B**), suggesting that the QMAGEX assay could be used to discriminate cells based on differential gene expression level. In addition to CK19, we also showed that QMAGEX with Her-2 capture probe is as effective in identifying SKBR3 cells among HeLa, Jurkat and WBCs (data not shown). These results thus prove the feasibility of using the QMAGEX assay for CTC detection in patient blood samples.

### Flow cytometry based QMAGEX assay (FC-QMAGEX)

Currently, CTC detection in patient blood samples requires a CTC enrichment step (e.g. immunomagnetic separation) followed by staining and scanning a large population of cells on a glass substrate for identification of rare, positively stained CTCs. Enrichment, deposition of cells on a glass substrate, and scanning using an automated digital microscope are laborious and time consuming procedures. In order to circumvent these steps, we tested the capability of the QMAGEX assay to stain cells in suspension and for the positively stained cells to be identified by flow cytometry.

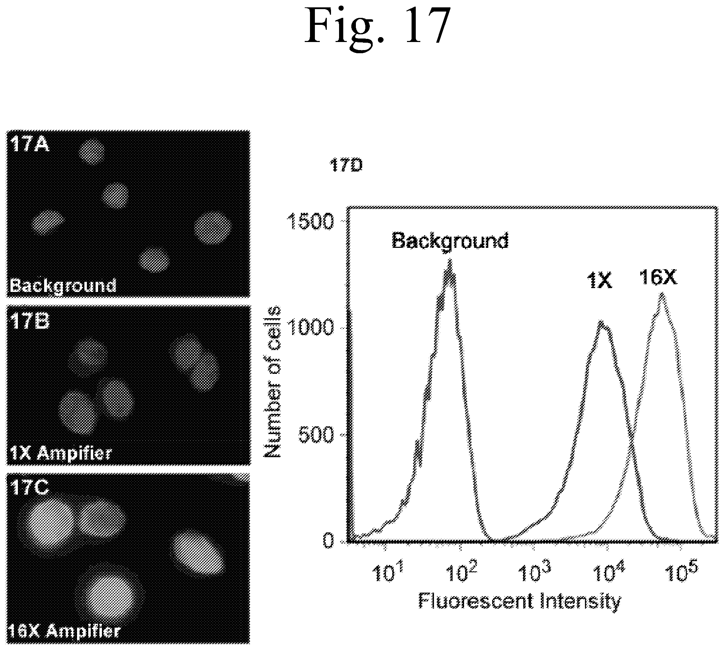

For the FC-QMAGEX assay, we first trypsinized HeLa cells grown on a substrate into suspension cells, and then hybridized the cells with 18S capture probes followed by signal amplification with either a 16X AMP2 or a 1X AMP3 and labeling using Alexa488. Positive staining was identified in the suspension HeLa cells by fluorescent microscopy and compared with control cells not hybridized with capture probes or signal amplifiers (**Figure 17** **Panels A-C**). The 16X AMP2 had a stronger fluorescent stain in rounded suspension HeLa cells than the 1X AMP3, consistent with the previous results on cells grown on substrate (**Figure 12** **Panels A-B**). We next determined the sensitivity of flow cytometry (LSR II, BD Biosciences) to detect and quantify 18S RNA expression in single cells with 50,000 cells counted per assay. The flow cytometric histogram (**Figure 17** **Panel D**) showed the detection of the 1X AMP3 having signals ∼100-fold above background, demonstrating a high level of detection sensitivity. Detection of cells with the 16X AMP2 lead to an approximately 10-fold increase in signal intensity over that seen with the 1X AMP3. Since the signal of 16X AMP2 is at the point of saturation in the detection scale, the 10-fold increase in signal over the 1X AMP3 is likely an underestimate of the true signal amplification achieved. To understand the contribution of background fluorescence in flow cytometry, we compared the background fluorescence from 1) cells hybridized with no capture probes and no signal amplifier or label probe (a measure of cellular autofluorescence); 2) cells hybridized with no capture probes but with 400X AMP1 and Alexa488 label probe; or 3) cells hybridized with 18S intron capture probes followed by 400X AMP1 and Alexa488 label probe. Little difference was seen in all the background fluorescence (data not shown) measured, suggesting that the background is mainly contributed by cellular autofluorescence. This result again demonstrates the value of the double "Z" design in reducing non-specific hybridization-related background, which had been several folds higher than cellular autofluorescence (e.g. Yu et al. (1991) "Sensitive detection of RNAs in single cells by flow cytometry" Nucleic Acids Res. 20(1):83-8). This study demonstrates that specific labeling and detection of 18S RNA can be achieved for HeLa cells in suspension and the 18S RNA level can be measured quantitatively by flow cytometry.

We tested a second marker, CK19, in the MCF7 cell line. We were also able to detect a strong positive signal over background by ∼400-fold (data not shown) These results demonstrate the feasibility of performing the QMAGEX assay in suspension, negating the need for a solid support and increasing the scanning speed to over 20,000 cells per second, far outpacing an automated digital microscope. Furthermore, the ability of a flow cytometer to detect a 1X amplification indicates that we can detect very low expressing transcripts and distinguish these from higher expressing mRNAs.

### Detection of low copy mRNA transcripts using FC-QMAGEX

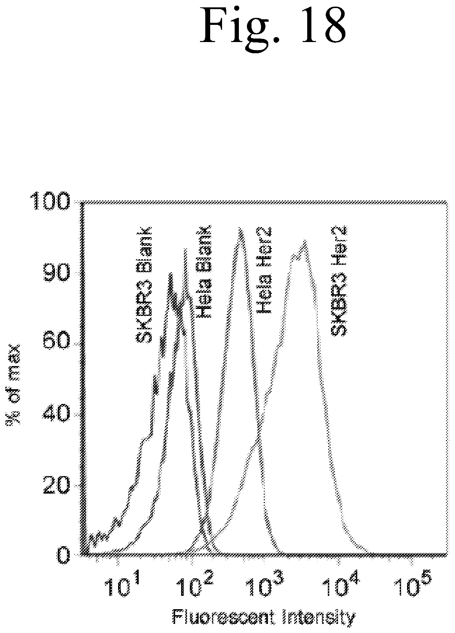

One of the hallmarks of cellular transformation is the upregulation of cancer specific genes. This increase in transcript number can be the result of genetic changes such as gene amplification, as is the case with a subset of breast cancers distinguished by an increase in HER2 gene copy number. To determine whether our flow cytometry based QMAGEX assay could distinguish these transformed cells from a general population that expresses only low basal levels of mRNA, we again used the SKBR3 cell line, which contains a HER2 gene amplification, and compared the Her-2 mRNA expression levels to those seen in the unamplified HeLa cell line. SKBR3 and HeLa cells were hybridized with Her2 capture probes, amplified with the 400X AMP1, and labeled with Alexa488. Unhybridized cells were used as a negative control for background fluorescence. The flow cytometric histogram showed an increase in signal intensity for both HeLa and SKBR3 cells over background (**Figure 18**). Since HeLa cells showed an average expression level of 5 copies of mRNA per cell in QuantiGene 2.0 and an average of 3 copies per cell in QMAGEX, this results suggest that the FC-QMAGEX assay is already highly sensitive, having detection sensitivity below 5 copies per cell. This result is in sharp contrast with the previous reported detection limit of -1,800 RNA transcripts in flow cytometry (Yu et al. (1991) "Sensitive detection of RNAs in single cells by flow cytometry" Nucleic Acids Res. 20(1):83-8), suggesting that FC-QMAGEX assays are able to detect a much greater number of functionally relevant genes in cell. In FC-QMAGEX, the SKBR3 cells, which contain a Her-2 gene amplification, showed an approximately 10-fold higher level of Her-2 expression than HeLa cells, consistent with previous observation when examined on glass substrate (**Figure 13** **Panels A-B**). Interestingly, the SKBR3 cell line shows a wider range of fluorescent intensities than HeLa cells. This is likely due to different levels of gene amplification in different cells resulting in varying degrees of Her-2 expression, a phenomenon that would not occur in HeLa cells carrying a normal gene copy number. These results demonstrate the feasibility of detecting both basal and overexpressed mRNAs in a mixed cell population using FC-QMAGEX. More importantly, these experiments indicate that CTCs overexpressing cancer cell markers can be identified by QMAGEX separately from WBCs without enrichment due to the fast sampling rate of over 20,000 cells per second by flow cytometry.

### Detection of mRNA transcripts in FFPE tissue sections and microarrays

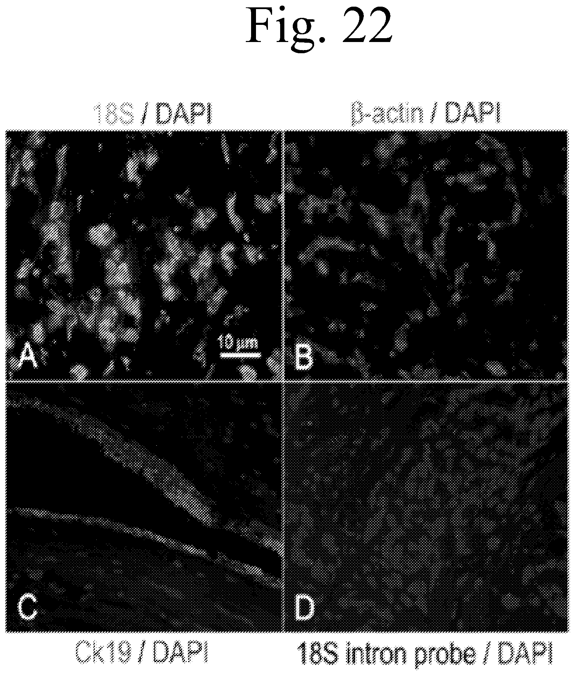

FFPE tissue section is a sample type widely used in pathology. FFPE tissue sections are generally considered to be more difficult to work with than cell lines and blood cells due to additional issues such as target access, RNA stability and autofluorescence. The techniques described herein, however, permit convenient detection of nucleic acids in FFPE tissue sections. The following experiments illustrate the potential and capability of QMAGEX for in situ detection of RNA transcripts in this particular sample type. **Figure 22** illustrates detection of various targets in breast cancer FFPE tissue section. **Figure 22** **Panels A and B** illustrate detection of genes with high levels of expression (>1,000 copies per cell), such as 18S (Alexa-488) and beta-actin (Fast Red) (**Figure 22** **Panels A and B**, respectively). Detection of mid-level expression genes (>100 and <1,000) such as CK19 (Fast Red) is illustrated in **Figure 22** **Panel C.** CK19 is a marker for epithelial cells and cancer epithelial cells. The fact that CK19 RNA is specifically detected in epithelial and cancer epithelial cells but not in neighboring stromal cells (**Figure 22** **Panel C**), and the fact the assay background is very low in FFPE tissue section (**Figure 22** **Panel D**), indicates that the FFPE-MAGEX assay is highly specific and is also applicable to very low copy RNA detection. Techniques are similar to those described for detection of RNA in situ in cell lines, although the FFPE tissue sections are also first subjected to de-paraffinization, de-crosslinking, and autofluorescence reduction using standard techniques.

A further experiment showing that techniques described herein permit detection of low copy RNAs in FFPE tissue sections is illustrated in Figure 23, which illustrates Her-2 mRNA detection in breast cancer FFPE samples. FFPE sections from breast cancer tissue were labeled using a MAGEX assay with either a probe set for the Her-2 marker (**Figure 23** **Panels A-C) or** no target probe (**Figure 23** **Panels D-F**). The left column **(Panels A and D)** shows Gill's Hematoxylin staining of the cell nuclei in the tissue section. The middle column **(Panels B and E)** shows the tissue section stained with a MAGEX assay using Her-2 probe **(Panel B)** or no target probe **(Panel E)** in combination with Fast Red substrate. The right column shows the merged pictures for Her-2/Gill's Hematoxylin (**Panel C**) and no target probe/Gill's Hematoxylin **(Panel F).** Low copy Her-2 is readily visualized and optionally quantitated in the FFPE samples.

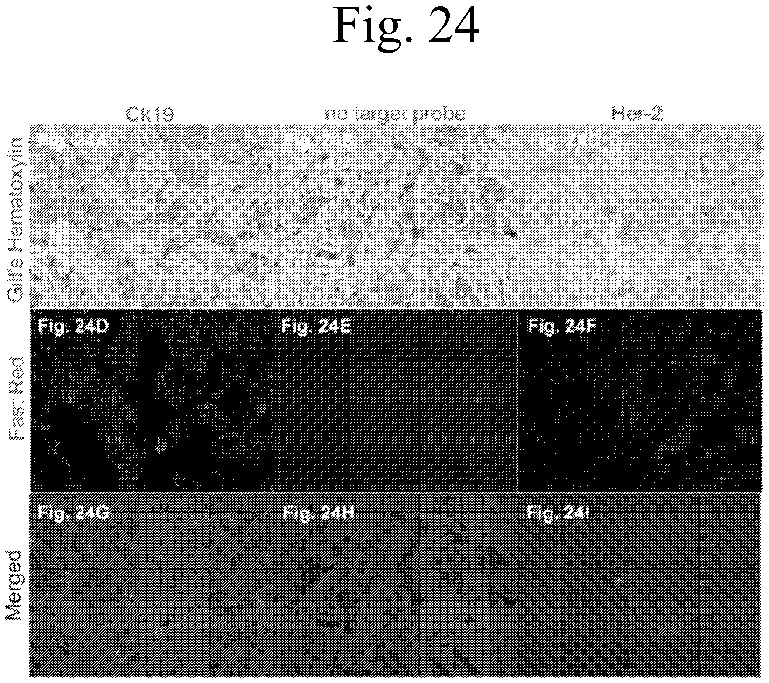

**Figure 24** illustrates mRNA detection in breast cancer tissue microarray (TMA) FFPE samples. FFPE tissue microarray from breast cancer tissues were labeled using a MAGEX assay with Ck19 (**Figure 24** left column, **Panels A, D and G**), Her-2 (right column, **Panels C, F, and I**) or no target probe (middle column, **Panels B, E, and H**). The top row (**Panels A-C**) shows Gill's Hematoxylin staining of the cell nuclei in the tissue sections. The middle row **(Panels D-F)** shows the tissue sections labeled with MAGEX assay using Ck19 probe (**Panel D**), Her-2 probe **(Panel F)** or no target probe **(Panel E)** in combination with Fast Red as a substrate. The bottom row shows merged pictures for Ck19/Gill's Hematoxylin (**Panel G**), Her-2/Gill's Hematoxylin (**Panel I**) and no target probe/Gill's Hematoxylin (**Panel H**).

### CTC identification in breast cancer patients

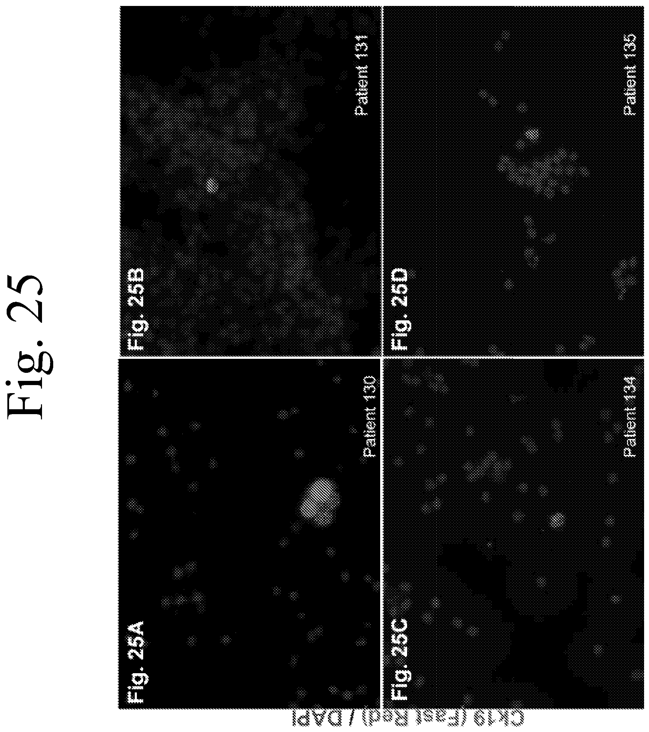

As noted, one exemplary application of techniques described herein is in identification of CTCs. **Figure 25** illustrates identification of CTCs in blood samples from breast cancer patients.

Nucleated cells were first purified from patient blood samples. Cells were then fixed onto glass slides and a MAGEX assay using Ck19 as the marker was used to identify the cancer cells. **Figure 25** **Panels A-D** show MAGEX Ck19 labeling of the cancer cells in four patient blood cell samples.

### Exemplary marker panel

As noted above, a number of markers can be employed to identify various cell types, including, for example, CTCs. As just one example, a panel of markers including mRNA transcripts CK19, MamA (mammaglobin A), CD45, and/or Her-2 can be employed, e.g., in a 4-plex QMAGEX assay identifying and characterizing SKBR3 cells spiked into blood or CTCs in metastatic breast cancer patients. CK19 has proven to be a highly expressed generic marker for tumor cells of epithelial origin. We have demonstrated its sensitivity and specificity in distinguishing cancer cells from white blood cells. MamA is another established marker for distinguishing breast cancer cell from blood cells (reviewed by Lacroix (2006) "Significance, detection and markers of disseminated breast cancer cells" Endocr Relat Cancer. 13(4): 1033-67). This marker is particularly useful in eliminating potential CK19 false positive skin epithelial cells which are introduced through needle aspiration of blood. CD45 can be used as a negative marker for cancer cell because it is a well known marker for blood cells and we have determined it to have no expression in cancer cells. Her-2 is used here to demonstrate the capability of QMAGEX for providing functional information on the CTCs. Several studies have shown that Her-2 gene amplification can be detected in CTCs not only in patients whose primary tumor is HER2+, but also in some patients whose primary tumor is HER2- (e.g., Hayes et al. (2002) "Monitoring expression of HER-2 on circulating epithelial cells in patients with advanced breast cancer" Int J Oncol. 21(5):1111-7, Meng et al. (2004) "HER-2 gene amplification can be acquired as breast cancer progresses" Proc. Nat. Acad. Sci. 101(25):9393-9398, and Wulfing et al. (2006) "HER2-positive circulating tumor cells indicate poor clinical outcome in stage I to III breast cancer patients" Clin Cancer Res. 12(6):1715-20). More interestingly, breast cancer patients whose primary tumor is HER2- but CTC HER2+ can respond to

Herceptin treatment, suggesting that determining HER2 status in CTC could be an effective way of guiding targeted therapy (Meng et al. (2004) supra). At the 2007 ASCO meeting, there were a number of studies showing that some patients with primary tumor HER2-status can also benefit from Herceptin treatment (e.g. Paik et al. (2007) "Benefit from adjuvant trastuzumab may not be confined to patients with IHC 3+ and/or FISH-positive tumors: central testing results from NSABP B-31" Program and abstracts of the 43rd American Society of Clinical Oncology Annual Meeting; June 1-5, 2007; Chicago, Illinois. Abstract 511). Thus it would be valuable to investigate whether HER2 status in CTCs can serve as a surrogate marker for targeted therapy selection. We believe that Her-2 mRNA is potentially a more accurate marker than HER2 DNA gene amplification because it is more directly related to its protein expression. In summary, three of the four RNA markers (CK19, MamA and CD45) are used to detect and distinguish breast cancer cells in blood through "Boolean Conditioning" (use of more than one independent markers to increase specificity of detection and decrease false positives, as described hereinabove) and one marker (Her-2) is used to provide functional information about the CTCs. Additional RNA markers for breast cancer cell detection in blood can also be employed (e.g., see review by Lacroix (2006) *supra*).

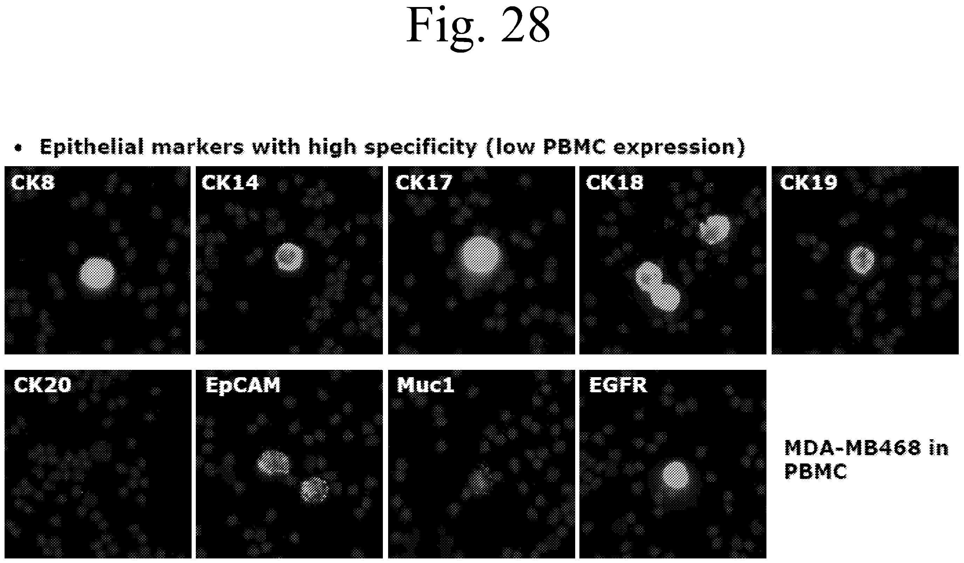

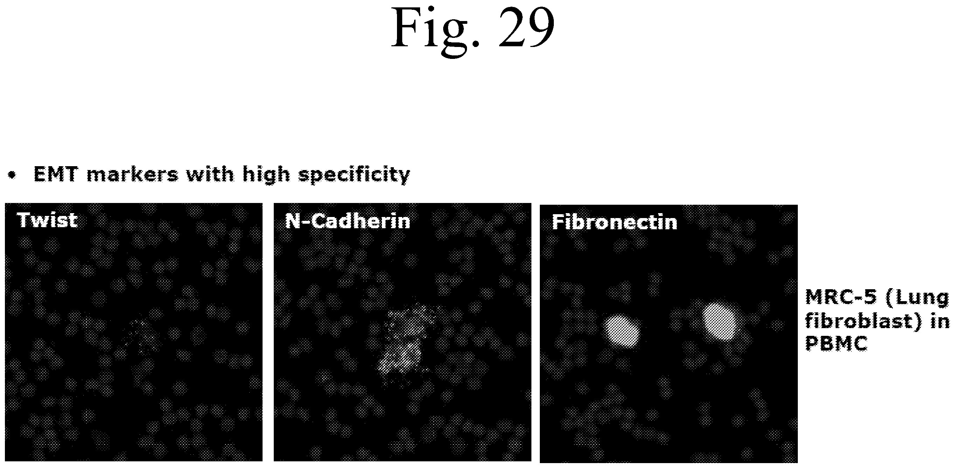

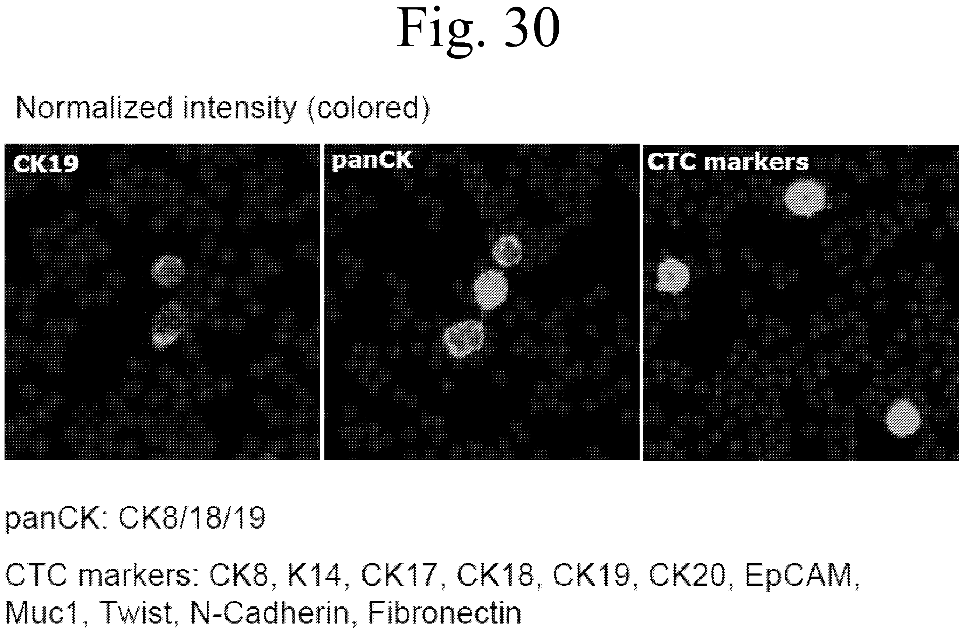

**Figure 28** shows the experimental validation data of nine RNA markers (CK8, CK14, CK17, CK18, CK19, CK20, EpCAM, Muc1 and EGFR) selected to identify epithelial-type CTCs. **Figure 29** shows the experimental validation data of three RNA markers (Twist, N-Cadherin and Fibronectin) selected to identify EMT CTCs. **Figure 30** shows the comparative signals from spiked in tumor cells using CK19, pan-CK and pan-CTC marker groups.

### MATERIALS AND METHODS

### Cell culture and PMA induction

All cell lines were obtained from American Type Cell Culture Collection (ATCC; Manassas, VA) and cultured in appropriate media. Cells were grown on glass coverslips coated with 1:10 dilution of poly-L-lysine solution (Sigma Diagnostics, Inc.; St. Louis, MO) using conditions provided by the ATCC. For PMA induction experiments, HeLa cells were cultured until 60%-70% confluency (18-20 hr at 37°C) in Dulbecco's Modified Eagle's Medium (DMEM, Invitrogen, Carlsbad, CA) containing 10 % serum followed by serum-free DMEM for 18 hr. Cells were then treated with 10 ng/ ml PMA (CalBiochem, San Diego) in serum-free DMEM and collected at various time point for analysis.

### Cell fixation and storage

Cells grown on coverslips were fixed with 4% formaldehyde in PBS (0.01 M phosphate buffer, pH7.5) at room temperature for 30 minutes. Fixed cells were washed in PBS, dehydrated through a graded ethanol series (50%, 70% and 100%) at room temperature and stored in 100% ethanol at -20°C. For in situ staining in suspension, cells were trypsinized and collected by centrifugation at 290 g for 10 min at room temperature. Pellets were re-suspended in 1XPBS and centrifuged at 290 g for 10 min at room temperature. Suspension cells were re-suspended in 4% formaldehyde in 1X PBS for 30 min at room temperature. Fixed cells were collected by centrifugation and dehydrated in the same way as for cells grown on coverslips.

### Oligonucleotide probes and signal amplification system

Target probes were designed using modified Probe Design Software (ProbeDesignerTM from Panomics, Inc.; see also Bushnell et al. (1999) "ProbeDesigner: for the design of probe sets for branched DNA (bDNA) signal amplification assays Bioinformatics 15:348-55). 13 pairs of DNA oligonucleotides containing sequence complementary to unique region of 18S rRNA were used to label 18S rRNAs. 52 pairs of DNA oligonucleotides complementary to region in ERBB2(Her-2) were used in detecting Her-2 mRNA. 23 pairs of DNA oligonucleotides complementary to region of Interlukin-6 (IL-6) were used in detecting IL-6 mRNA. 20 pairs of DNA oligonucleotides complementary to unique region of Interlukin-8 (IL-8) were used in detecting IL-8 mRNA. Signal amplification system including preAMP and AMP and fluorescent molecules or Alkaline phosphatase (AP)-conjugated label probes.

### RNA in situ hybridization on cells grown on coverslips

Fixed cells were re-hydrated through a graded ethanol series (100%, 70% and 50%) and washed 3 times in PBS. To access nuclear RNA, cells were washed in 1XPBS containing 0.1%Tween 20 for 3 min at room temperature. Cells were incubated in 2.5-5 µg/ml proteinase K in PBS for 10 min at room temperature and washed 3 times with PBS for 10 min total. After the proteinase K treatment, cells were incubated with 1 pmole of target probes in target buffer containing 6X SSC, 25% formamide, 0.2% Brij-35, 0.2% casein and 0.25% Blocking Reagent (Roche Diagnostics, Indianapolis, IN) at 40°C in a humidifying chamber for 3 hrs. For detecting 18S rRNA, 0.2 pmole target probe and 1.5 hr incubation time at 45°C in a humidifying chamber is sufficient. Cells were washed at room temperature with 2X SSC, 0.2X SSC and 0.1 X SSC containing 0.0025% Brij-35 detergent for 2 min each. Cells were then incubated with 100 fmole preAMP in Hybridization buffer B (15% formamide, 5X SSC, 0.3% SDS, 10 % Dextran Sulfate, 1 mM ZnCl2, 10 mM MgCl2, 0.025% Blocking Reagent (Roche Diagnostics, Indianapolis, IN), 0.1 mg/ml denatured ss DNA and 50 µg/ml yeast tRNA) in a humidifying chamber at 40°C for 25 min. Coverslips were washed in 0.1X SSC containing 1 mM EDTA 2 times for 2 min and 5 min at room temperature. Cells were incubated with 100 fmole AMP in hybridization buffer B in a humidifying chamber at 40°C for 15 min. Coverslips were washed in 0.1X SSC containing 1 mM EDTA 2 times for 2 min and 5 min at room temperature. Cells were incubated with 100 fmole AP-conjugated label probe or 5 pmole fluorescent molecules-conjugated label probe in hybridization buffer C (5X SSC, 0.3% SDS, 10 % Dextran Sulfate, 1 mM ZnCl2, 10 mM MgCl2, 0.025% Blocking Reagent, 0.1 mg/ml denatured ss DNA and 50 µg/ml yeast tRNA) in a humidifying chamber at 40°C for 15 min. Coverslips were washed in 0.1X SSC containing 1 mM EDTA 2 times for 2 min and 5 min at room temperature. If the AP-conjugated label probe was used, cells were incubated in Tris-HCl, pH8 containing 0.1% Brij-35, 1 mM ZnCl2 and 10 mM MgCl2 for 5 min followed by exposing the cells to Fast Red Substrate (Dako, Carpinteria, CA) for 10 min at room temperature. For using 16X AMP system, preAMP, AMP and label probes were used at 1 pmole, 1 pmole and 5 pmole concentrations. Coverslips were mounted onto slides using Vectashield containing DAPI (Vector Laboratories Inc., Burlingame, CA) or Prolong Gold anti-Fade Mounting medium (Invitrongen, Carlsbad,CA).

### RNA in situ hybridization on cells in suspension

Fixed cells were collected by centrifuging at 290 g for 5 min at room temperature. Cells were re-hydrated through Ethanol series (100%, 70% and 50%) and washed with 100 µl 1XPBS containing 2% BSA for 2 times. Cells were re-suspended and incubated in 100 µl of 1XPBS containing 0.25-0.5 µg proteinase K for 8 min at room temperature. Immediately after 8 min incubation with proteinase K solution, 25 µl of 10% BSA was added and cells were centrifuged at 290 g for 2 min. Supernatant was removed and cells were re-suspended in 100 µl 1XPBS containing 2% BSA. Cells were centrifuged at 290 g for 5 min and re-suspended in 100 µl 1XPBS containing 2% BSA. After centrifuging at 290 g for 5 min, supernatant was removed and cells were re-suspended in 100 µl of target buffer containing 1 pmole of target probes to incubate at 40°C water bath for 3 hrs. After hybridization, 25 µl of 10% BSA was added to each sample and centrifuged at 290 g for 5 min. Cells were washed at room temperature with 2X SSC, 0.2X SSC and 0.1 X SSC containing 0.0025% Brij-35 and 2% BSA for 2 min each. Cells were then incubated with 300 fmole preAMP in Hybridization buffer B' B (15% formamide, 5X SSC, 0.3% SDS, 5% Dextran Sulfate, 1 mM ZnCl2, 10 mM MgCl2, 0.025% Blocking Reagent (Roche Diagnostics, Indianapolis, IN), 0.1 mg/ml denatured ss DNA and 50 µg/ml yeast tRNA) in a 40°C water bath for 25 min. After hybridization, 25 µl of 10% BSA was added to each sample and centrifuged at 290 g for 5 min to collect cell pellets. Pellets were re-suspended and washed in 0.1X SSC containing 1 mM EDTA and 2% BSA for 2 times for 2 min and 5 min at room temperature. Cells were incubated with 300 fmole AMP in hybridization buffer B' at 40°C water bath for 15 min. After hybridization, 25 µl of 10% BSA was added to each sample and centrifuged at 290 g for 5 min to collect cell pellets. Cells were washed in 0.1X SSC containing 1 mM EDTA and 2% BSA for 2 times for 2 min and 5 min at room temperature. Cells were incubated with 300 fmole AP-conjugated label probe or 15 pmole fluorescent molecules-conjugated label probe in hybridization buffer C' (5X SSC, 0.3% SDS, 5 % Dextran Sulfate, 1 mM ZnCl2, 10 mM MgCl2, 0.025% Blocking Reagent (Roche Diagnostics, Indianapolis, IN), 0.1 mg/ml denatured ss DNA and 50 µg/ml yeast tRNA) at 40°C water bath for 15 min. After hybridization, 25 µl of 10% BSA was added to each sample and centrifuged at 290 g for 5 min to collect cell pellets. Cells were washed in 0.1X SSC containing 1 mM EDTA and 2% BSA for 2 times for 2 min and 5 min at room temperature. If the AP-conjugated label probe was used, cells were incubated in Tris-HCl, pH8 containing 0.1% Brij-35, 1 mM ZnCl2 and 10 mM MgCl2 for 5 min followed by exposing the cells to Fast Red Substrate (Dako, Carpinteria, CA) for 10 min at room temperature. For using 16X preAMP/AMP system, preAMP, AMP and label probes were used at 3 pmole, 3 pmole and 15 pmole concentrations. Fluorescent intensity of individual cells was analyzed using LSR flow cytometer (BD Biosciences, Franklin Lakes, NJ).

### Flow cytometric analysis

Labeled cells in suspension were analyzed using an LSR flow cytometer (BD Biosciences, Franklin Lakes, NJ). Flow cytometric data were analyzed using FlowJo Software (Tree Star Inc., Ashland, OR).

### Microscope and imaging

Slides were viewed under an Olympus IX71 fluorescent microscope and images were taken using Micro Suite B3 software. Fluorescent dot intensity was measured using CellProfiler (www (dot) cellprofiler (dot) org) and images were generated using Adobe Photoshop.

### Cell density and mRNA copy number estimation

To estimate the cell number on each coverslip, 4 coverslips were transferred to a clean 24-well dish, washed with PBS and treated with trypsin (Gibco) for 5-10 min at room temperature until the cells were detached. Trypsin was inactivated by adding 2 volume of medium containing 10% serum and cells were centrifuged at 200 g at room temperature for 5 min. Cells were re-suspended in 100 µl medium and cell number was estimated using a hemocytometer or Z2 Coulter Particle Counter (Beckman Coulter, Fullerton, CA). To estimate the average number of mRNA transcripts within each cell, 4 coverslips were transferred to clean 24-well dish and wash with PBS. Cell lysates were prepared, stored and mRNA copy numbers per cell were assayed according to QuantiGene 2.0 kit protocol (Panomics, Fremont, CA). RNA copy number was estimated by comparing signals from in vitro transcribed RNAs.

## Claims

1. A method of detecting an individual cell of a specified type in a sample comprising a mixture of cell types, which mixture comprises at least one cell of the specified type, wherein the cell comprises a first nucleic acid target and a second nucleic acid target, wherein the first and second nucleic acid targets always co-exist in the cell, the method comprising:
(a) providing a first label probe comprising a first label, and providing a second label probe comprising a second label, wherein a first signal from the first label is indistinguishable from a second signal from the second label;
(b) capturing, in the cell, the first label probe to the first nucleic acid target, when present in the cell, and the second label probe to the second nucleic acid target, when present in the cell, wherein the first and second nucleic acid targets are separate molecules, wherein the capturing comprises:
providing for the first nucleic acid target at least a first capture probe and providing for the second nucleic acid target at least a second capture probe;
capturing the first label probe to the first nucleic acid target,
comprising hybridizing in the cell the first capture probe to the first target nucleic acid, and hybridizing the first label probe to the first capture probe, thereby capturing the first label probe to the first nucleic acid target; and
capturing the second label probe to the second nucleic acid target,
comprising hybridizing in the cell the second capture probe to the second target nucleic acid, and hybridizing the second label probe to the second capture probe, thereby capturing the second label probe to the second nucleic acid target;
(c) detecting the signal from the labels;
(d) correlating the signal detected from the cell with the presence or amount of the first and second nucleic acid targets in the cell; and
(e) identifying the cell as being of the specified type based on detection of the presence or amount of the first and second nucleic acid targets within the cell, wherein the specified type of cell is distinguishable from the other cell type(s) in the mixture on the basis of either the presence or amount of the first nucleic acid target and the presence or amount of the second nucleic acid target in the cell.

2. The method of claim 1, wherein providing at least a first capture probe comprises providing two or more different first capture probes as a set, wherein providing at least a second capture probe comprises providing two or more different second capture probes as a set; wherein capturing the first label probe to the first nucleic acid target comprises hybridizing in the cell the two or more different first capture probes to the first target nucleic acid, and hybridizing the first label probe to the two or more different first capture probes, thereby capturing the first label probe to the first nucleic acid target; wherein capturing the second label probe to the second nucleic acid target comprises hybridizing in the cell the two or more different second capture probes to the second target nucleic acid, and hybridizing the second label probe to the two or more different second capture probes, thereby capturing the second label probe to the second nucleic acid target.

3. The method of claim 1, wherein providing at least a first capture probe comprises providing two or more different first capture probes as a set; wherein providing at least a second capture probe comprises providing two or more different second capture probes as a set; wherein the method further comprises providing an amplifier, wherein amplifier is a molecule capable of hybridizing to multiple label probes; wherein capturing the first label probe to the first nucleic acid target comprises hybridizing in the cell the first target nucleic acid to the two or more different first capture probes, hybridizing the two or more different first capture probes to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the amplifier, and hybridizing the amplifier to the second label probe, thereby capturing the second label probe to the second nucleic acid target.

4. The method of claim 1, wherein providing at least a first capture probe comprises providing two or more different first capture probes as a set; wherein providing at least a second capture probe comprises providing two or more different second capture probes as a set; wherein the method further comprises providing an amplifier and a preamplifier; wherein capturing the first label probe to the first nucleic acid target comprises hybridizing in the cell the first target nucleic acid to the two or more different first capture probes, hybridizing the two or more different first capture probes to the preamplifier, hybridizing the preamplifier to the amplifier, and hybridizing the amplifier to the first label probe, thereby capturing the first label probe to the first nucleic acid target; wherein capturing the second label probe to the second nucleic acid target comprises hybridizing in the cell the second target nucleic acid to the two or more different second capture probes, hybridizing the two or more different second capture probes to the preamplifier, hybridizing the preamplifier to the amplifier, and hybridizing the amplifier to the second label probe, thereby capturing the second label probe to the second nucleic acid target.

5. The method of any one of claims 2-4, wherein hybridizing the two or more different capture probes to the label probe, amplifier, or preamplifier is performed at a hybridization temperature that is greater than a melting temperature Tm of a complex between each individual capture probe and the label probe, amplifier, or preamplifier.

6. The method of claim 5, wherein the two or more different capture probes hybridize to unique and adjacent sections on the nucleic acid target.

7. The method of any one of claims 2-4, wherein hybridizing the two or more different capture probes to their corresponding nucleic acid target is performed at a hybridization temperature that is lower than a melting temperature Tm of a complex between each individual capture probe and the nucleic acid target, or
wherein hybridizing the two or more different capture probes to their corresponding nucleic acid target is performed at a hybridization temperature that is greater than a melting temperature Tm of a complex between each individual capture probe and the nucleic acid target.

8. The method of claim 1, further comprising providing a plurality of additional nucleic acid targets; wherein the method further comprises: providing a plurality of label probes each comprising a label, wherein the signal from each label is indistinguishable from the first and second signals or from each other; providing at least one capture probe for each of the additional nucleic acid targets, hybridizing in the cell each capture probe to its corresponding nucleic acid target, when present in the cell, and hybridizing each label probe to its corresponding capture probe, and detecting the signal from the labels.

9. The method of any one of claims 1-8, wherein the first nucleic acid target and the second nucleic acid target are independently selected from the group consisting of: a DNA, an RNA, a chromosomal DNA, an mRNA, a nucleic acid endogenous to the cell, a nucleic acid introduced to or expressed in the cell by infection of the cell with a pathogen and a cytoplasmic RNA.

10. The method of claim 9, wherein:
(i) the first nucleic acid target is a first mRNA and wherein the second nucleic acid target is a second mRNA; or
(ii) the first nucleic acid target comprises a first chromosomal DNA polynucleotide sequence and wherein the second nucleic acid target comprises a second chromosomal DNA polynucleotide sequence; or
(iii) the first nucleic acid target and/or the second nucleic acid target comprises a double-stranded DNA molecule, the method comprising denaturing the double-stranded DNA prior to hybridization of the first and second capture probes to the first and second nucleic acid targets.

11. The method of any one of claims 1-8, wherein
(i) about 1000 copies or less of the first nucleic acid target and/or about 1000 copies or less of the second nucleic acid target are present in the cell; or
(ii) about 100 copies or less of the first nucleic acid target and/or about 100 copies or less of the second nucleic acid target are present in the cell; or
(iii) about 10 copies or less of the first nucleic acid target and/or about 10 copies or less of the second nucleic acid target are present in the cell; or
(iv) about 5 copies or less of the first nucleic acid target and/or about 5 copies or less of the second nucleic acid target are present in the cell; or
(v) a single copy of the first nucleic acid target and/or a single copy of the second nucleic acid target is present in the cell.

12. The method of any one of claims 1-8, wherein the first nucleic acid target, the second nucleic acid target, and/or the additional nucleic acid target is selected from the group consisting of: CK8, CK14, CK17, CK18, CK19, CK20, EpCAM, MucI, EGFR, Twist, N-Cadherin and Fibronectin.

13. A method of detecting the presence or absence of a plurality of nucleic acid targets in an individual cell in a sample comprising the cell, which cell comprises or is suspected of comprising said plurality of nucleic acid targets, wherein said nucleic acid targets always co-exist in the cell, the method comprising:
(a) providing, for each of the plurality of nucleic acid targets, a label probe comprising a label, wherein a signal from the label of a first nucleic acid target in the plurality of nucleic acid targets is indistinguishable from a signal from the label of a second nucleic acid target in the plurality of nucleic acid targets, wherein the first and the second nucleic acid targets are separate molecules;
(b) providing, for each of the plurality of nucleic acid targets, at least a capture probe;
(c) hybridizing in the cell, for each of the plurality of nucleic acid targets, the capture probe to its corresponding nucleic acid target, when present in the cell;
(d) capturing, for each of the plurality of nucleic acid targets, the label probe to the capture probe, thereby capturing the label probe to its corresponding nucleic acid target; and
(e) detecting the signal from the label of the label probes captured to the plurality of nucleic acid targets,
wherein the presence of signal indicates the presence of a member of the plurality of nucleic acid targets and the absence of signal indicates the absence of the nucleic acid targets in the cell.

14. The method of claim 13, wherein the plurality of nucleic acid targets comprise housekeeping genes of different tissues in a particular species, wherein the species is preferably a human.

15. The method of any one of claims 13-14, wherein
(i) providing at least a capture probe comprises providing two or more capture probes; wherein each of the two or more capture probes comprises a T section which is complementary to a region of its corresponding nucleic acid target and a L section which is complementary to a region of its corresponding label probe; further, the T sections of two or more capture probes are complementary to non-overlapping regions of the nucleic acid target and the L sections of the two or more capture probes are complementary to non-overlapping regions of the label probe, and wherein the method comprises hybridizing a label probe to the two or more capture probes;
(ii) providing at least a capture probe comprises providing two or more capture probes; wherein the method further comprises providing an amplifier; and wherein the method comprises hybridizing a label probe to an amplifier and hybridizing the amplifier to said two or more capture probes; or
(iii) providing at least a capture probe comprises providing two or more capture probes; wherein the method further comprises providing an amplifier and a preamplifier; and wherein the method comprises hybridizing the preamplifier to the two or more capture probes, hybridizing the amplifier to the preamplifier and hybridizing a label probe to the amplifier.

16. The method of claim 15(i), comprising the step of hybridizing each set of two or more capture probes to the corresponding target nucleic acid at a hybridization temperature (a) greater than the melting temperature of each T section of two or more capture probes in the set, or (b) greater than the melting temperature of each L section of two or more capture probes in the set.

17. The method of claim 16, comprising the step of hybridizing each set of two or more capture probes to the corresponding target nucleic acid at a hybridization temperature (a) greater than the melting temperature of each T section of two or more capture probes in the set and lower than the melting temperature of each L section of two or more capture probes in the set, or (b) greater than the melting temperature of each L section of two or more capture probes in the set and lower than the melting temperature of each T section of two or more capture probes in the set.

18. The method of any one of claims 13-17, wherein the plurality of nucleic acid targets are independently selected from the group consisting of: a DNA, an RNA, a chromosomal DNA, an mRNA, a nucleic acid endogenous to the cell, a nucleic acid introduced to or expressed in the cell by infection of the cell with a pathogen and cytoplasmic RNAs.

19. The method of any one of claims 15(i), and 16-17, wherein the two or more capture probes in each set all have the T sections 5 ' of the L sections, or wherein the two or more capture probes in each set all have the T sections 3' of the L sections.

20. The method of any one of claims 1-19, wherein each hybridization or capture step is accomplished for all of the nucleic acid targets at the same time.

21. The method of any one of claims 1-20, wherein the cell is a circulating tumor cell.

22. The method of any one of claims 1-20, wherein the cell is in suspension in the sample comprising the cell, and/or wherein the cell is in suspension during the hybridizing, capturing, and/or detecting steps.

23. The method of any one of claims 1-20, wherein the sample comprising the cell is derived from a bodily fluid, blood, or a tissue section.

24. The method of any one of claims 1-20, wherein providing a sample comprises fixing and permeabilizing the cell.

25. The method of any one of claims 1-20, wherein the method comprises washing the cell to remove materials not captured to one of the nucleic acid targets.

26. The method of any one of claims 1-20, wherein detecting the signal comprises performing flow cytometry.

## Patentansprüche

1. Verfahren zum Nachweisen einer einzelnen Zelle eines spezifizierten Typs in einer Probe, umfassend eine Mischung von Zelltypen, wobei die Mischung wenigstens eine Zelle des spezifizierten Typs umfasst, wobei die Zelle ein erstes Nukleinsäuretarget und ein zweites Nukleinsäuretarget umfasst, wobei die ersten und zweiten Nukleinsäuretargets immer gleichzeitig in der Zelle existieren, wobei das Verfahren umfasst:
(a) Bereitstellen einer ersten Markierungssonde, die eine erste Markierung umfasst, und Bereitstellen einer zweiten Markierungssonde, die eine zweite Markierung umfasst, wobei ein erstes Signal der ersten Markierung von einem zweiten Signal der zweiten Markierung nicht unterscheidbar ist;
(b) Einfangen in der Zelle der ersten Markierungssonde an dem ersten Nukleinsäuretarget, wenn es in der Zelle anwesend ist, und der zweiten Markierungssonde an dem zweiten Nukleinsäuretarget, wenn es in der Zelle anwesend ist, wobei die ersten und zweiten Nukleinsäuretargets separate Moleküle sind, wobei das Einfangen umfasst:
Bereitstellen wenigstens einer ersten Einfangsonde für das erste Nukleinsäuretarget und Bereitstellen wenigstens einer zweiten Einfangsonde für das zweite Nukleinsäuretarget;
Einfangen der ersten Markierungssonde an dem ersten Nukleinsäuretarget, umfassend ein Hybridisieren der ersten Einfangsonde an die erste Zielnukleinsäure und ein Hybridisieren der ersten Markierungssonde an die erste Einfangsonde in der Zelle, wodurch die erste Markierungssonde an dem ersten Nukleinsäuretarget eingefangen wird; und
Einfangen der zweiten Markierungssonde an dem zweiten Nukleinsäuretarget, umfassend ein Hybridisieren der zweiten Einfangsonde an die zweite Zielnukleinsäure und ein Hybridisieren der zweiten Markierungssonde an die zweite Einfangsonde in der Zelle, wodurch die zweite Markierungssonde an dem zweiten Nukleinsäuretarget eingefangen wird;
(c) Nachweisen des Signals von den Markierungen;
(d) Korrelieren des von der Zelle detektierten Signals mit der Anwesenheit oder der Menge der ersten und der zweiten Nukleinsäuretargets in der Zelle; und
(e) Identifizieren der Zelle, dass sie vom spezifizierten Typ ist, basierend auf dem Nachweis der Anwesenheit oder der Menge der ersten und der zweiten Nukleinsäuretargets innerhalb der Zelle,
wobei der spezifizierte Zelltyp von dem oder den anderen Zelltypen in der Mischung auf der Basis von entweder der Anwesenheit oder Menge des ersten Nukleinsäuretargets und der Anwesenheit oder Menge des zweiten Nukleinsäuretargets in der Zelle unterscheidbar ist.

2. Verfahren nach Anspruch 1, wobei das Bereitstellen wenigstens einer ersten Einfangsonde ein Bereitstellen von zwei oder mehr verschiedenen ersten Einfangsonden als Satz umfasst, wobei das Bereitstellen wenigstens einer zweiten Einfangsonde ein Bereitstellen von zwei oder mehr verschiedenen zweiten Einfangsonden als Satz umfasst; wobei das Einfangen der ersten Markierungssonde an dem ersten Nukleinsäuretarget ein Hybridisieren der zwei oder mehr unterschiedlichen ersten Einfangsonden an die erste Targetnukleinsäure und ein Hybridisieren der ersten Markierungssonde an die zwei oder mehr unterschiedlichen ersten Einfangsonden in der Zelle umfasst, wodurch ein Einfangen der ersten Markierungssonde an dem ersten Nukleinsäuretarget erfolgt; wobei das Einfangen der zweiten Markierungssonde an dem zweiten Nukleinsäuretarget ein Hybridisieren der zwei oder mehr verschiedenen zweiten Einfangsonden an der zweiten Targetnukleinsäure und ein Hybridisieren der zweiten Markierungssonde an die zwei oder mehr verschiedenen zweiten Einfangsonden in der Zelle umfasst, wodurch die zweite Markierungssonde an dem zweiten Nukleinsäuretarget eingefangen wird.

3. Verfahren nach Anspruch 1, wobei das Bereitstellen wenigstens einer ersten Einfangsonde ein Bereitstellen von zwei oder mehr verschiedenen ersten Einfangsonden als Satz umfasst; wobei das Bereitstellen wenigstens einer zweiten Einfangsonde ein Bereitstellen von zwei oder mehr verschiedenen zweiten Einfangsonden als Satz umfasst; wobei das Verfahren ferner ein Bereitstellen eines Verstärkers umfasst, wobei der Verstärker ein Molekül ist, das zur Hybridisierung an mehrere Markierungssonden fähig ist; wobei das Einfangen der ersten Markierungssonde an dem ersten Nukleinsäuretarget ein Hybridisieren der ersten Targetnukleinsäure an die zwei oder mehr verschiedenen ersten Einfangsonden, ein Hybridisieren der zwei oder mehr verschiedenen ersten Einfangsonden an den Verstärker und ein Hybridisieren des Verstärkers an die erste Markierungssonde in der Zelle umfasst, wodurch die erste Markierungssonde an dem ersten Nukleinsäuretarget eingefangen wird; wobei das Einfangen der zweiten Markierungssonde an dem zweiten Nukleinsäuretarget ein Hybridisieren der zweiten Targetnukleinsäure an die zwei oder mehr verschiedenen zweiten Einfangsonden, ein Hybridisieren der zwei oder mehr verschiedenen zweiten Einfangsonden an den Verstärker und ein Hybridisieren des Verstärkers an die zweite Markierungssonde in der Zelle umfasst, wodurch die zweite Markierungssonde an dem zweiten Nukleinsäuretarget eingefangen wird.

4. Verfahren nach Anspruch 1, wobei das Bereitstellen wenigstens einer ersten Einfangsonde ein Bereitstellen von zwei oder mehr verschiedenen ersten Einfangsonden als Satz umfasst; wobei das Bereitstellen wenigstens einer zweiten Einfangsonde ein Bereitstellen von zwei oder mehr verschiedenen zweiten Einfangsonden als Satz umfasst; wobei das Verfahren ferner ein Bereitstellen eines Verstärkers und eines Vorverstärkers umfasst; wobei das Einfangen der ersten Markierungssonde an dem ersten Nukleinsäuretarget ein Hybridisieren des ersten Nukleinsäuretargets an die zwei oder mehr unterschiedlichen ersten Einfangsonden, ein Hybridisieren der zwei oder mehr unterschiedlichen ersten Einfangsonden an den Vorverstärker, ein Hybridisieren des Vorverstärkers an den Verstärker und ein Hybridisieren des Verstärkers an die erste Markierungssonde in der Zelle umfasst, wodurch die erste Markierungssonde an dem ersten Neukleinsäuretargt eingefangen wird; wobei das Einfangen der zweiten Markierungssonde an dem zweiten Nukleinsäuretarget ein Hybridisieren des zweiten Nukleinsäuretargets an die zwei oder mehr unterschiedlichen Einfangsonden, ein Hybridisieren der zwei oder mehr unterschiedlichen zweiten Einfangsonden an den Vorverstärker, ein Hybridisieren des Vorverstärkers an den Verstärker und ein Hybridisieren des Verstärkers an die zweite Markierungssonde in der Zelle umfasst, wodurch die zweite Markierungssonde an dem zweiten Nukleinsäuretarget eingefangen wird.

5. Verfahren nach einem der Ansprüche 2 bis 4, wobei das Hybridisieren der zwei oder mehr verschiedenen Einfangsonden an die Markierungssonde, den Verstärker oder den Vorverstärker bei einer Hybridisierungstemperatur durchgeführt wird, die größer ist als eine Schmelztemperatur Tm eines Komplexes zwischen jeder einzelnen Einfangsonde und der Markierungssonde, dem Verstärker oder dem Vorverstärker.

6. Verfahren nach Anspruch 5, wobei die zwei oder mehr verschiedenen Einfangsonden an einzigartige und benachbarte Abschnitte auf dem Nukleinsäuretarget hybridisieren.

7. Verfahren nach einem der Ansprüche 2 bis 4, wobei das Hybridisieren der zwei oder mehr verschiedenen Einfangsonden an ihr entsprechendes Nukleinsäuretarget bei einer Hybridisierungstemperatur durchgeführt wird, die niedriger ist als eine Schmelztemperatur Tm eines Komplexes zwischen jeder einzelnen Einfangsonde und dem Nukleinsäuretarget oder
wobei das Hybridisieren der zwei oder mehr verschiedenen Einfangsonden an ihr entsprechendes Nukleinsäuretarget bei einer Hybridisierungstemperatur durchgeführt wird, die größer ist als eine Schmelztemperatur Tm eines Komplexes zwischen jeder einzelnen Einfangsonde und dem Nukleinsäuretarget.

8. Verfahren nach Anspruch 1, ferner umfassend ein Bereitstellen einer Vielzahl von zusätzlicher Nukleinsäuretargets; wobei das Verfahren ferner umfasst: Bereitstellen einer Vielzahl von Markierungssonden, die jeweils eine Markierung umfassen, wobei das Signal von jeder Markierung von den ersten und zweiten Signalen oder voneinander nicht unterscheidbar ist; Bereitstellen wenigstens einer Einfangsonde für jedes der zusätzlichen Nukleinsäuretargets, Hybridisieren jeder Einfangsonde an ihr entsprechendes Nukleinsäuretarget in der Zelle, sofern in der Zelle anwesend, und Hybridisieren jeder Markierungssonde an ihre entsprechende Einfangsonde und Nachweisen des Signals von den Markierungen.

9. Verfahren nach einem der Ansprüche 1 bis 8, wobei das erste Nukleinsäuretarget und das zweite Nukleinsäuretarget unabhängig voneinander ausgewählt sind aus der Gruppe bestehend aus: einer DNA, einer RNA, einer chromosomalen DNA, einer mRNA, einer für die Zelle endogenen Nukleinsäure, einer Nukleinsäure, die durch Infektion der Zelle mit einem Pathogen in die Zelle eingeführt oder von dieser exprimiert wird, und einer cytoplasmatischen RNA.

10. Verfahren nach Anspruch 9, wobei:
(i) das erste Nukleinsäuretarget eine erste mRNA ist und wobei das zweite Nukleinsäuretarget eine zweite mRNA ist; oder
(ii) das erste Nukleinsäuretarget eine erste chromosomale DNA-Polynukleotidsequenz umfasst und wobei das zweite Nukleinsäuretarget eine zweite chromosomale DNA-Polynukleotidsequenz umfasst; oder
(iii) das erste Nukleinsäuretarget und/oder das zweite Nukleinsäuretarget ein doppelsträngiges DNA-Molekül umfasst, wobei das Verfahren das Denaturieren der doppelsträngigen DNA vor der Hybridisierung der ersten und zweiten Einfangsonde an die ersten und zweiten Nukleinsäuretargets umfasst.

11. Verfahren nach einem der Ansprüche 1 bis 8, wobei
(i) ungefähr 1000 Kopien oder weniger des ersten Nukleinsäuretargets und/oder ungefähr 1000 Kopien oder weniger des zweiten Nukleinsäuretargets in der Zelle anwesend sind; oder
(ii) ungefähr 100 Kopien oder weniger des ersten Nukleinsäuretargets und/oder ungefähr 100 Kopien oder weniger des zweiten Nukleinsäuretargets in der Zelle anwesend sind; oder
(iii) ungefähr 10 Kopien oder weniger des ersten Nukleinsäuretargets und/oder ungefähr 10 Kopien oder weniger des zweiten Nukleinsäuretargets in der Zelle anwesend sind; oder
(iv) ungefähr 5 Kopien oder weniger des ersten Nukleinsäuretargets und/oder ungefähr 5 Kopien oder weniger des zweiten Nukleinsäuretargets in der Zelle anwesend sind; oder
(v) eine einzelne Kopie des ersten Nukleinsäuretargets und/oder eine einzelne Kopie des zweiten Nukleinsäuretargets in der Zelle anwesend ist.

12. Verfahren nach einem der Ansprüche 1 bis 8, wobei das erste Nukleinsäuretarget, das zweite Nukleinsäuretarget und/oder das zusätzliche Nukleinsäuretarget ausgewählt sind aus der Gruppe bestehend aus: CK8, CK14, CK17, CK18, CK19, CK20, EpCAM, MucI, EGFR, Twist, N-Cadherin und Fibronectin.

13. Verfahren zum Nachweisen der Anwesenheit oder Abwesenheit einer Vielzahl von Nukleinsäuretargets in einer einzelnen Zelle in einer Probe, die die Zelle umfasst, wobei die Zelle die Vielzahl von Nukleinsäuretargets umfasst oder von dieser vermutet wird, diese zu umfassen, wobei die Nukleinsäuretargets immer gleichzeitig in der Zelle existieren, wobei das Verfahren umfasst:
(a) Bereitstellen einer Markierungssonde, die eine Markierung umfasst, für jede der Vielzahl von Nukleinsäuretargets, wobei ein Signal von der Markierung eines ersten Nukleinsäuretargets in der Vielzahl von Nukleinsäuretargets von einem Signal von der Markierung eines zweiten Nukleinsäuretargets in der Vielzahl von Nukleinsäuretargets nicht unterscheidbar ist, wobei das erste und das zweite Nukleinsäuretarget separate Moleküle sind;
(b) Bereitstellen von wenigstens einer Einfangsonde für jede der Vielzahl von Nukleinsäuretargets;
(c) Hybridisieren der Einfangsonde für jede der Vielzahl von Nukleinsäuretargets an ihr entsprechendes Nukleinsäuretarget in der Zelle, sofern in der Zelle anwesend;
(d) Einfangen der Markierungssonde an der Einfangsonde für jede der Vielzahl von Nukleinsäuretargets, wodurch die Markierungssonde an ihrem entsprechenden Nukleinsäuretarget eingefangen wird; und
(e) Nachweisen des Signals von der Markierung der Markierungssonden, die an der Vielzahl von Nukleinsäuretargets eingefangenen wurden, wobei die Anwesenheit eines Signals die Anwesenheit eines Mitglieds der Vielzahl von Nukleinsäuretargets anzeigt und die Abwesenheit eines Signals die Abwesenheit der Nukleinsäuretargets in der Zelle anzeigt.

14. Verfahren nach Anspruch 13, wobei die Vielzahl von Nukleinsäuretargets Housekeeping-Gene verschiedener Gewebe in einer bestimmten Spezies umfasst, wobei die Spezies vorzugsweise ein Mensch ist.

15. Verfahren nach einem der Ansprüche 13 bis 14, wobei
(i) Bereitstellen wenigstens einer Einfangsonde ein Bereitstellen von zwei oder mehr Einfangsonden umfasst; wobei jede der zwei oder mehr Einfangsonden einen T-Abschnitt, der zu einer Region ihrer entsprechenden Nukleinsäuretargets komplementär ist, und einen L-Abschnitt, der zu einer Region ihrer entsprechenden Markierungssonde komplementär ist, umfasst; wobei ferner die T-Abschnitte von zwei oder mehr Einfangsonden zu nicht überlappenden Bereichen des Nukleinsäuretargets komplementär sind und die L-Abschnitte der zwei oder mehr Einfangsonden zu nicht überlappenden Bereichen der Markierungssonde komplementär sind und wobei das Verfahren ein Hybridisieren einer Markierungssonde an die zwei oder mehr Einfangsonden umfasst;
(ii) Bereitstellen wenigstens einer Einfangsonde ein Bereitstellen von zwei oder mehr Einfangsonden umfasst; wobei das Verfahren ferner ein Bereitstellen eines Verstärkers umfasst; und wobei das Verfahren ein Hybridisieren einer Markierungssonde an einen Verstärker und ein Hybridisieren des Verstärkers an die zwei oder mehr Einfangsonden umfasst; oder
(iii) Bereitstellen wenigstens einer Einfangsonde ein Bereitstellen von zwei oder mehr Einfangsonden umfasst; wobei das Verfahren ferner ein Bereitstellen eines Verstärkers und eines Vorverstärkers umfasst; und wobei das Verfahren ein Hybridisieren des Vorverstärkers an die zwei oder mehr Einfangsonden, ein Hybridisieren des Verstärkers an den Vorverstärker und ein Hybridisieren einer Markierungssonde an den Verstärker umfasst.

16. Verfahren nach Anspruch 15(i), umfassend den Schritt des Hybridisierens jedes Satzes von zwei oder mehr Einfangsonden an das entsprechende Nukleinsäuretarget bei einer Hybridisierungstemperatur, die (a) größer ist als die Schmelztemperatur jedes T-Abschnitts von zwei oder mehr Einfangsonden im Satz oder (b) größer ist als die Schmelztemperatur jedes L-Abschnitts von zwei oder mehr Einfangsonden im Satz.

17. Verfahren nach Anspruch 16, umfassend den Schritt des Hybridisierens jedes Satzes von zwei oder mehr Einfangsonden an das entsprechende Nukleinsäuretarget bei einer Hybridisierungstemperatur, die (a) größer ist als die Schmelztemperatur jedes T-Abschnitts von zwei oder mehr Einfangsonden im Satz und niedriger ist als die Schmelztemperatur jedes L-Abschnitts von zwei oder mehr Einfangsonden im Satz oder (b) größer ist als die Schmelztemperatur jedes L-Abschnitts von zwei oder mehr Einfangsonden im Satz und niedriger ist als die Schmelztemperatur jedes T-Abschnitts von zwei oder mehr Einfangsonden im Satz.

18. Verfahren nach einem der Ansprüche 13 bis 17, wobei die Vielzahl von Nukleinsäuretargets unabhängig voneinander ausgewählt ist aus der Gruppe bestehend aus: einer DNA, einer RNA, einer chromosomalen DNA, einer mRNA, einer für die Zelle endogenen Nukleinsäure, einer Nukleinsäure, die durch Infektion der Zelle mit einem Pathogen in die Zelle eingeführt oder von dieser exprimiert wird, und cytoplasmatischen RNAs.

19. Verfahren nach einem der Ansprüche 15(i) und 16 bis 17, wobei die zwei oder mehr Einfangsonden in jedem Satz alle die T-Abschnitte 5' von den L-Abschnitten aufweisen oder wobei die zwei oder mehr Einfangsonden in jedem Satz alle die T-Abschnitte 3' von den L-Abschnitten aufweisen.

20. Verfahren nach einem der Ansprüche 1 bis 19, wobei jeder Hybridisierungs- oder Einfangschritt für alle Nukleinsäuretargets zur gleichen Zeit durchgeführt wird.

21. Verfahren nach einem der Ansprüche 1 bis 20, wobei die Zelle eine zirkulierende Tumorzelle ist.

22. Verfahren nach einem der Ansprüche 1 bis 20, wobei sich die Zelle in der die Zelle umfassenden Probe in Suspension befindet und/oder wobei sich die Zelle während der Hybridisierungs-, Einfangs- und/oder Nachweisschritte in Suspension befindet.

23. Verfahren nach einem der Ansprüche 1 bis 20, wobei die Probe, die die Zelle umfasst, aus einer Körperflüssigkeit, Blut oder einem Gewebeabschnitt stammt.

24. Verfahren nach einem der Ansprüche 1 bis 20, wobei das Bereitstellen einer Probe das Fixieren und Permeabilisieren der Zelle umfasst.

25. Verfahren nach einem der Ansprüche 1 bis 20, wobei das Verfahren das Waschen der Zelle umfasst, um Materialien zu entfernen, die nicht an einem der Nukleinsäuretargets eingefangen sind.

26. Verfahren nach einem der Ansprüche 1 bis 20, wobei das Nachweisen des Signals das Durchführen von Durchflusszytometrie umfasst.

## Revendications

1. Procédé de détection d'une cellule individuelle d'un type spécifié dans un échantillon comprenant un mélange de types de cellule, lequel mélange comprend au moins une cellule du type spécifié, dans lequel la cellule comprend une première cible d'acide nucléique et une seconde cible d'acide nucléique, dans lequel les première et seconde cibles d'acide nucléique coexistent toujours dans la cellule, le procédé comprenant:
(a) la fourniture d'une première sonde de marquage comprenant un premier marqueur, et la fourniture d'une seconde sonde de marquage comprenant un second marqueur, dans lequel un premier signal provenant du premier marqueur ne peut pas être distingué d'un second signal provenant du second marqueur;
(b) la capture, dans la cellule, de la première sonde de marquage sur la première cible d'acide nucléique, quand elle est présente dans la cellule, et de la seconde cible de marquage sur la seconde cible d'acide nucléique, quand elle est présente dans la cellule, dans lequel les première et seconde cibles d'acide nucléique sont des molécules séparées, dans lequel la capture comprend:
la fourniture pour la première cible d'acide nucléique d'au moins une première sonde de capture et la fourniture pour la seconde cible d'acide nucléique d'au moins une seconde sonde de capture;
la capture de la première sonde de marquage sur la première cible d'acide nucléique, comprenant l'hybridation dans la cellule de la première sonde de capture au premier acide nucléique cible, et l'hybridation de la première sonde de marquage à la première sonde de capture, pour ainsi capturer la première sonde de marquage sur la première cible d'acide nucléique ; et
la capture de la seconde sonde de marquage sur la seconde cible d'acide nucléique, comprenant l'hybridation dans la cellule de la seconde sonde de capture au second acide nucléique cible, et l'hybridation de la seconde sonde de marquage à la seconde sonde de capture, pour ainsi capturer la seconde sonde de marquage sur la seconde cible d'acide nucléique ;
(c) la détection du signal provenant des marqueurs;
(d) la mise en corrélation du signal détecté à partir de la cellule avec la présence ou la quantité des première et seconde cibles d'acide nucléique dans la cellule; et
(e) le fait d'identifier que la cellule est du type spécifié sur la base de la détection de la présence ou de la quantité des première et seconde cibles d'acide nucléique au sein de la cellule, dans lequel le type spécifié de cellule peut être distingué de l'autre type ou des autres types de cellule dans le mélange sur la base de la présence ou de la quantité de la première cible d'acide nucléique et de la présence ou de la quantité de la seconde cible d'acide nucléique dans la cellule.

2. Procédé selon la revendication 1, dans lequel la fourniture d'au moins une première sonde de capture comprend la fourniture de deux ou plus de deux premières sondes de capture différentes sous la forme d'un ensemble, dans lequel la fourniture d'au moins une seconde sonde de capture comprend la fourniture de deux ou plus de deux secondes sondes de capture différentes sous la forme d'un ensemble; dans lequel la capture de la première sonde de marquage sur la première cible d'acide nucléique comprend l'hybridation dans la cellule des deux ou plus de deux premières sondes de capture différentes au premier acide nucléique cible, et l'hybridation de la première sonde de marquage aux deux ou plus de deux premières sondes de capture différentes, pour ainsi capturer la première sonde de marquage sur la première cible d'acide nucléique; dans lequel la capture de la seconde sonde de marquage sur la seconde cible d'acide nucléique comprend l'hybridation dans la cellule des deux ou plus de deux secondes sondes de capture différentes au second acide nucléique cible, et l'hybridation de la seconde sonde de marquage aux deux ou plus de deux secondes sondes de capture différentes, pour ainsi capturer la seconde sonde de marquage sur la seconde cible d'acide nucléique.

3. Procédé selon la revendication 1, dans lequel la fourniture d'au moins une première sonde de capture comprend la fourniture de deux ou plus de deux premières sondes de capture différentes sous la forme d'un ensemble; dans lequel la fourniture d'au moins une seconde sonde de capture comprend la fourniture de deux ou plus de deux secondes sondes de capture différentes sous la forme d'un ensemble; dans lequel le procédé comprend en outre la fourniture d'un amplificateur, dans lequel l'amplificateur est une molécule capable de s'hybrider à multiples sondes de marquage ; dans lequel la capture de la première sonde de marquage sur la première cible d'acide nucléique comprend l'hybridation dans la cellule du premier acide nucléique cible aux deux ou plus de deux premières sondes de capture différentes, l'hybridation des deux ou plus de deux premières sondes de capture différentes à l'amplificateur, et l'hybridation de l'amplificateur à la première sonde de marquage, pour ainsi capturer la première sonde de marquage sur la première cible d'acide nucléique; dans lequel la capture de la seconde sonde de marquage sur la seconde cible d'acide nucléique comprend l'hybridation dans la cellule du second acide nucléique cible aux deux ou plus de deux secondes sondes de capture différentes, l'hybridation des deux ou plus de deux secondes sondes de capture différentes à l'amplificateur, et l'hybridation de l'amplificateur à la seconde sonde de marquage, pour ainsi capturer la seconde sonde de marquage sur la seconde cible d'acide nucléique.

4. Procédé selon la revendication 1, dans lequel la fourniture d'au moins une première sonde de capture comprend la fourniture de deux ou plus de deux premières sondes de capture différentes sous la forme d'un ensemble; dans lequel la fourniture d'au moins une seconde sonde de capture comprend la fourniture de deux ou plus de deux secondes sondes de capture différentes sous la forme d'un ensemble; dans lequel le procédé comprend en outre la fourniture d'un amplificateur et d'un préamplificateur; dans lequel la capture de la première sonde de marquage sur la première cible d'acide nucléique comprend l'hybridation dans la cellule du premier acide nucléique cible aux deux ou plus de deux premières sondes de capture différentes, l'hybridation des deux ou plus de deux premières sondes de capture différentes au préamplificateur, l'hybridation du préamplificateur à l'amplificateur, et l'hybridation de l'amplificateur à la première sonde de marquage, pour ainsi capturer la première sonde de marquage sur la première cible d'acide nucléique; dans lequel la capture de la seconde sonde de marquage sur la seconde cible d'acide nucléique comprend l'hybridation dans la cellule du second acide nucléique cible aux deux ou plus de deux secondes sondes de capture différentes, l'hybridation des deux ou plus de deux secondes sondes de capture différentes au préamplificateur, l'hybridation du préamplificateur à l'amplificateur, et l'hybridation de l'amplificateur à la seconde sonde de marquage, pour ainsi capturer la seconde sonde de marquage sur la seconde cible d'acide nucléique.

5. Procédé selon l'une quelconque des revendications 2 à 4, dans lequel l'hybridation des deux ou plus de deux sondes de capture différentes à la sonde de marquage, à l'amplificateur, ou au préamplificateur est effectuée à une température d'hybridation qui est supérieure à une température de fusion Tm d'un complexe entre chaque sonde de capture individuelle et la sonde de marquage, l'amplificateur, ou le préamplificateur.

6. Procédé selon la revendication 5, dans lequel les deux ou plus de deux sondes de capture différentes s'hybrident à des sections uniques et adjacentes sur la cible d'acide nucléique.

7. Procédé selon l'une quelconque des revendications 2 à 4, dans lequel l'hybridation des deux ou plus de deux sondes de capture différentes à leur cible d'acide nucléique correspondante est effectuée à une température d'hybridation qui est inférieure à une température de fusion Tm d'un complexe entre chaque sonde de capture individuelle et la cible d'acide nucléique, ou
dans lequel l'hybridation des deux ou plus de deux sondes de capture différentes à leur cible d'acide nucléique correspondante est effectuée à une température d'hybridation qui est supérieure à une température de fusion Tm d'un complexe entre chaque sonde de capture individuelle et la cible d'acide nucléique.

8. Procédé selon la revendication 1, comprenant en outre la fourniture d'une pluralité de cibles d'acide nucléique supplémentaires; dans lequel le procédé comprend en outre: la fourniture d'une pluralité de sondes de marquage comprenant chacune un marqueur, dans lequel le signal provenant de chaque marqueur ne peut pas être distingué des premier et second signaux ou l'un de l'autre; la fourniture d'au moins une sonde de capture pour chacune des cibles d'acide nucléique supplémentaires, l'hybridation dans la cellule de chaque sonde de capture à sa cible d'acide nucléique correspondante, quand elle est présente dans la cellule, et l'hybridation de chaque sonde de marquage à sa sonde de capture correspondante, et la détection du signal provenant des marqueurs.

9. Procédé selon l'une quelconque des revendications 1 à 8, dans lequel la première cible d'acide nucléique et la seconde cible d'acide nucléique sont sélectionnées indépendamment dans le groupe constitué: d'un ADN, d'un ARN, d'un ADN chromosomique, d'un ARNm, d'un acide nucléique endogène à la cellule, d'un acide nucléique introduit ou exprimé dans la cellule par infection de la cellule avec un pathogène et d'un ARN cytoplasmique.

10. Procédé selon la revendication 9, dans lequel:
(i) la première cible d'acide nucléique est un premier ARNm et dans lequel la seconde cible d'acide nucléique est un second ARNm; ou
(ii) la première cible d'acide nucléique comprend une première séquence polynucléotidique d'ADN chromosomique et dans lequel la seconde cible d'acide nucléique comprend une seconde séquence polynucléotidique d'ADN chromosomique; ou
(iii) la première cible d'acide nucléique et/ou la seconde cible d'acide nucléique comprend une molécule d'ADN bicaténaire, le procédé comprenant la dénaturation de l'ADN bicaténaire avant l'hybridation de la première et de la seconde sondes de capture aux première et seconde cibles d'acide nucléique.

11. Procédé selon l'une quelconque des revendications 1 à 8, dans lequel
(i) environ 1000 copies ou moins de la première cible d'acide nucléique et/ou environ 1000 copies ou moins de la seconde cible d'acide nucléique sont présentes dans la cellule; ou
(ii) environ 100 copies ou moins de la première cible d'acide nucléique et/ou environ 100 copies ou moins de la seconde cible d'acide nucléique sont présentes dans la cellule; ou
(iii) environ 10 copies ou moins de la première cible d'acide nucléique et/ou environ 10 copies ou moins de la seconde cible d'acide nucléique sont présentes dans la cellule; ou
(iv) environ 5 copies ou moins de la première cible d'acide nucléique et/ou environ 5 copies ou moins de la seconde cible d'acide nucléique sont présentes dans la cellule; ou
(v) une unique copie de la première cible d'acide nucléique et/ou une unique copie de la seconde cible d'acide nucléique sont présentes dans la cellule.

12. Procédé selon l'une quelconque des revendications 1 à 8, dans lequel la première cible d'acide nucléique, la seconde cible d'acide nucléique, et/ou la cible d'acide nucléique supplémentaire sont sélectionnées dans le groupe constitué de: CK8, CK14, CK17, CK18, CK19, CK20, EpCAM, MucI, EGFR, Twist, N-cadhérine et fibronectine.

13. Procédé de détection de la présence ou de l'absence d'une pluralité de cibles d'acide nucléique dans une cellule individuelle dans un échantillon comprenant la cellule, laquelle cellule comprend ou est suspectée de comprendre ladite pluralité de cibles d'acide nucléique, dans lequel lesdites cibles d'acide nucléique coexistent toujours dans la cellule, le procédé comprenant:
(a) la fourniture, pour chacune de la pluralité de cibles d'acide nucléique, d'une sonde de marquage comprenant un marqueur, dans lequel un signal provenant du marqueur de la première cible d'acide nucléique dans la pluralité de cibles d'acide nucléique ne peut pas être distingué d'un signal provenant du marqueur d'une seconde cible d'acide nucléique dans la pluralité de cibles d'acide nucléique, dans lequel la première et la seconde cible d'acide nucléique sont des molécules séparées;
(b) la fourniture, pour chacune de la pluralité de cibles d'acide nucléique, d'au moins une sonde de capture;
(c) l'hybridation dans la cellule, pour chacune de la pluralité de cibles d'acide nucléique, de la sonde de capture à sa cible d'acide nucléique correspondante, quand elle est présente dans la cellule;
(d) la capture, pour chacune de la pluralité de cibles d'acide nucléique, de la sonde de marquage sur la sonde de capture, pour ainsi capturer la sonde de marquage sur sa cible d'acide nucléique correspondante; et
(e) la détection du signal provenant du marqueur des sondes de marquage capturées sur la pluralité de cibles d'acide nucléique,
dans lequel la présence d'un signal indique la présence d'un membre de la pluralité de cibles d'acide nucléique et l'absence de signal indique l'absence des cibles d'acide nucléique dans la cellule.

14. Procédé selon la revendication 13, dans lequel la pluralité de cibles d'acide nucléique comprend des gènes domestiques de différents tissus dans une espèce particulière, dans lequel l'espèce est de préférence un humain.

15. Procédé selon l'une quelconque des revendications 13 et 14, dans lequel
(i) la fourniture d'au moins une sonde de capture comprend la fourniture de deux ou plus de deux sondes de capture; dans lequel chacune des deux ou plus de deux sondes de capture comprend une section T qui est complémentaire à une région de sa cible d'acide nucléique correspondante et une section L qui est complémentaire à une région de sa sonde de marquage correspondante; en outre, les sections T de deux ou plus de deux sondes de capture sont complémentaires à des régions non chevauchantes de la cible d'acide nucléique et les sections L des deux ou plus de deux sondes de capture sont complémentaires à des régions non chevauchantes de la sonde de marquage, et dans lequel le procédé comprend l'hybridation d'une sonde de marquage aux deux ou plus de deux sondes de capture;
(ii) la fourniture d'au moins une sonde de capture comprend la fourniture de deux ou plus de deux sondes de capture; dans lequel le procédé comprend en outre la fourniture d'un amplificateur; et dans lequel le procédé comprend l'hybridation d'une sonde de marquage à un amplificateur et l'hybridation de l'amplificateur auxdites deux ou plus de deux sondes de capture; ou
(iii) la fourniture d'au moins une sonde de capture comprend la fourniture de deux ou plus de deux sondes de capture; dans lequel le procédé comprend en outre la fourniture d'un amplificateur et d'un préamplificateur; et dans lequel le procédé comprend l'hybridation du préamplificateur aux deux ou plus de deux sondes de capture, l'hybridation de l'amplificateur au préamplificateur et l'hybridation d'une sonde de marquage à l'amplificateur.

16. Procédé selon la revendication 15 (i), comprenant l'étape d'hybridation de chaque ensemble de deux ou plus de deux sondes de capture à l'acide nucléique cible correspondant à une température d'hybridation (a) supérieure à la température de fusion de chaque section T de deux ou plus de deux sondes de capture dans l'ensemble, ou (b) supérieure à la température de fusion de chaque section L de deux ou plus de deux sondes de capture dans l'ensemble.

17. Procédé selon la revendication 16, comprenant l'étape d'hybridation de chaque ensemble de deux ou plus de deux sondes de capture à l'acide nucléique cible correspondant à une température d'hybridation (a) supérieure à la température de fusion de chaque section T de deux ou plus de deux sondes de capture dans l'ensemble et inférieure à la température de fusion de chaque section L de deux ou plus de deux sondes de capture dans l'ensemble, ou (b) supérieure à la température de fusion de chaque section L de deux ou plus de deux sondes de capture dans l'ensemble et inférieure à la température de fusion de chaque section T de deux ou plus de deux sondes de capture dans l'ensemble.

18. Procédé selon l'une quelconque des revendications 13 à 17, dans lequel la pluralité de cibles d'acide nucléique sont sélectionnées indépendamment dans le groupe constitué: d'un ADN, d'un ARN, d'un ADN chromosomique, d'un ARNm, d'un acide nucléique endogène à la cellule, d'un acide nucléique introduit ou exprimé dans la cellule par infection de la cellule avec un pathogène et des ARN cytoplasmiques.

19. Procédé selon l'une quelconque des revendications 15 (i), et 16 et 17, dans lequel toutes les deux ou plus de deux sondes de capture dans chaque ensemble ont les sections T en 5' des sections L, ou dans lequel toutes les deux ou plus de deux sondes de capture dans chaque ensemble ont les sections T en 3' des sections L.

20. Procédé selon l'une quelconque des revendications 1 à 19, dans lequel chaque étape d'hybridation ou de capture est accomplie pour toutes les cibles d'acide nucléique en même temps.

21. Procédé selon l'une quelconque des revendications 1 à 20, dans lequel la cellule est une cellule tumorale circulante.

22. Procédé selon l'une quelconque des revendications 1 à 20, dans lequel la cellule est en suspension dans l'échantillon comprenant la cellule, et/ou dans lequel la cellule est en suspension lors des étapes d'hybridation, de capture, et/ou de détection.

23. Procédé selon l'une quelconque des revendications 1 à 20, dans lequel l'échantillon comprenant la cellule est dérivé d'un fluide corporel, du sang, ou d'une section de tissu.

24. Procédé selon l'une quelconque des revendications 1 à 20, dans lequel la fourniture d'un échantillon comprend la fixation et la perméabilisation de la cellule.

25. Procédé selon l'une quelconque des revendications 1 à 20, dans lequel le procédé comprend le lavage de la cellule pour éliminer les matériels non capturés sur l'une des cibles d'acide nucléique.

26. Procédé selon l'une quelconque des revendications 1 à 20, dans lequel la détection du signal comprend la mise en oeuvre d'une cytométrie en flux.
